# EUROPÄISCHE PATENTSCHRIFT

(11) **EP 2 931 715 B1**
(45) Veröffentlichungstag und Bekanntmachung des Hinweises auf die Patenterteilung: **21.12.2016**
(21) Anmeldenummer: 13821793.0
(22) Anmeldetag: 14.11.2013
(51) Int. Cl.: C07D 309/40, C07D 405/12, C07D 213/69, A61K 8/49, A61Q 5/00, A61Q 17/04, A61Q 19/08

(54) **3-HYDROXY-4-OXO-4H-PYRAN- ODER 3-HYDROXY-4-OXO-1,4-DIHYDRO-PYRIDIN-DERIVATE ALS PROTEIN-ADHÄSIVE WIRKSTOFFE**
3-HYDROXY-4-OXO-4H-PYRANE OR 3-HYDROXY-4-OXO-1,4-DIHYDRO-PYRIDINE DERIVATIVES AS PROTEIN-ADHESIVE ACTIVE AGENTS
DÉRIVÉS DE 3-HYDROXY-4-OXO-4H-PYRANE OU 3-HYDROXY-4-OXO-1,4-DIHYDRO-PYRIDINE COMME AGENTS ACTIFS ADHÉSIFS AUX PROTÉINES

(30) Priorität: 13.12.2012 EP 12008308
(43) Veröffentlichungstag der Anmeldung: 21.10.2015
(73) Patentinhaber: Merck Patent GmbH, 64293 Darmstadt (DE)
(72) Erfinder: RUDOLPH, Thomas, 64291 Darmstadt (DE); BUEHLE, Philipp, 64673 Zwingenberg (DE)
(86) Internationale Anmeldenummer: PCT/EP2013/003428
(87) Internationale Veröffentlichungsnummer: WO 2014/090363

(56) Entgegenhaltungen:
- WO-A1-93/00339
- WO-A1-2008/086949
- WO-A2-2008/017346
- KI YOUNG SHIN ET AL: "A novel compound, maltolyl p-coumarate, attenuates cognitive deficits and shows neuroprotective effects in vitro and in vivo dementia models", JOURNAL OF NEUROSCIENCE RESEARCH, Bd. 85, Nr. 11, 2007, Seiten 2500-2511, XP055103451, ISSN: 0360-4012, DOI: 10.1002/jnr.21397
- AFSHIN FASSIHI ET AL: "Synthesis, antimicrobial evaluation and QSAR study of some 3-hydroxypyridine-4-one and 3-hydroxypyran-4-one derivatives", EUROPEAN JOURNAL OF MEDICINAL CHEMISTRY, Bd. 44, Nr. 5, 2009, Seiten 2145-2157, XP026029622, ISSN: 0223-5234, DOI: 10.1016/J.EJMECH.2008.10.022 [gefunden am 2008-10-30] in der Anmeldung erwähnt

## Beschreibung

Gegenstand der Erfindung ist die Verwendung von 4-Oxo-4H-pyran- oder 4-Oxo-1,4-dihydro-pyridin-Derivaten als protein-adhäsive Wirkstoffe, insbesondere als haut- oder haaranhaftende und/oder haut-oder haarbindende UV-Absorber, sowie zum Schutz der Haut, des Haares oder der Nägel vor Schäden verursacht durch ultraviolette Strahlung, spezielle 4-Oxo-4H-pyran- oder 4-Oxo-1,4-dihydropyridin-Derivate, deren Herstellung und Zubereitungen diese enthaltend.
Ein weiterer Gegenstand der Erfindung ist die Verwendung der Struktureinheit 4-Oxo-4H-pyran-3-O-yl oder 4-Oxo-1,4-dihydro-pyridin-3-O-yl als Linker zur Funktionalisierung von proteinhaltigen Matrices.

Ein bevorzugtes Einsatzgebiet der 4-Oxo-4H-pyran- oder 4-Oxo-1,4-dihydro-pyridin-Derivate nach Formel I, wie nachfolgend beschrieben, ist der UV-Schutz oder mit anderen Worten der Schutz der Haut, des Haares und der Nägel, der sogenannten proteinhaltigen Matrices, vor Schäden, die durch ultraviolette Strahlung verursacht werden können oder mit anderen Worten zum Schutz vor UV-induzierter Schädigungen der Haut, des Haares und der Nägel. Schäden, die durch ultraviolette Strahlung verursacht werden können sind beispielsweise Sonnenallergie, die Erzeugung von Erythemen oder Sonnenbrand, Entstehung von Hautkrebs und eine Abschwächung des Immunsystems, insbesondere des Immunsystems der Haut. Die menschliche Haut unterliegt natürlich auch gewissen Alterungsprozessen, die teilweise auf intrinsische Prozesse (chronoaging) und teilweise auf exogene Faktoren (environmental, z.B. photoaging) zurückzuführen sind. Photoalterung geht dabei einher mit dem Verlust der Elastizität der Haut und Pigmentflecken.
Ultraviolette Strahlung ist beispielsweise ultraviolette Solarstrahlung oder sonnenähnliches Licht. Unter sonnenähnlichem Licht versteht man eine Strahlung in einer spektralen Zusammensetzung analog der Sonnenstrahlung bzw. des Sonnenlichtes. Derartiges sonnenähnliches Licht kann zum Beispiel durch einen Solarsimulator erzeugt werden oder in Solarien zum Einsatz kommen, wobei die spektrale Zusammensetzung der Solarienstrahlung dem Sonnenlicht nicht exakt gleicht. Demzufolge soll unter sonnenähnlichem Licht auch Licht verstanden werden, das zumindest teilweise der spektralen Zusammensetzung des Sonnenlichtes gleicht oder das eine sonnenlichtähnliche Wirkung entfaltet.

Zu den exogenen Faktoren zählen insbesondere das Sonnenlicht oder künstliche Strahlungsquellen mit vergleichbarem Spektrum sowie Verbindungen, die durch die Strahlung entstehen können, wie undefinierte reaktive Photoprodukte, die auch radikalisch oder ionisch sein können.

Es ist eine Vielzahl von organischen und anorganischen UV-Filtern und Antioxidantien bekannt, die UV-Strahlung absorbieren und freie Radikale abfangen können. Sie sind so in der Lage, die menschliche Haut zu schützen. Durch diese Verbindungen wird die Transformation von UV-Licht in Wärme katalysiert.

Aufgrund mangelnder Hauthaftung ist aber die Schutzdauer begrenzt, insbesondere weil konventionelle UV-Absorber bzw. UV-Filter sehr leicht abgewaschen werden können, beispielsweise von Schweiß oder Wasser. Es ist eine beispielsweise aus WO 2006/018104 bekannte Strategie, UV-Absorber, UV-Filter oder Selbstbräunersubstanzen derart zu derivatisieren, dass sie über einen reaktiven Molekülpart kovalent an das Stratum Corneum der Epidermis binden können und so die Haut mit dem UV-Absorber, UV-Filter oder Selbstbräuner funktionalisieren. Für die effektive Anbindung an Proteine und Aminosäuren der äußeren Hautschichten ist es erforderlich, dass die entsprechenden UV-Absorber, UV-Filter oder Selbstbräuner, über eine möglichst hohe Reaktivität ihrer bindungsfähigen Molekülteile verfügen.

Ascorbinsäurederivate, insbesondere von in 6- und/oder 5-Position durch UV-Chromophore substituierte Ascorbinsäurederivate, sind beispielsweise aus WO 2008/017346 bekannt. Bei der Herstellung und Lagerung dieser Verbindungen arbeitet man unter inerten Bedingungen, beispielsweise unter Sauerstoffausschluss, da die Substanzen oxidationsempfindlich sind.

Es besteht daher ein zunehmender Bedarf nach hautverträglichen und oxidationsstabilen Verbindungen, die in der Lage sind, proteinhaltige Matrices zu funktionalisieren und sich in geeigneter Weise in kosmetische oder pharmakologische Zubereitungen einarbeiten lassen.

Es wurde nun überraschend festgestellt, dass sich die Verbindungen der Formel I, wie nachfolgend beschrieben, in hervorragender Weise als protein-adhäsive Wirkstoffe oder mit anderen Worten sich zur Funktionalisierung von proteinhaltigen Matrices eignen. Unter dem Begriff protein-adhäsiver Wirkstoff versteht man einen Wirkstoff, der aufgrund mindestens einer Adhäsion an eine proteinhaltige Matrix bindet. Bevorzugte Matrices sind hierbei Haut, Haare und/oder Nägel, wobei das generelle Prinzip auch auf die Funktionalisierung von synthetischen Polymermatrices, enthaltend Aminogruppen oder Thiolgruppen, isolierte Proteine oder Gelatine angewendet werden kann. Die durch Adhäsion und/oder Bindung an solche Matrices entstandenen Produkte können als beispielsweise kosmetische Wirkstoffe zur Herstellung kosmetischer Mittel verwendet werden. Die den Zusammenhalt bewirkenden Kräfte für eine Adhäsion sind nicht vollständig erforscht, so dass es verschiedene Adhäsionstheorien gibt, die sowohl die mechanische Adhäsion aufgrund physikalischmechanischer Kräfte und die spezifische Adhäsion aufgrund chemischer und physikalischer Kräfte umfassen. Es ist beispielsweise möglich, dass die Verbindungen der Formel I, wie nachfolgend beschrieben, bedingt durch ihre Amphiphilie, mit den Matrixmolekülen, wie zuvor beschrieben, physikalisch wechselwirken oder eine kovalente Bindung an eine Amino- oder Thiolgruppe der Matrix ausbilden. Die protein-adhäsiven Wirkstoffe können demzufolge mit der Matrix physikalisch wechselwirken oder eine kovalente Bindung ausbilden. Bedingt durch die Amphiphilie besitzen die Verbindungen der Formel I, wie nachfolgend beschrieben, auch oberflächenaktive Eigenschaften. Bevorzugt sind die Verbindungen der Formel I, wie nachfolgend beschrieben, haut- oder haaranhaftende und/oder haut-oder haarbindende UV-Absorber.
Die Verbindungen der Formel I, wie nachfolgend beschrieben, sind ebenfalls filmbildende Wirkstoffe. Auf Grund der homogenen Verteilung durch gleichmäßige Oberflächenadhäsion und/oder Bindung liegt ein wesentlicher Vorteil neben der langanhaltenden Haftung an der Matrix darin, dass weniger Wirksubstanz benötigt wird als bei Verwendung herkömmlicher UV-Absorber.
Die Verbindungen der Formel I, wie nachfolgend beschrieben, sind ebenfalls geeignete Stabilisatoren für Zubereitungen, insbesondere kosmetische Zubereitungen oder Medizinprodukte, gegen UV-Strahlung. Erfindungsgemäße Verbindungen der Formel I, wie nachfolgend beschrieben, eignen sich als Antiglykierungsmittel (Antiglycation agents) und wirken der Bildung von AGEs (advanced glycation endproducts) entgegen.

Ein erster Gegenstand der Erfindung ist demzufolge die nicht-therapeutische Verwendung von Verbindungen der Formel I, 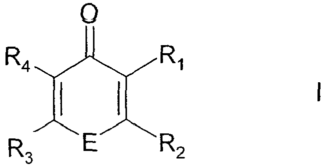 wobei
E NR₅ oder O bedeutet,
R₁ ein UV-Chromophor bedeutet, welches über ein O-Atom gebunden ist und die Verbindung der Formel I in die Lage versetzt, UV-Strahlung in dem Bereich von 400 bis 200 nm zu absorbieren,
R₂, R₃ oder R₄ jeweils unabhängig voneinander -H, -A, -OA, -(CH₂)ₚ-OH,-C(O)OA, COOH oder COOX bedeuten,
p eine ganze Zahl von 1 bis 4 bedeutet,
X das Gegenion zu der [COO⁻]-Gruppe ist,
R₅ A bedeutet und
A Alkyl mit 1 bis 20 C-Atomen bedeutet
und/oder deren Salze, Tautomere, Stereoisomere und/oder Solvate, einschließlich deren Mischungen in allen Verhältnissen, als protein-adhäsive Wirkstoffe, insbesondere als haut- oder haaranhaftende und/oder haut-oder haarbindende UV-Absorber.

Ein weiterer Gegenstand der Erfindung ist auch die nicht-therapeutische Verwendung von Verbindungen der Formel I, 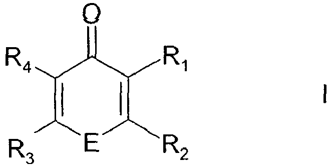 wobei
E NR₅ oder O bedeutet,
R₁ ein UV-Chromophor bedeutet, welches über ein O-Atom gebunden ist und die Verbindung der Formel I in die Lage versetzt, UV-Strahlung in dem Bereich von 400 bis 200nm zu absorbieren,
R₂, R₃ oder R₄ jeweils unabhängig voneinander -H, -A, -OA, -(CH₂)ₚ-OH,-C(O)OA, COOH oder COOX bedeuten,
p eine ganze Zahl von 1 bis 4 bedeutet,
X das Gegenion zu der [COO⁻]-Gruppe ist,
R₅ A bedeutet und
A Alkyl mit 1 bis 20 C-Atomen bedeutet und/oder deren Salze, Tautomere, Stereoisomere und/oder Solvate, einschließlich deren Mischungen in allen Verhältnissen, zum Schutz der Haut, des Haares und/oder der Nägel vor UV-induzierten Schäden.

Im Rahmen der Erfindung sind die Verbindungen der Formel I, so definiert, dass man darunter auch pharmazeutisch oder kosmetisch verwendbare Salze, Hydrate, Solvate der Verbindungen, Tautomere und optisch aktive Formen (wie z.B. Stereoisomere, Diastereomere, Enantiomere, Racemate) versteht. Unter Solvaten der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate. Die Verbindungen der Formel I können cis-trans-tsomere oder Tautomere bilden. Die Formel I schließt alle diese Formen ein.

Eine Base der Formel I kann mit einer Säure in das zugehörige Säureadditionssalz überführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern.

Andererseits können Säuren der Formel I mit Basen (z.B. Natrium- oder Kaliumhydroxid oder -carbonat) in die entsprechenden Metall-, insbesondere Alkalimetall- oder Erdalkalimetall- oder in die entsprechenden Ammoniumsalze umgewandelt werden.

Erfindungsgemäß werden Verbindungen der Formel I, wie zuvor beschrieben, verwendet, wenn

R₁ ein Substituent der Formel II, III, IV, V, VI, VII, VIII, IX, X, XI, XII, XIII, XIV oder XV ist, 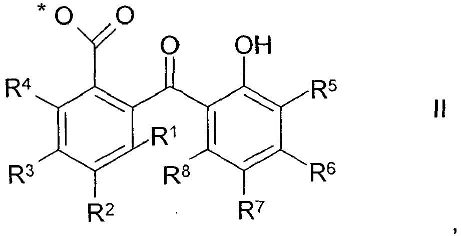 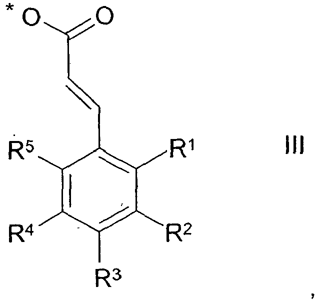 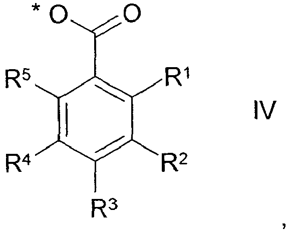 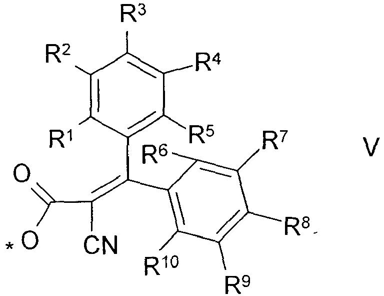 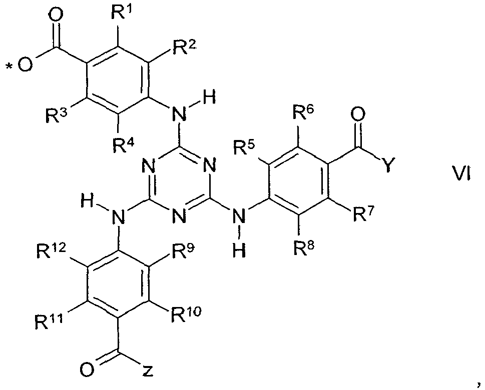 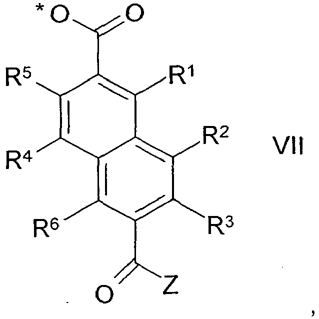 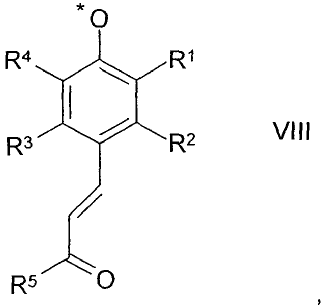 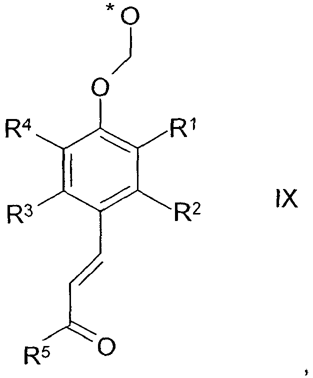 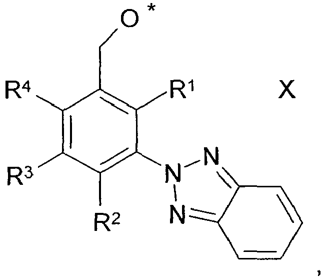 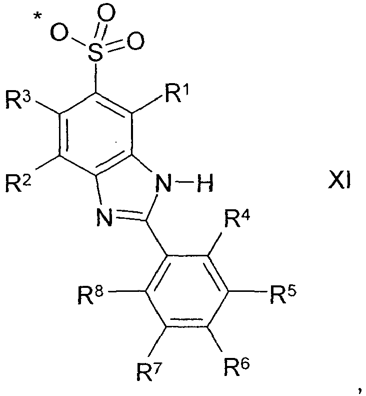 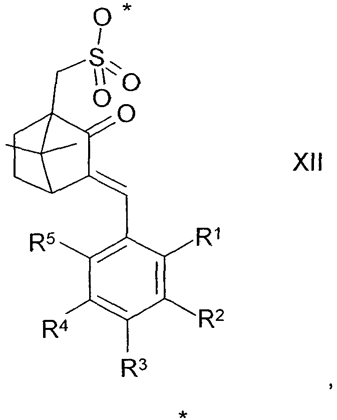 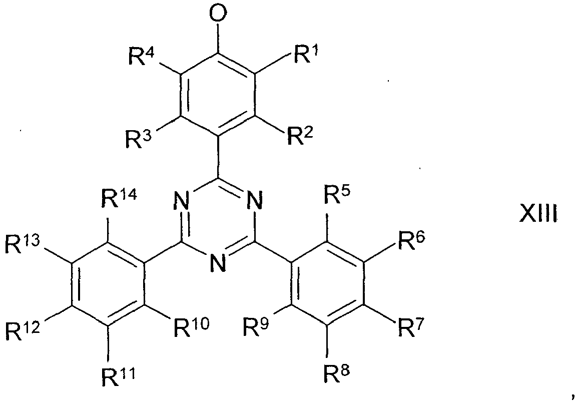 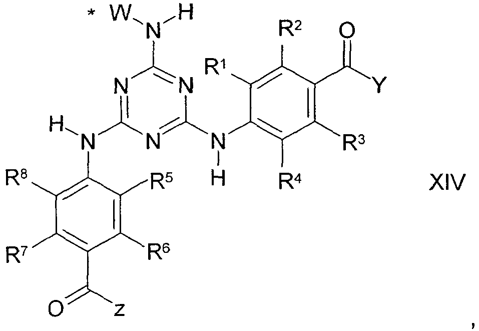 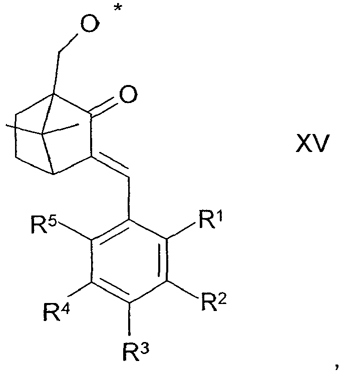 wobei
R¹ bis R¹⁴ jeweils unabhängig voneinander -H, -OH, -OA, -A, -NH₂, -NHA, - NA₂, -NH-(CH₂-CH₂-O)ₙ-H, -N[(CH₂-CH₂-O)ₙ-H]₂, -[NHA₂]X, -[NA₃]X, -SO₃H, -[SO₃]X, 2H-Benzotriazol-2-yl- oder 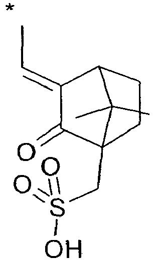 bedeuten,
n eine ganze Zahl von 1 bis 25 ist,
X das Gegenion zu den Kationen [NHA₂]⁺ und [NA₃]⁺ oder dem Anion [SO₃]⁻ ist und
Y und Z jeweils unabhängig voneinander die Struktureinheit I-1 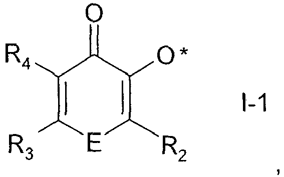 A, Hydroxy, -OA oder -NH-C(CH₃)₃ bedeuten, wobei R₂, R₃, R₄ und E eine zuvor für die Verbindungen der Formel I genannte Bedeutung haben,
W -(CH₂)ₘ-O oder -(CH₂)ₒ-C(O)-O bedeutet und
m und o jeweils unabhängig voneinander eine ganze Zahl von 1 bis 4 bedeuten,
mit der Bedingung, dass mindestens ein Susbstituent der Substituenten R¹ bis R¹⁴ in den Formeln II, III, IV, VII, X, XII und XV
OH, -OA, -A, -NH₂, -NHA, -NA₂, -NH'-(CH₂-CH₂-O)ₙ-H, -N[(CH₂-CH₂-O)ₙ-H]₂, -[NHA₂]X, -[NA₃]X, -SO₃H, -[SO₃]X, 2H-Benzotriazol-2-yl- oder 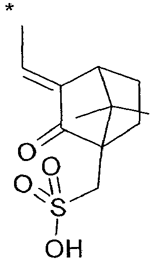 bedeutet.

Ein weiterer Gegenstand der Erfindung sind die Verbindungen der Formel I, 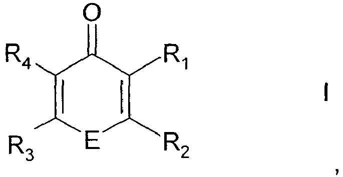 wobei
E NR₅ oder O bedeutet,
R₁ ein Substituent der Formel II, III, IV, V, VI, VII, VIII, IX, X, XI, XII, XIII, XIV oder XV ist, 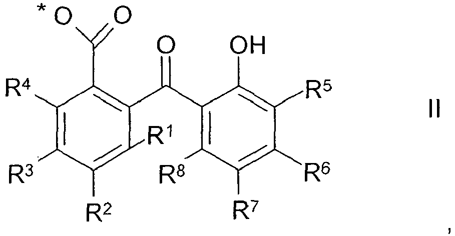 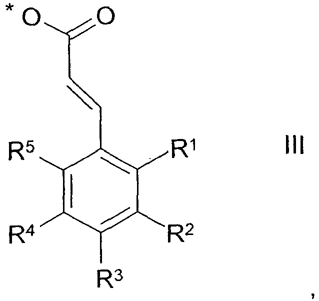 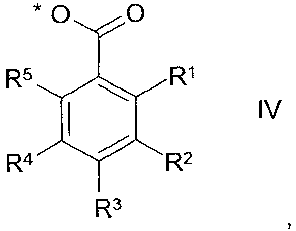 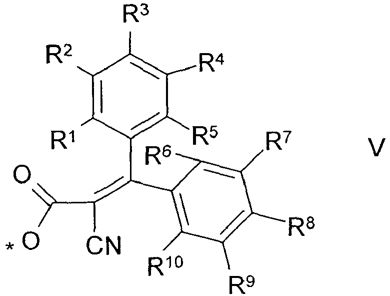 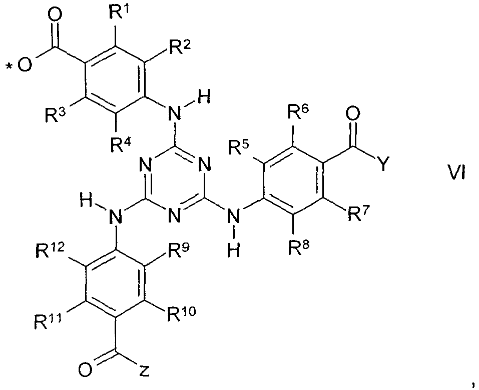 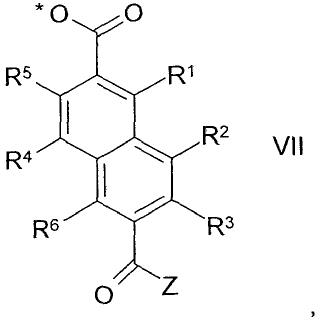 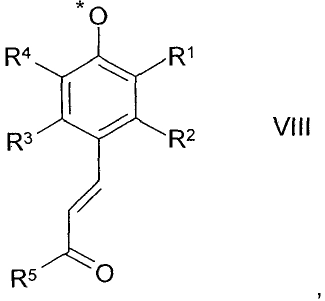 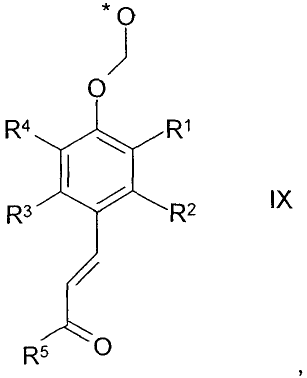 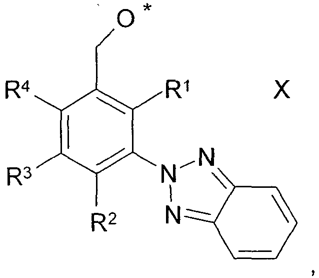 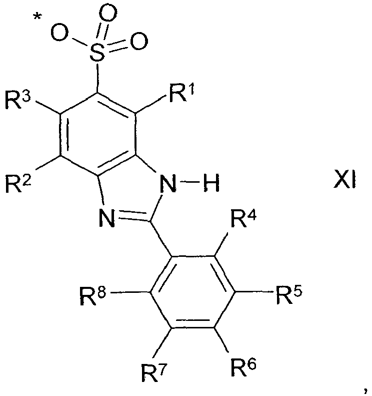 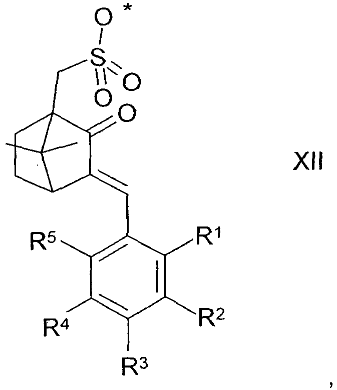 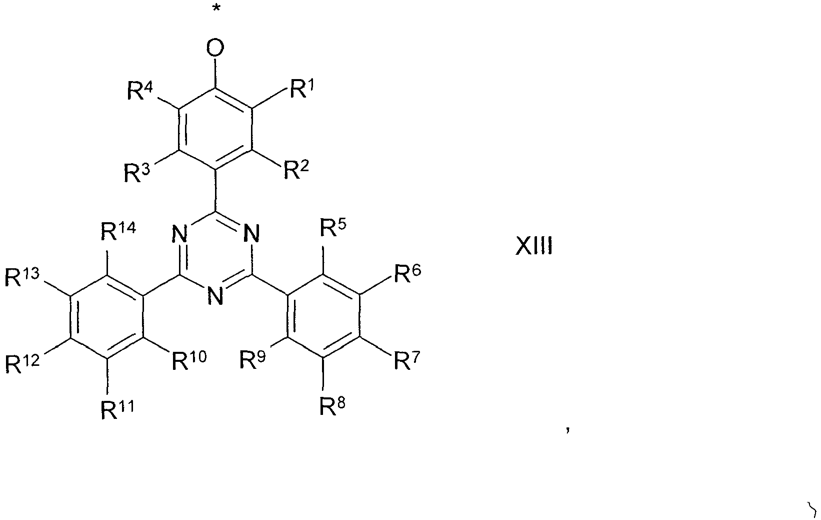 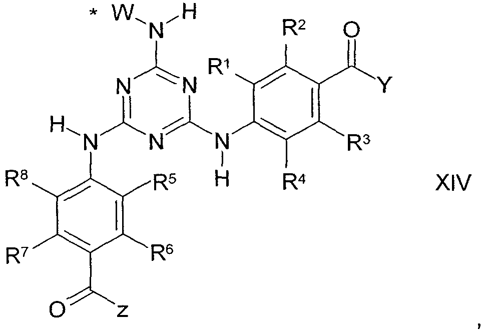 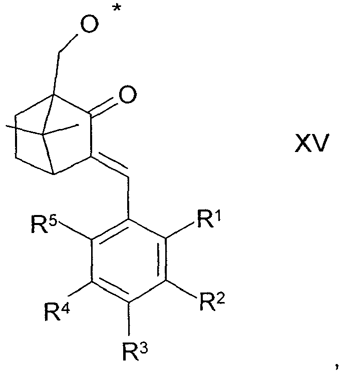 wobei
   R¹ bis R¹⁴ jeweils unabhängig voneinander -H, -OH, -OA, -A, -NH₂, -NHA, - NA₂, -NH-(CH₂-CH₂-O)ₙ-H, -N[(CH₂-CH₂-O)ₙ-H]₂, -[NHA₂]X, -[NA₃]X, -SO₃H, -[SO₃]X, 2H-Benzotriazol-2-yl- oder 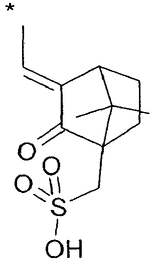 bedeuten,
n eine ganze Zahl von 1 bis 25 ist,
X das Gegenion zu den Kationen [NHA₂]⁺ und [NA₃]⁺ oder dem Anion [SO₃]⁻ ist und
Y und Z jeweils unabhängig voneinander die Struktureinheit I-1 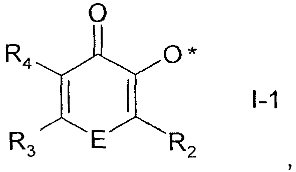 A, Hydroxy, -OA oder -NH-C(CH₃)₃ bedeuten, W -(CH₂)ₘ-O oder -(CH₂)ₒ-C(O)-O bedeutet und
m und o jeweils unabhängig voneinander eine ganze Zahl von 1 bis 4 bedeuten,
mit der Bedingung, dass mindestens ein Susbstituent der Substituenten R¹ bis R¹⁴ in den Formeln II, III, VII, X, XII und XV
   OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X, 2H-Benzotriazol-2-yl- oder 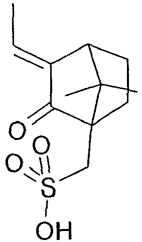 bedeutet,
R₂ oder R₄ jeweils unabhängig voneinander -H, -A, -OA, -(CH₂)ₚ-OH,-C(O)OA, COOH oder COOX bedeuten,
R₃ -H, Hydroxymethyl, -C(O)OEthyl oder -C(O)OMethyl bedeutet,
p eine ganze Zahl von 1 bis 4 bedeutet,
X das Gegenion zu der [COO⁻]-Gruppe ist,
R₅ A bedeutet und
A Alkyl mit 1 bis 20 C-Atomen bedeutet,
wobei, falls R₁ ein Substituent der Formel IV bedeutet, R² 2H-Benzotriazol-2-yl bedeutet, R⁴ und R⁵ H bedeuten, ein Substituent von R oder R H bedeutet und der andere Substituent -OH, -OA, -A, -NH₂, -NHA, -NA₂, -NH-(CH₂-CH₂-O)ₙ-H oder - N[(CH₂-CH₂-O)ₙ-H]₂ bedeutet, und
wobei die Verbindung (E)-3-(4-Hydroxy-phenyl)-acrylsäure-2-methyl-4-oxo-4H-pyran-3-yl-ester ausgenommen ist.

Die Abkürzung A steht für Alkyl mit 1 bis 20 C-Atomen, d.h. mit anderen Worten für eine lineare oder verzweigte Alkylgruppe mit 1 bis 20 C-Atomen, beispielsweise Methyl, Ethyl, Isopropyl, Propyl, Butyl, sec-Butyl oder tert-Butyl, weiterhin Pentyl, 1-, 2- oder 3-Methylbutyl, 1,1-, 1,2- oder 2,2-Dimethylpropyl, 1-Ethylpropyl, n-Hexyl, n-Heptyl, n-Octyl, weiterhin Nonyl, Decyl, Undecyl, Dodecyl, Tridecyl, Tetradecyl, Pentadecyl, Hexadecyl, Heptadecyl, Oktadecyl, Nonadecyl und Eicosyl. Bevorzugt wird aus der geradkettigen oder verzweigten Alkylgruppe mit 1 bis 20 C-Atomen die geradkettige oder verzweigte Alkylgruppe mit 1 bis 8 C-Atomen, d.h. Methyl, Ethyl, Isopropyl, Propyl, Butyl, sec-Butyl oder tert-Butyl, weiterhin Pentyl, 1-, 2- oder 3-Methylbutyl, 1,1-, 1,2- oder 2,2-Dimethylpropyl, 1-Ethylpropyl, n-Hexyl, n-Heptyl, n-Octyl, ausgewählt. Besonders bevorzugt wird aus der geradkettigen oder verzweigten Alkylgruppe mit 1 bis 20 C-Atomen die geradkettige oder verzweigte Alkylgruppe mit 1 bis 4 C-Atomen ausgewählt, d.h. Methyl, Ethyl, Isopropyl, Propyl, Butyl, sec-Butyl oder tert-Butyl.

Die Abkürzung -OA steht für O-Alkyl mit 1 bis 20 C-Atomen, gleichbedeutend für Alkoxy mit 1 bis 20 C-Atomen, wobei A wie zuvor definiert verwendet wird. Entsprechend steht die Abkürzung -NHA für Alkylamino und -NA₂ für Dialkylamino.

Die Variable n steht für eine ganze Zahl von 1 bis 25, vorzugsweise für eine ganze Zahl von 1, 2, 3, 4 oder 5.

Die Variablen m und o stehen jeweils unabhängig voneinander für 1, 2, 3 oder 4. Die Variable m ist bevorzugt 2. Die Variable o ist bevorzugt 3.

X beschreibt das Gegenion für die Kationen [NHA₂]⁺ und [NA₃]⁺, wobei A eine der zuvor angegebenen Bedeutungen hat, vorzugsweise Cl⁻, Br⁻, I⁻ oder [SO₄]²⁻ oder das Gegenion des Anions [COO]⁻ oder [SO₃]⁻, vorzugsweise ein Ammoniumion oder ein Alkalimetall- oder Erdalkalimetallkation wie Na⁺, K⁺, Mg²⁺ oder Ca²⁺.

In einer Ausführungsform sind Verbindungen der Formel I bevorzugt oder bevorzugt zu verwenden, bei denen der Substituent R₄ H bedeutet.

In einer Ausführungsform sind Verbindungen der Formel I bevorzugt oder bevorzugt zu verwenden, bei denen der Substituent R₃ H, -(CH₂)ₚ-OH,-C(O)OA, COOH oder COOX bedeutet, wobei A, X und p eine zuvor angegebene oder bevorzugt angegebene Bedeutung haben. Besonders bevorzugt ist der Substiuent R₃ H, Hydroxymethyl, -C(O)OEthyl oder -C(O)OMethyl. Ganz besonders bevorzugt ist der Substituent R₃ H.

In einer Ausführungsform sind Verbindungen der Formel bevorzugt oder bevorzugt zu verwenden, bei denen der Substituent R₂ A bedeutet, wobei A eine der zuvor angegebenen Bedeutungen hat. Besonders bevorzugt ist der Substituent R₂ Methyl oder Ethyl.

In einer Ausführungsform sind Verbindungen der Formel I bevorzugt oder bevorzugt zu verwenden, bei denen der Substituent R₅ A bedeutet und A eine der als bevorzugt angegebenen Bedeutungen hat. Besonders bevorzugt ist R₅ Methyl oder Ethyl. Ganz besonders bevorzugt ist R₅ Methyl.

In einer bevorzugten Ausführungsform sind Verbindungen der Formel I bevorzugt oder bevorzugt zu verwenden, bei denen die Variable E O oder NR₅ bedeutet und die Substituenten R₅, R₄, R₃, R₂ und R₁ eine der zuvor angegebenen oder nachfolgend angegebenen bevorzugten Bedeutungen oder besonders bevorzugt angegebenen Bedeutungen haben.

Demzufolge sind die Verbindungen der Formeln I-a, I-b, I-c und I-d besonders bevorzugt: 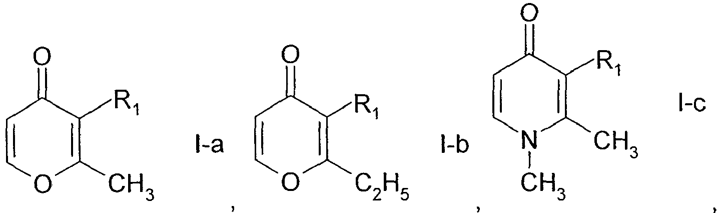 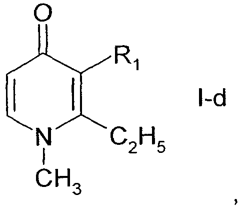 wobei R₁ eine der nachfolgend angegebenen Bedeutungen der gelisteten Teilformeln hat oder eine oder mehrere besonders bevorzugte Bedeutungen der nachfolgend gelisteten Teilformeln hat.

In einer Ausführungsform sind Verbindungen der Formel I bevorzugt oder bevorzugt zu verwenden, bei denen die Variable E O bedeutet.

In einer Ausführungsform sind Verbindungen der Formel I bevorzugt oder bevorzugt zu verwenden, bei denen die Variable E N-R₅ bedeutet und R₅ A bedeutet, wobei A eine der zuvor angegebenen oder bevorzugt angegebenen Bedeutungen hat. Bevorzugt bedeutet die Variable E N-Methyl.

In einer bevorzugten Ausführungsform sind Verbindungen der Formel I bevorzugt oder bevorzugt zu verwenden, bei denen die Variable E O bedeutet und die Substituenten R₄, R₃, R₂ und R₁ eine der zuvor angegebenen oder nachfolgend angegebenen bevorzugten Bedeutungen oder besonders bevorzugt angegebenen Bedeutungen haben. In dieser Ausführungsform sind die Verbindungen der Formel I-a oder I-b besonders bevorzugt. In dieser Ausführungsform sind die Verbindungen der Formel I-a ganz besonders bevorzugt.

In einer bevorzugten Ausführungsform sind Verbindungen der Formel I bevorzugt oder bevorzugt zu verwenden, bei denen die Variable E NR₅ bedeutet und die Substituenten R₅, R₄, R₃, R₂ und R₁ eine der zuvor angegebenen oder nachfolgend angegebenen bevorzugten Bedeutungen oder besonders bevorzugt angegebenen Bedeutungen haben. In dieser Ausführungsform sind die Verbindungen der Formel I-c oder I-d besonders bevorzugt. In dieser Ausführungsform sind die Verbindungen der Formel I-c ganz besonders bevorzugt.

Im Folgenden werden bevorzugte Ausführungsformen für den Substituenten R₁ gelistet, der durch die Formeln II, III, IV, V, VI, VII, VIII, IX, X, XI, XII, XIII, XIV und XV beschrieben wird. Jede bevorzugte oder besonders bevorzugte Ausführungsform einer der genannten Formeln II bis XV kann mit einer oder mehreren der genannten bevorzugten oder besonders bevorzugten Ausführungsformen der übrigen Formeln II bis XV kombiniert werden, um den Substituenten R₁ bei den Verbindungen der Formel I, I-a, I-b, I-c oder I-d bevorzugt zu beschreiben.

In einer Ausführungsform für die Substituenten der Formel II ist R⁶ vorzugsweise -OH, -OA, -A, -NH₂, -NHA, -NA₂, -NH-(CH₂-CH₂-O)ₙ-H,-N[(CH₂-CH₂-O)ₙ-H]₂, SO₃H, SO₃X oder 2H-Benzotriazol-2-yl. Besonders bevorzugt sind die Radikale R¹ bis R⁵, R⁷ und R⁸ H und
R⁶ bedeutet -OH, -OA, -A, -NH₂, -NHA, -NA₂, -NH-(CH₂-CH₂-O)ₙ-H,-N[(CH₂-CH₂-O)ₙ-H]₂, SO₃H, SO₃X oder 2H-Benzotriazol-2-yl, ganz besonders bevorzugt -OA, -NH₂, -NHA oder -NA₂. Aus der angegebenen Liste -OA, -NH₂, -NHA oder -NA₂ für R⁶ ist -OA oder -NA₂ bevorzugt auszuwählen. Aus der angegebenen Liste -OA, -NH₂, -NHA oder -NA₂ für R⁶ ist -NA₂ besonders bevorzugt auszuwählen. A hat eine der zuvor angegebenen Bedeutungen oder als bevorzugt angegebenen Bedeutungen. Für R⁶ bedeutet A bevorzugt eine lineare oder verzweigte Alkylgruppe mit 2 bis 4 C-Atomen, besonders bevorzugt Ethyl, iso-Propyl, n-Propyl, n-Butyl, sec.-Butyl oder tert.-Butyl.

In einer Ausführungsform für die Substituenten der Formel III ist R³ -OH,-OA, -A, -NH₂, -NHA, -NA₂, -NH-(CH₂-CH₂-O)ₙ-H, -N[(CH₂-CH₂-O)ₙ-H]₂, SO₃H, SO₃X oder 2H-Benzotriazol-2-yl. Besonders bevorzugt sind die Radikale R¹, R², R⁴ und R⁵ H und
R³ -OH, -OA, -A, -NH₂, -NHA, -NA₂, -NH-(CH₂-CH₂-O)ₙ-H, -N[(CH₂-CH₂-O)ₙ-H]₂, SO₃H, SO₃X oder 2H-Benzotriazol-2-yl, ganz besonders bevorzugt -OA, -NH₂, -NHA oder -NA₂. Aus der angegebenen Liste -OA, -NH₂, -NHA oder -NA₂ für R³ ist -OA oder -NA₂ bevorzugt auszuwählen. Aus der angegebenen Liste -OA, -NH₂, -NHA oder -NA₂ für R³ ist -OA besonders bevorzugt auszuwählen. A hat eine der zuvor angegebenen Bedeutungen oder als bevorzugt angegebenen Bedeutungen.

In einer Ausführungsform für die Substituenten der Formel IV sind R³ oder R⁵ H, -OH, -OA, -A, -NH₂, -NHA, -NA₂, -NH-(CH₂-CH₂-O)ₙ-H, -N[(CH₂-CH₂-O)ₙ-H]₂ SO₃H, SO₃X oder 2H-Benzotriazol-2-yl bedeuten, wobei mindestens ein Substituent R³ oder R⁵ -OH, -OA, -A, -NH₂, -NHA, -NA₂,-NH-(CH₂-CH₂-O)ₙ-H, -N[(CH₂-CH₂-O)ₙ-H]₂, SO₃H, SO₃X oder 2H-Benzotriazol-2-yl bedeutet. Besonders bevorzugt sind die Radikale R¹, R² und R₄ H und
R³ oder R⁵ bedeuten H, -OH, -OA, -A, -NH₂, -NHA, -NA₂, -NH-(CH₂-CH₂-O)ₙ-H, -N[(CH₂-CH₂-O)ₙ-H]₂, SO₃H, SO₃X oder 2H-Benzotriazol-2-yl, wobei mindestens ein Substituent R³ oder R⁵ -OH, -OA, -A, -NH₂, -NHA, -NA₂,-NH-(CH₂-CH₂-O)ₙ-H, -N[(CH₂-CH₂-O)ₙ-H]₂, SO₃H, SO₃X oder 2H-Benzotriazol-2-yl bedeutet. R³ oder R⁵ bedeuten ganz besonders bevorzugt H, -OH, -OA, -NH₂, -NHA oder -NA₂, wobei mindestens ein Substituent R³ oder R⁵ -OH, -OA, -NH₂, -NHA oder -NA₂ bedeutet. Aus der angegebenen Liste -OH, -OA, -NH₂, -NHA oder -NA₂ für R³ oder R⁵ ist jeweils unabhängig -OA oder -NA₂ bevorzugt auszuwählen. Aus der angegebenen Liste -OH, -OA, -NH₂, -NHA oder -NA₂ für R³ oder R⁵ ist jeweils unabhängig-NA₂ besonders bevorzugt auszuwählen. A hat eine der zuvor angegebenen Bedeutungen oder als bevorzugt angegebenen Bedeutungen. In einer Ausführungsform ist es bevorzugt, wenn R⁵ OH bedeutet und R¹ bis R⁴ jeweils unabhängig ausgewählt werden aus H, -OH, -OA, -A, -NH₂, -NHA, -NA₂, -NH-(CH₂-CH₂-O)ₙ-H, -N[(CH₂-CH₂-O)ₙ-H]₂ oder 2H-Benzotriazol-2-yl. In einer Ausführungsform ist es bevorzugt, wenn R⁵ OH bedeutet, R¹, R² und R⁴ H bedeuten und R³ ausgewählt wird aus -OA, - A, -NH₂, -NHA, -NA₂, -NH-(CH₂-CH₂-O)ₙ-H, -N[(CH₂-CH₂-O)ₙ-H]₂ oder 2H-Benzotriazol-2-yl. In einer Ausführungsform ist es besonders bevorzugt, wenn R⁵ OH bedeutet, R¹, R² und R⁴ H bedeuten und R³ ausgewählt wird aus -OA, -A, -NH₂, -NHA oder -NA₂.

In einer Ausführungsform für die Substituenten der Formel IV ist R² 2H-Benzotriazol-2-yl und R¹ oder R³ sind jeweils unabhängig vorzugsweise H, - OH, -OA, -A, -NH₂, -NHA, -NA₂, -NH-(CH₂-CH₂-O)ₙ-H oder -N[(CH₂-CH₂-O)ₙ-H]₂. Besonders bevorzugt sind die Radikale R⁴ und R⁵ H und
R² bedeutet 2H-Benzotriazol-2-yl und R¹ oder R³ bedeuten jeweils unabhängig voneinander H, -OH, -OA, -A, -NH₂, -NHA, -NA₂, -NH-(CH₂-CH₂-O)ₙ-H oder -N[(CH₂-CH₂-O)ₙ-H]₂, ganz besonders bevorzugt -OH. Bevorzugt ist ein Substituent von R¹ oder R³ H und der andere Substituent ist vorzugsweise -OH, -OA, -A, -NH₂, -NHA, -NA₂, -NH-(CH₂-CH₂-O)ₙ-H oder -N[(CH₂-CH₂-O)ₙ-H]₂ oder besonders bevorzugt OH. A hat eine der zuvor angegebenen Bedeutungen oder als bevorzugt angegebenen Bedeutungen.

In einer Ausführungsform für die Substituenten der Formel V sind die Substituenten R², R⁴, R⁷ und R⁹ vorzugsweise H und die Substituenten R¹, R³, R⁵, R⁶, R⁸ und R¹⁰ sind jeweils unabhängig voneinander H, -OH, -OA, - A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X oder 2H-Benzotriazol-2-yl. Bevorzugt sind R¹, R³, R⁵, R⁶, R⁸ und R¹⁰ in Formel V jeweils unabhängig voneinander vorzugsweise H, -OA, -NH₂, -NHA oder -NA₂.

Aus der angegebenen Liste -H, -OA, -NH₂, -NHA oder -NA₂ sind bevorzugt alle Substituenten H und/oder R³ und/oder R⁸ sind -OA und R¹, R⁵, R⁶ und R¹⁰ sind H. A hat eine zuvor angegebenen Bedeutung oder eine zuvor als bevorzugt angegebene Bedeutung.

In einer Ausführungsform für die Substituenten der Formel VI sind die Substituenten R², R⁴, R⁵, R⁸, R⁹ und R¹² vorzugsweise jeweils unabhängig voneinander H oder OH und die Substituenten R¹, R³, R⁶, R⁷, R¹⁰ und R¹¹ sind jeweils unabhängig voneinander H, -OH, -OA, -A, -NH₂, -NHA, -NA₂,-NH-(CH₂-CH₂-O)ₙ-H, -N[(CH₂-CH₂-O)ₙ-H]₂, -[NHA₂]X, -[NA₃]X, -SO₃H,-[SO₃]X oder 2H-Benzotriazol-2-yl und Y und Z bedeuten vorzugsweise Hydroxy, -OA oder -NH-C(CH₃)₃, wobei A eine der zuvor angegebenen Bedeutungen oder als bevorzugt angegebenen Bedeutungen besitzt. Die Substituenten R¹, R³, R⁶, R⁷, R¹⁰ und R⁸ in Formel VI sind jeweils unabhängig voneinander vorzugsweise H, -OA, -NH₂, -NHA oder -NA₂. Die Substituenten R¹, R³, R⁶, R⁷, R¹⁰ und R⁸ in Formel VI sind besonders bevorzugt H. Die Variablen Y und Z bedeuten besonders bevorzugt OA. Die Variablen Y und Z bedeuten ganz besonders bevorzugt n-Hexyloxy oder 2-Ethylhexyloxy.

In einer Ausführungsform für die Substituenten der Formel VII sind die Substituenten R² und R⁴ vorzugsweise H, mindestens einer der Substituenten R¹, R³, R⁵ und R⁶ -OH, -OA, -A, -NH₂, -NHA, -NA₂, -NH-(CH₂-CH₂-O)ₙ-H, -N[(CH₂-CH₂-O)ₙ-H]₂, SO₃H, SO₃X oder 2H-Benzotriazol-2-yl ist und Z eine zuvor angegebene Bedeutung hat.
Z in Formel VII bedeutet bevorzugt -OH oder -OA, wobei A eine zuvor angegebene Bedeutung hat oder eine als bevorzugt angegebene Bedeutung hat.
In einer Ausführungsform für die Substituenten der Formel VII sind die Substituenten R² und R⁴ H, mindestens einer der Substituenten R¹, R³, R⁵ und R⁶ ist vorzugsweise -OH oder -OA und Z hat eine zuvor angegebene Bedeutung.

In einer Ausführungsform für die Substituenten der Formel VII sind die Substituenten R² und R⁴ H, mindestens einer der Substituenten R¹, R³, R⁵ und R⁶ ist -OH und Z hat eine zuvor angegebene Bedeutung.

In einer Ausführungsform für die Substituenten der Formel VIII ist R⁵ vorzugsweise -OH, -OA, -A, -NH₂, -NHA, -NA₂, -NH-(CH₂-CH₂-O)ₙ-H,-N[(CH₂-CH₂-O)ₙ-H]₂, SO₃H, SO₃X oder 2H-Benzotriazol-2-yl. Besonders bevorzugt sind die Radikale R¹, R², R³ und R⁴ H und
R⁵ bedeutet -OH, -OA, -A, -NH₂, -NHA, -NA₂, -NH-(CH₂-CH₂-O)ₙ-H,-N[(CH₂-CH₂-0Oₙ-H], SO₃H, SO₃X oder 2H-Benzotriazol-2-yl. R⁵ in Formel VIII bedeutet besonders bevorzugt -OA, wobei A eine der zuvor angegebenen Bedeutungen oder als bevorzugt angegebenen Bedeutungen hat. In Verbindungen der Formel VIII bedeutet A besonders bevorzugt 2-Ethylhexyl.

In einer Ausführungsform für die Substituenten der Formel IX ist R⁵ vorzugsweise -OH, -OA, -A, -NH₂, -NHA, -NA₂, -NH-(CH₂-CH₂-O)ₙ-H,-N[(CH₂-CH₂-O)ₙ-H]₂, SO₃H, SO₃X oder 2H-Benzotriazol-2-yl. Besonders bevorzugt sind die Radikale R¹, R², R³ und R⁴ H und
R⁵ bedeutet -OH, -OA, -A, -NH₂, -NHA, -NA₂, -NH-(CH₂-CH₂-O)ₙ-H,-N[(CH₂-CH₂-O)ₙ-H]₂, SO₃H, SO₃X oder 2H-Benzotriazol-2-yl, ganz besonders bevorzugt -OA. A hat eine der zuvor angegebenen Bedeutungen oder als bevorzugt angegebenen Bedeutungen. In Verbindungen der Formel IX bedeutet A besonders bevorzugt 2-Ethylhexyl.

In einer Ausführungsform für die Substituenten der Formel X sind R¹ und/oder R² vorzugsweise -OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X oder 2H-Benzotriazol-2-yl. Besonders bevorzugt sind die Radikale R³ und R⁴ H und R¹ und/oder R² bedeuten -OH, -OA, -A, -NH₂, -NHA, -NA₂, -NH-(CH₂-CH₂-O)ₙ-H, -N[(CH₂-CH₂-O)ₙ-H]₂ oder 2H-Benzotriazol-2-yl, ganz besonders bevorzugt -OH. A hat eine der zuvor angegebenen oder als bevorzugt angegebenen Bedeutungen.

In einer Ausführungsform für die Substituenten der Formel XI ist R⁶ vorzugsweise H, -OH, -OA, -A, -NH₂, -NHA, -NA₂, -NH-(CH₂-CH₂-O)ₙ-H,-N[(CH₂-CH₂-O)ₙ-H]₂, -[NHA₂]X, -[NA₃]X, -SO₃H, -[SO₃]X oder 2H-Benzotriazol-2-yl. Besonders bevorzugt sind die Substituenten R¹, R², R³, R⁴, R⁵, R⁷ und R⁸ vorzugsweise H und
R⁶ bedeutet H, -OH, -OA, -A, -NH₂, -NHA, -NA₂, -NH-(CH₂-CH₂-O)ₙ-H,-N[(CH₂-CH₂-O)ₙ-H]₂, -[NHA₂]X, -[NA₃]X, -SO₃H, -[SO₃]X oder 2H-Benzotriazol-2-yl. A hat eine der zuvor angegebenen Bedeutungen oder als bevorzugt angegebenen Bedeutungen.
R⁶ in Formel XI bedeutet besonders bevorzugt H.

In einer Ausführungsform für die Substituenten der Formel XII ist R³ vorzugsweise -OH, -OA, -A, -NH₂, -NHA, -NA₂, -NH-(CH₂-CH₂-O)ₙ-H,-N[(CH₂-CH₂-O)ₙ-H] -[NHA₂]X, -[NA₃]X, -SO₃H, -[SO₃]X, 2H-Benzotriazol-2-yl oder 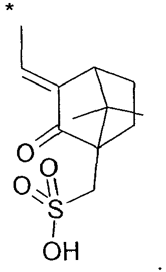
Besonders bevorzugt sind die Substituenten R¹, R², R⁴ und R⁵ vorzugsweise H und
R³ bedeutet -OH, -OA, -A, -NH₂, -NHA, -NA₂, -NH-(CH₂-CH₂-O)ₙ-H,-N[(CH₂-CH₂-O)ₙ-H]₂, -[NHA₂]X, -[NA₃]X, -SO₃H, -[SO₃]X, 2H-Benzotriazol-2-yl oder 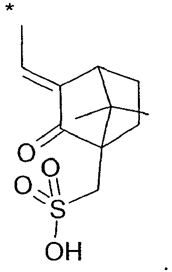 A hat eine der zuvor angegebenen Bedeutungen oder als bevorzugt angegebenen Bedeutungen.
R³ in Formel XII bedeutet besonders bevorzugt A.

In einer alternativen Ausführungsform sind Verbindungen der Formel XII bevorzugt, die als Substituent R³ die Teilstruktur 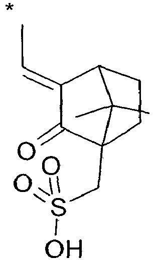 aufweisen.

In einer Ausführungsform für die Substituenten der Formel XIII sind R⁵, R⁷, R¹² und R¹⁴ jeweils unabhängig voneinander vorzugsweise -OH, -OA, -A,-NH₂, -NHA, -NA₂, -NH-(CH₂-CH₂-O)ₙ-H, -N[(CH₂-CH₂-O)ₙ-H]₂ oder 2H-Benzotriazol-2-yl. Besonders bevorzugt sind die Substituenten R¹, R², R³, R⁴, R⁶, R⁸, R⁹, R¹⁰, R⁸ und R¹³ H und
R⁵, R⁷, R¹² und R¹⁴ bedeuten jeweils unabhängig voneinander -OH, -OA, - A, -NH₂, -NHA, -NA₂, -NH-(CH₂-CH₂-O)ₙ-H, -N[(CH₂-CH₂-O)ₙ-H]₂ oder 2H-Benzotriazol-2-yl. In einer Ausführungsform der Substituenten der Formel XIII sind die Substituenten R⁵ und R¹⁴ OH oder OA und R⁷ und R¹² sind jeweils unabhängig voneinander -OA und A hat eine der zuvor angegebenen oder als bevorzugt angegebenen Bedeutungen. In einer Ausführungsform der Substituenten der Formel XIII sind die Substituenten R⁵ und R¹⁴ bevorzugt OH und R⁷ und R¹² jeweils unabhängig voneinander OA und A hat eine der zuvor angegebenen oder als bevorzugt angegebenen Bedeutungen. A ist in Formel XIII bevorzugt Methyl oder 2-Ethylhexyl.

In einer Ausführungsform für die Substituenten der Formel XIV ist W bevorzugt -CH₂-CH₂-O oder -CH₂-CH₂-CH₂-C(O)O. Besonders bevorzugt sind die Substituenten R¹, R², R³, R⁴, R⁵, R⁶, R⁷ und R⁸H und
W ist bevorzugt -CH₂-CH₂-O oder -CH₂-CH₂-CH₂-C(O)O. Y und Z sind in Verbindungen der Formel XIV bevorzugt OA und A hat eine der zuvor angegebenen Bedeutungen. Bevorzugt ist A in Formel XIV n-Butyl.

In einer Ausführungsform für die Substituenten der Formel XV ist R³ vorzugsweise -OH, -OA, -A, -NH₂, -NHA, -NA₂, -NH-(CH₂-CH₂-O)ₙ-H,-N[(CH₂-CH₂-O)ₙ-H]₂, -[NHA₂]X, -[NA₃]X, -SO₃H, -[SO₃]X, 2H-Benzotriazol-2-yl oder 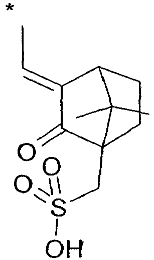 Besonders bevorzugt sind die Substituenten R¹, R², R⁴ und R⁵ vorzugsweise H und
R³ bedeutet -OH, -OA, -A, -NH₂, -NHA, -NA₂, -NH-(CH₂-CH₂-O)ₙ-H,-N[(CH₂-CH₂-O)ₙ-H]₂, -[NHA₂]X, -[NA₃]X, -SO₃H, -[SO₃]X, 2H-Benzotriazol-2-yl oder 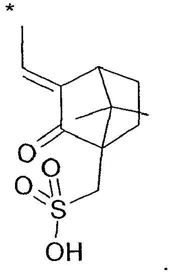 A hat eine der zuvor angegebenen Bedeutungen oder als bevorzugt angegebenen Bedeutungen.

R³ in Formel XV bedeutet besonders bevorzugt A, OA, NA₂ oder

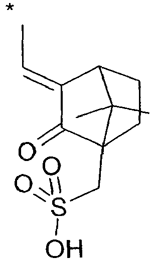

R³ in Formel XV bedeutet besonders bevorzugt A.

Weitere bevorzugte Kombinationen sind in den Ansprüchen offenbart. Bevorzugte Kombinationen sind auch Kombinationen, wobei für jede Teilformel II bis XV jeweils unabhängig voneinander die bevorzugten, besonders bevorzugten oder ganz besonders bevorzugten Bedeutungen verwendet werden.

Besonders bevorzugte Ausführungsformen des Restes R₁ sind in den folgenden Teilstrukturen zu sehen:

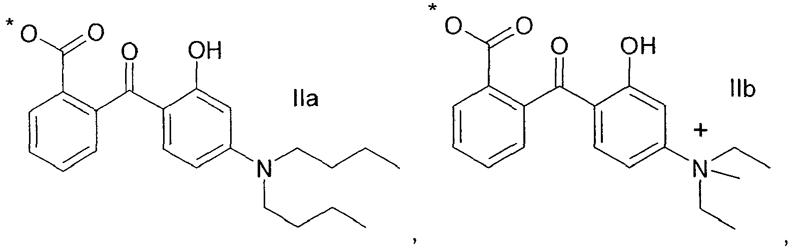

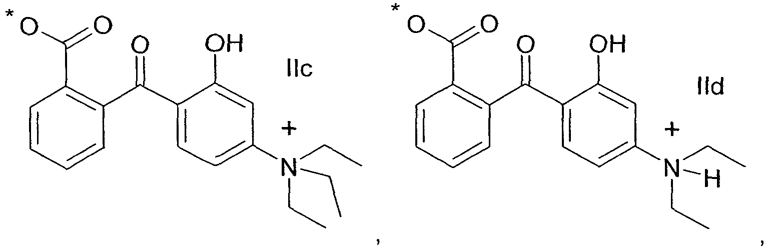

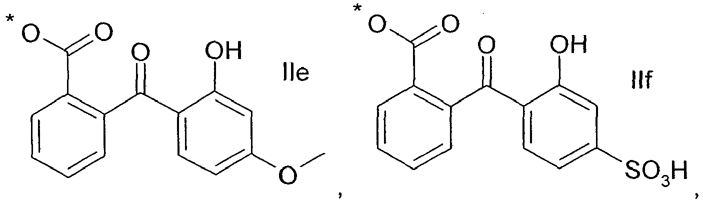

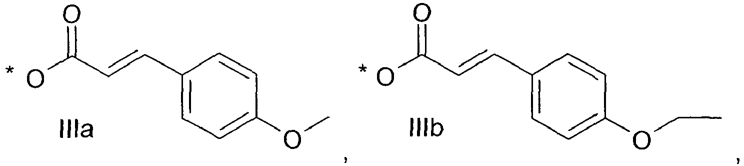

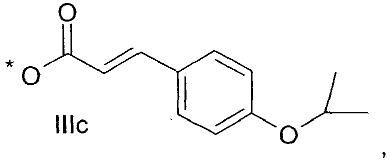

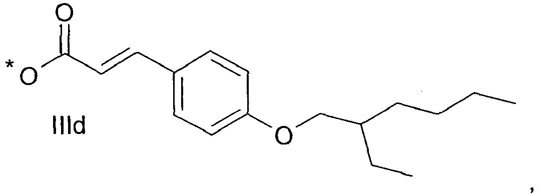

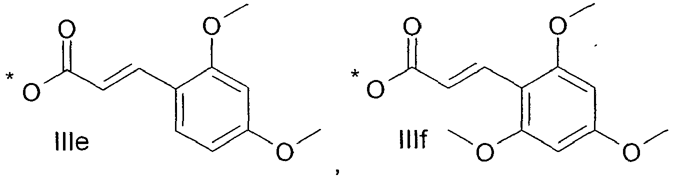

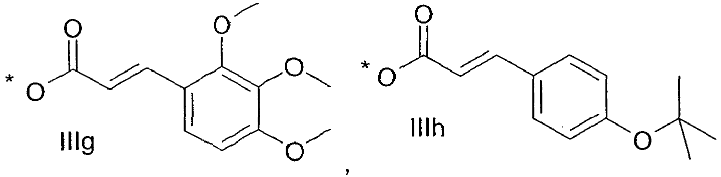

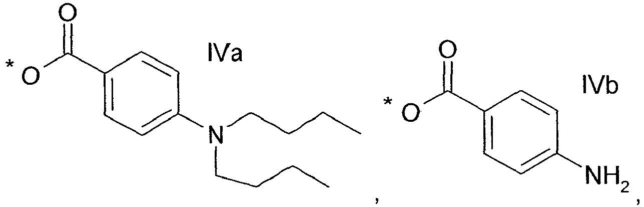

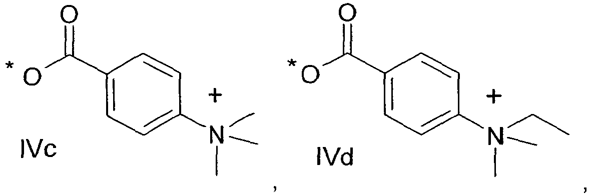

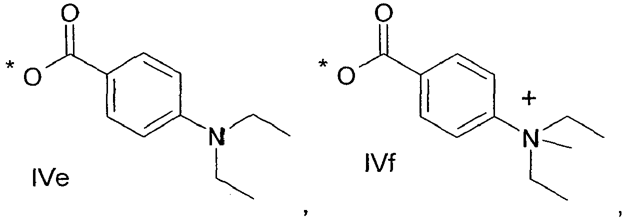

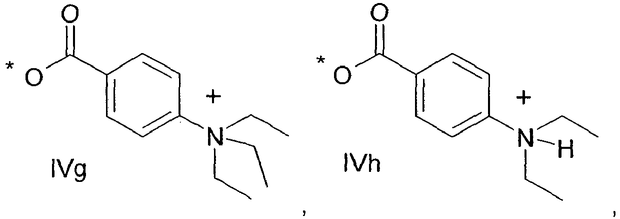

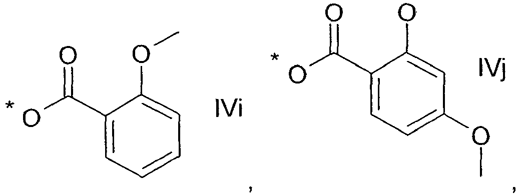

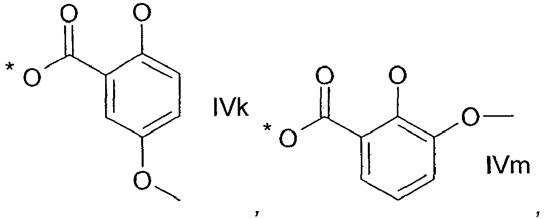

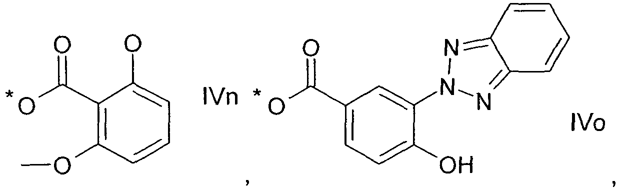

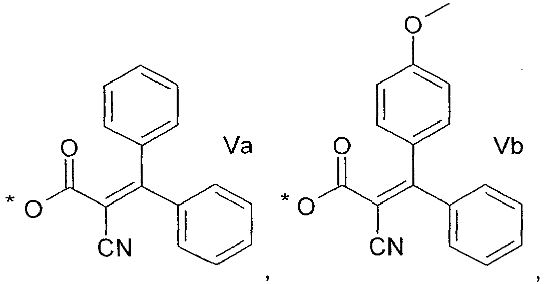

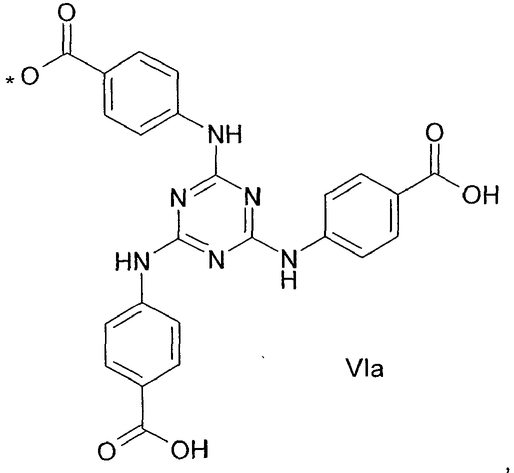

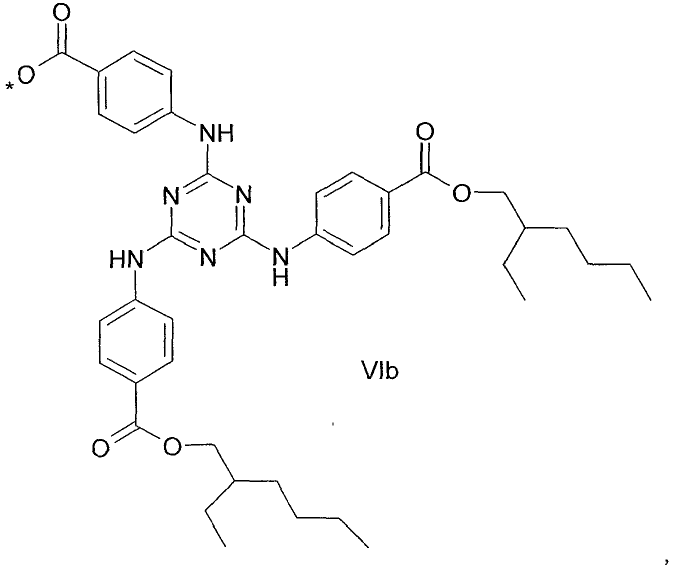

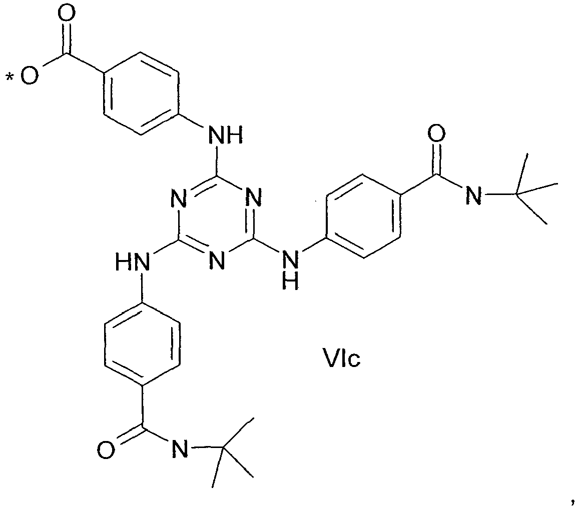

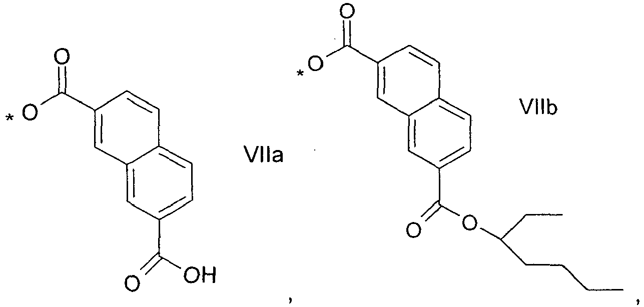

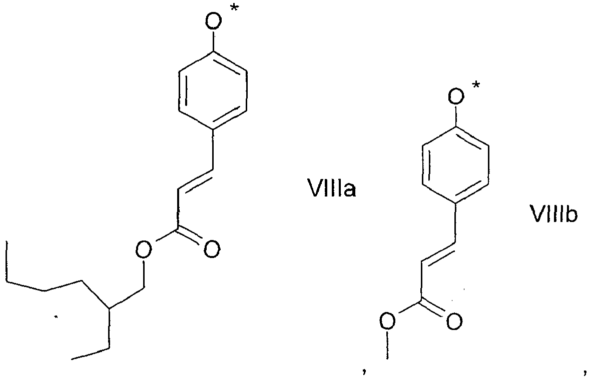

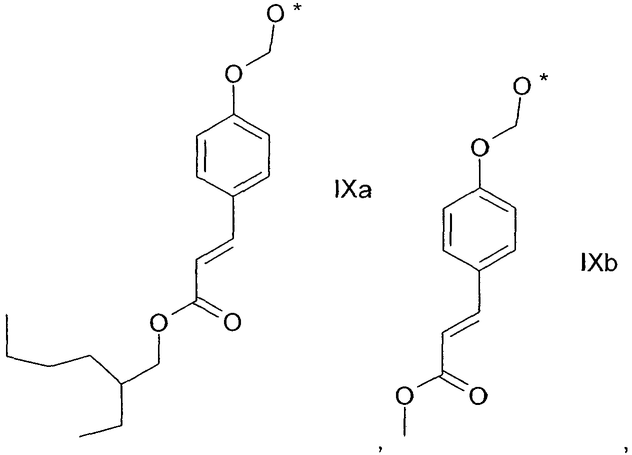

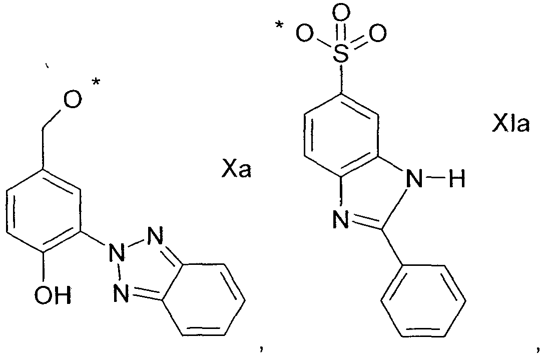

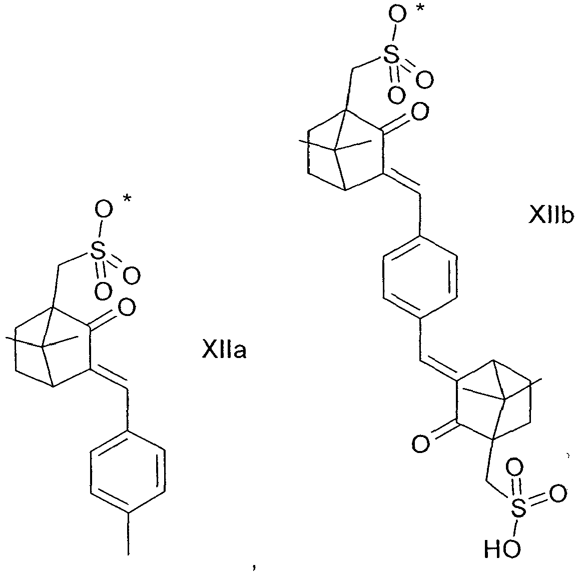

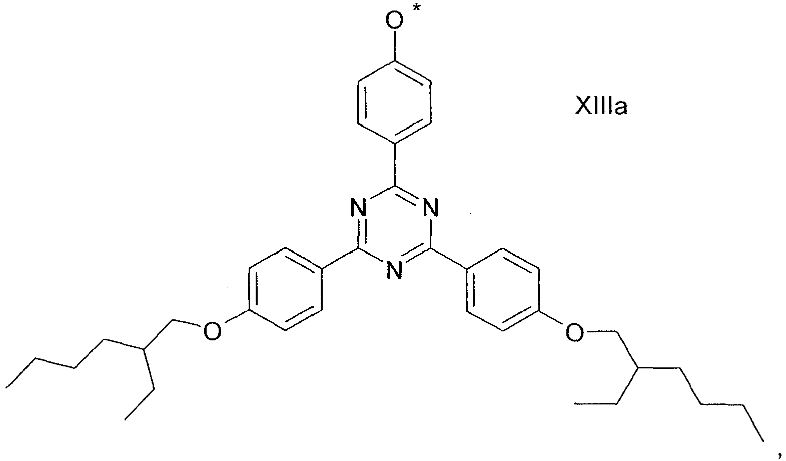

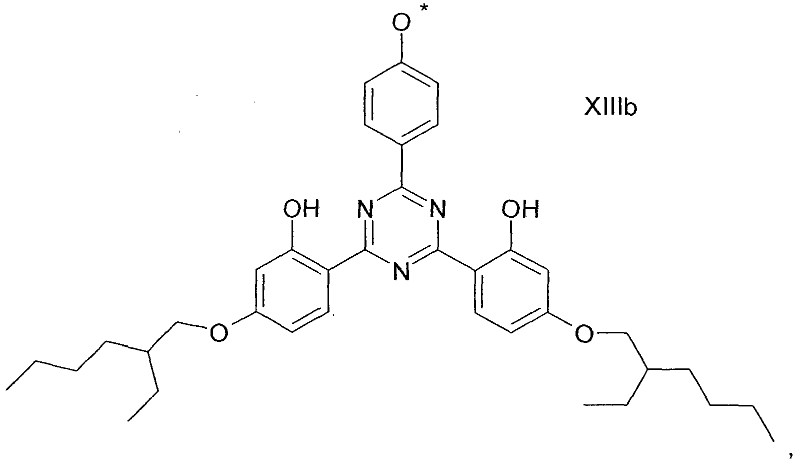

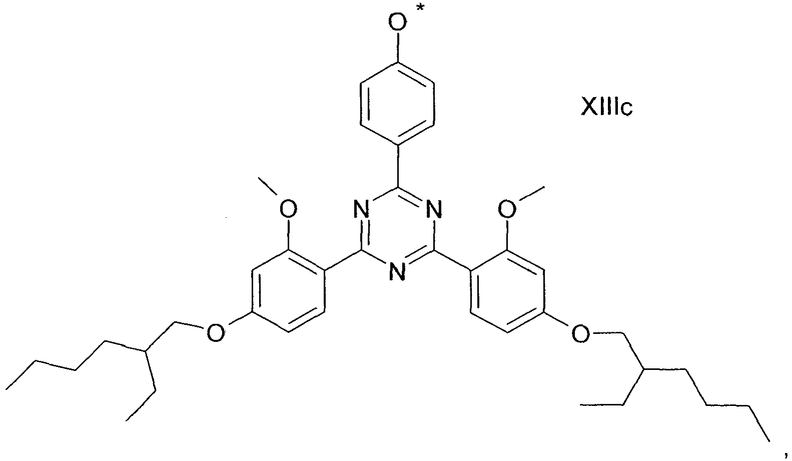

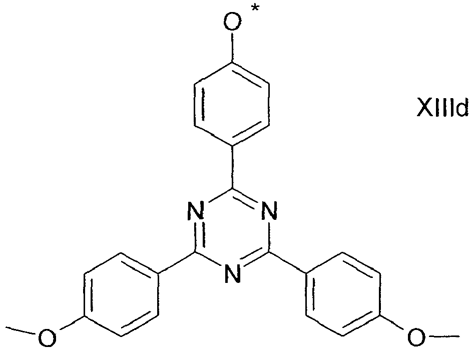

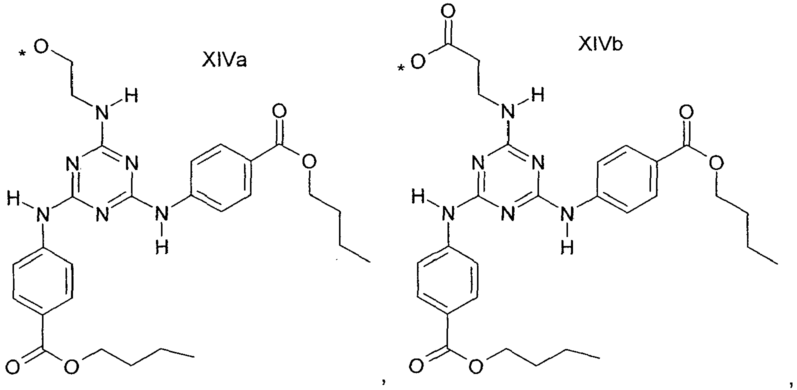

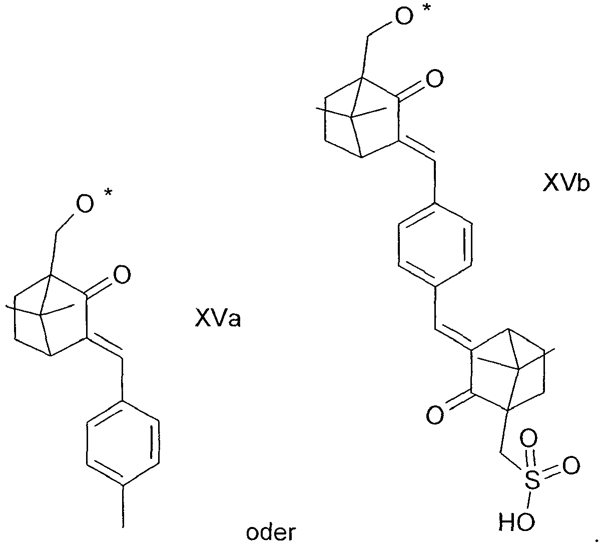

Bevorzugt ist der Rest R₁, wie zuvor beschrieben, über eine Carbonyloxyfunktion gebunden. R₁ entspricht daher bevorzugt der Formel II, III, IV, V, VI oder VII, wobei die Reste R¹ bis R¹⁴ eine der zuvor oder nachstehend genannten Bedeutungen oder bevorzugt angegebenen Bedeutungen haben oder den entsprechenden Teilformeln haben.

Ganz besonders bevorzugt entspricht R₁ der Formel II oder III, wobei R¹ bis R⁸ eine zuvor angegebene Bedeutung oder bevorzugte Bedeutung haben, insbesondere entspricht R₁ den Teilformeln IIa bis IIf und IIIa bis IIIh, ganz besonders bevorzugt der Teilformeln IIa und IIIa.

Ganz besonders bevorzugt entspricht R₁ der Formel III, wobei R¹ bis R⁵ eine zuvor angegebene Bedeutung oder bevorzugte Bedeutung haben, insbesondere den Teilformeln IIIa bis IIIh, ganz besonders bevorzugt der Teilformel IIIa.

Die Herstellung der Verbindungen der Formel I, wie zuvor beschrieben, kann dabei nach dem Fachmann an sich aus der Literatur bekannten Methoden erfolgen. Die Reaktionsbedingungen für Veresterungen oder Veretherungen sind gängiger Stand der Technik und das Auswählen der geeigneten Reaktionsbedingungen gehört zum Standardfachwissen des Synthesefachmanns.

Gegenstand der Erfindung ist ebenfalls ein Verfahren zur Herstellung von Verbindungen nach Formel I, wie zuvor beschrieben, dadurch gekennzeichnet, dass
a) eine Verbindung der Formel XVI 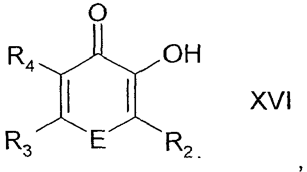 wobei E, R₂, R₃ und R₄ eine zuvor angegebene Bedeutung haben, mit einer Verbindung der Formel XVII

   R₁-M XVII

   umgesetzt wird,
   worin R₁ eine der zuvor beschriebenen Bedeutungen hat, wobei die Teilformeln XI und XII ausgeschlossen sind, und
   M Alkalimetall- oder Erdalkalimetallkation oder H bedeutet oder
b) eine Verbindung der Formel XVI 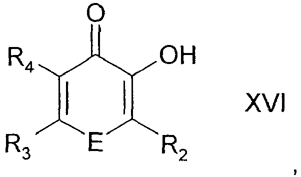 wobei E, R₂, R₃ und R₄ eine zuvor angegebene Bedeutung haben,
   mit einem Säurehalogenid oder einem Aktivester der freien Säuren, abgeleitet von den Formeln II, III, IV, V, VI, VII, XI, XII und XIV umgesetzt wird.

Die Verbindungen der Formel XVI sind zum Teil kommerziell erhältlich, beispielsweise 3-Hydroxy-2-methyl-4-pyranon oder können nach bekannten Literaturmethoden hergestellt werden, beispielweise basierend auf R. Suzuki et al, Heterocyles, 1977, 6(9-10), 1575-80 oder A. Fassihi et al, European Journal of Medicinal Chemistry, 2009, 44(5), 2145-2157).

Die Verbindungen der Formel XVII oder die freien Säuren abgeleitet von den Formeln II, III, IV, V, VI, VII, XI, XII und XIV sind zum Teil kommerziell erhältlich, beispielsweise p-Methoxyzimtsäure, p-Hydroxyzimtsäure, 2-(4-Dimethylamino-2-hydroxybenzoyl)-benzoesäure oder 2-(4-Methoxy-2-hydroxybenzoyl)-benzoesäure, oder können nach Methoden synthetisiert werden, die z.B. in den Standardwerken wie Houben-Weyl, Methoden der organischen Chemie, Georg-Thieme-Verlag, Stuttgart beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Etherbildung durch Umsetzung von Verbindungen der Formel XVII, in denen R₁ den Teilformeln VIII, IX, X, XIII, XIV oder XV entspricht, und bei denen M = H bedeutet mit einer Verbindung der Formel XVI, wie zuvor beschrieben, findet vorzugsweise in Gegenwart von Triphenylphosphin und Diisopropylazodicarboxylat, in einem inerten Lösungsmittel, z.B. einem halogenierten Kohlenwasserstoff wie Dichlormethan, einem Ether wie Tetrahydrofuran oder Dioxan, einem Amid wie DMF oder Dimethylacetamid, einem Nitril wie Acetonitril, in Dimethylsulfoxid oder in Gegenwart dieser Lösungsmittel, bei Temperaturen zwischen etwa -10 und 40, vorzugsweise zwischen 0 und 30° statt. Die Reaktionszeit liegt je nach den angewendeten Bedingungen zwischen einigen Minuten und mehreren Tagen.

Werden für die Synthese der Verbindungen der Formel I, in denen R₁ einer der Teilstrukturen II, III, IV, V, VI, VII, XI, XII und XIV entspricht, ein Säurehalogenid oder ein Aktivester der freien Säure abgeleitet von den Formeln II, III, IV, V, VI, VII, XI, XII und XIV eingesetzt,
so findet eine klassische nukleophile Substitution statt. Die Reaktionsbedingungen einer nukleophilen Substitution sind dem Synthesefachmann hinlänglich bekannt.

### Bevorzugte Säurehalogenide sind Säurechloride.

Wird eine Verbindung der Formel XVI, wie zuvor beschrieben, beispielsweise 3-Hydroxy-2-methyl-4-pyranon, mit einer Verbindung der Formel XVII umgesetzt, wobei M = H ist und R₁ vorzugsweise der Teilformel II, III, IV, V, VI, VII oder XIV entspricht, so findet die Kupplungsreaktion vorzugsweise in Gegenwart eines Dehydratisierungsmittels, z.B. eines Carbodiimids wie Dicyclohexylcarbodiimid (DCC), N-(3-Dimethylaminopropyl)-N'-ethyl-carbodiimid-hydrochlorid (EDC) oder Diisopropylcarbodiimid (DIC), ferner z.B. Propanphosphonsäureanhydrid (vgl. Angew. Chem. 1980, 92, 129), Diphenylphosphorylazid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie Tetrahydrofuran oder Dioxan, einem Amid wie DMF oder Dimethylacetamid, einem Nitril wie Acetonitril, in Dimethylsulfoxid oder in Gegenwart dieser Lösungsmittel, bei Temperaturen zwischen etwa -10 und 40, vorzugsweise zwischen 0 und 30° statt. Die Reaktionszeit liegt je nach den angewendeten Bedingungen zwischen einigen Minuten und mehreren Tagen.

Anstelle von Verbindungen der Formel XVII, wie zuvor definiert, können auch Derivate der Formel XVII, vorzugsweise eine voraktivierte Carbonsäure, oder ein Carbonsäurehalogenid, ein symmetrisches oder gemischtes Anhydrid oder ein Aktivester, eingesetzt werden. Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart) beschrieben. Aktivierte Ester werden zweckmäßig in situ gebildet, z.B. durch Zusatz von HOBt (1-Hydroxybenzotriazol) oder N-Hydroxysuccinimid.

Die Umsetzung erfolgt in der Regel in einem inerten Lösungsmittel, bei Verwendung eines Säurehalogenids in Gegenwart eines säurebindenden Mittels vorzugsweise einer organischen Base wie Triethylamin, Dimethylanilin, Pyridin, Dimethylaminopyridin oder Chinolin.

Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Die beschriebenen Verbindungen der Formel I sind in der Lage an proteinhaltige Textilien oder Textilfasern zu adsorbieren und/oder zu binden und so ihre jeweils sich nach dem Rest R₁ richtende Wirkung zu entfalten, beispielsweise UV-Schutz. Unerwünschte Materialschäden werden so reduziert.

Die erfindungsgemäßen Verbindungen der Formel I, wie zuvor beschrieben oder als bevorzugt beschrieben, sind oxidationsstabil und zeigen bei Lagerung eine reduzierte Gelbfärbung bis keine Gelbfärbung.

Die erfindungsgemäßen Verbindungen der Formel I, wie zuvor beschrieben oder als bevorzugt beschrieben, haben beispielsweise auch anti-aging-Effekte, d.h. sie dienen beispielsweise der Hautregeneration und bewirken eine Faltenreduzierung von (licht)gealteter Haut, sie erhöhen beispielsweise weiterhin die Hautreliefdichte, wirken hautstraffend oder verstärken beispielsweise die Dermis-Epidermis-Verbindung (Papillen-Index). Sie schützen die Haut vor UV-induzierten Schäden oder sie haben beispielsweise eine hautbleichende Wirkung. Sie haben beispielsweise eine antibakterielle Wirkung, d.h. sie können Schweißgeruch reduzieren oder das Hautbild bei Hautunreinheiten und/oder Akne verbessern.

Erfindungsgemäße Verbindungen der Formel I, wie zuvor beschrieben, eignen sich ebenfalls als Antiglykierungsmittel (Antiglycation agents) und wirken der Bildung von AGEs (advanced glycation endproducts) entgegen. Erfindungsgemäße Verbindungen eignen sich als Kontrastreduktionsmittel, d.h. sie können dunkle Hautstellen gegebenenfalls aufhellen bzw. helle Hautstellen gegebenenfalls abdunkeln.

Die erfindungsgemäßen Verbindungen der Formel I, wie zuvor beschrieben oder als bevorzugt beschrieben, sind in der Lage, an Haare zu binden und können so die durch UV-Licht bzw. die durch Oxidation hervorgerufenen Haarschäden insbesondere im Hinblick auf Farbe und Morphologie unterbinden. Beispielsweise kann man so vor dem Ausbleichen der Haare schützen.

Die erfindungsgemäßen Verbindungen der Formel I, wie zuvor beschrieben oder als bevorzugt beschrieben, sind in der Lage, nicht nur an stickstoffhaltige, sondern auch an schwefelhaltige Haarfunktionalitäten zu binden, wie z.B. an thiolische Gruppen.

Ein weiterer Gegenstand der vorliegenden Erfindung ist eine Zubereitung enthaltend zumindest eine Verbindung der Formel I, wie zuvor beschrieben oder als bevorzugt beschrieben oder als bevorzugte Einzelverbindungen beschrieben, wobei die Verbindungen (E)-3-(4-hydroxy-phenyl)-acrylsäure-2-methyl-4-oxo-4H-pyran-3-yl-ester oder 2-Hydroxy-benzoesäure 2-methyl-4-oxo-4H-pyran-3-yl-ester ausgenommen sind.

Ein weiterer Gegenstand der Erfindung ist eine Zubereitung enthaltend (E)-3-(4-hydroxy-phenyl)-acrylsäure-2-methyl-4-oxo-4H-pyran-3-yl-ester oder 2-Hydroxy-benzoesäure 2-methyl-4-oxo-4H-pyran-3-yl-ester, einen kosmetischen, dermatologischen oder pharmakologisch verträglichen Träger und mindestens einen weiteren Wirkstoff, ausgewählt aus der Gruppe UV-Filter, Antioxidantien, Vitamine, Anti-aging-Wirkstoffe, Anti-Cellulite-Wirkstoffe, Selbstbräunungssubstanzen, Antipigmentierstoffe oder hautaufhellender Stoffe.

Auf Grund der oberflächenadhäsiven, filmbildenden Eigenschaft erfindungsgemäßer Verbindungen können in erfindungsgemäßen Zubereitungen sowohl vor als auch bei deren Verwendung weitere in der erfindungsgemäßen Zubereitung enthaltenen Wirkstoffe vor oxidativer Zersetzung besonders gut geschützt werden. Solche weiteren im erfindungsgemäßen Mittel enthaltenen Wirkstoffe sind z.B. Farbstoffe, insbesondere Haarfarbstoffe und Haarfärbemittel, oder andere oxidationsempfindliche Substanzen, wie ungesättigte Fettsäuren, Squalen, Vitamine, Antioxidantien, Selbstbräunungsstoffe, wie z.B. Dihydroxyaceton, Retinoide, wie z.B. Retinol.

Ferner werden weitere in den erfindungsgemäßen Mitteln enthaltene Wirkstoffe auf Grund der filmbildenden Eigenschaft erfindungsgemäßer Stoffe optimal auf der Wirkoberfläche verteilt und so in ihrer Wirkleistung verstärkt.

Deshalb können zum Beispiel erfindungsgemäße Mittel als Haarbehandlungsmittel verwendet werden zur
- Vitalisierung von Haaren und/oder
- Anregung des Energiestoffwechsels in Haarfolikeln und/oder
- Aktivierung von Haarfolikeln und/oder
- Förderung oder Verstärkung des Haarwuchses und/oder
- Haarverdickung und/oder
- Behandlung von Haarausfall und/oder
- Beeinflussung der Keratinsynthese, insbesondere deren Anregung und/oder
- Verbesserung der Zugfestigkeit von keratinischen Fasern, insbesondere menschlicher Haare und/oder
- Stabilisierung des Feuchtigkeitshaushaltes von keratinischen Fasern, insbesondere menschlicher Haare und/oder
- Verbesserung der Kämmbarkeit von keratinischen Fasern, insbesondere menschlicher Haare und/oder
- Verzögerung des Alterungsprozesses von keratinischen Fasern, insbesondere menschlicher Haare und/oder
- Verbesserung der Restrukturierbarkeit von keratinischen Fasern, insbesondere menschlicher Haare und/oder
- Verringerung der Elastizitätsabnahme von keratinischen Fasern, insbesondere menschlicher Haare bei Beschädigungen durch atmosphärische Einwirkungen.

Bei der Zubereitung handelt es sich dabei üblicherweise um eine topisch anwendbare Zubereitung, beispielsweise um kosmetische oder dermatologische Formulierungen oder Medizinprodukte. Die Zubereitungen enthalten in diesem Fall einen kosmetisch oder dermatologisch geeigneten Träger und je nach gewünschtem Eigenschaftsprofil optional weitere geeignete Inhaltsstoffe. Handelt es sich um pharmazeutische Zubereitungen, so enthalten die Zubereitungen in diesem Fall einen pharmazeutisch verträglichen Träger und optional weitere pharmazeutische Wirkstoffe. Handelt e sich um Medizinprodukte, so enthalten die Zubereitungen einen für das Medizinprodukt geeigneten Träger.

Topisch anwendbar bedeutet hier, dass die Zubereitung äußerlich und örtlich angewendet wird, d.h. dass die Zubereitung geeignet sein muss, um beispielsweise auf die Haut aufgetragen werden zu können.
Bevorzugt werden die topischen Zubereitungen als kosmetische oder dermatologische Zubereitung eingesetzt, insbesondere bevorzugt als kosmetische Zubereitung.

Der Begriff Mittel wird gleichbedeutend auch als Zubereitung oder Formulierung verstanden.

Die Zubereitungen können die genannten notwendigen oder optionalen Bestandteile umfassen oder enthalten, daraus im Wesentlichen oder daraus bestehen. Alle Verbindungen oder Komponenten, die in den Zubereitungen verwendet werden können, sind entweder bekannt und käuflich erwerbbar oder können nach bekannten Verfahren synthetisiert werden.

Zubereitungen enthaltend (E)-3-(4-hydroxy-phenyl)-acrylsäure-2-methyl-4-oxo-4H-pyran-3-yl-ester oder 2-Hydroxy-benzoesäure 2-methyl-4-oxo-4H-pyran-3-yl-ester enthalten notwendigerweise einen weiteren Wirkstoff, wie zuvor beschrieben.

Die Verbindungen der Formel I oder (E)-3-(4-hydroxy-phenyl)-acrylsäure-2-methyl-4-oxo-4H-pyran-3-yl-ester oder 2-Hydroxy-benzoesäure 2-methyl-4-oxo-4H-pyran-3-yl-ester werden erfindungsgemäß typisch in Mengen von 0,01 bis 20 Gew.-%, vorzugsweise in Mengen von 0,05 Gew.-% bis 10 Gew.-% eingesetzt. Dabei bereitet es dem Fachmann keinerlei Schwierigkeiten die Mengen abhängig von der beabsichtigten Wirkung der Zubereitung entsprechend auszuwählen.
Die Funktionalisierung der Matrix bzw. der Bindungsnachweis kann anhand einiger Bindungstests überprüft werden. Diese Bindungstests werden im Beispielteil beschrieben.

Die erfindungsgemäßen Zubereitungen enthalten bevorzugt mindestens einen weiteren Wirkstoff ausgewählt aus der Gruppe UV-Filter, Antioxidantien, Vitamine, Anti-aging-Wirkstoffe, Anti-Cellulite-Wirkstoffe, Selbstbräunungssubstanzen, Antipigmentierstoffe oder hautaufhellender Stoffe.

Ein Gegenstand der Erfindung ist auch ein Verfahren zur Herstellung einer Zubereitung, wie zuvor beschrieben, wobei mindestens eine Verbindung nach Formel I, wie zuvor beschrieben oder als bevorzugt beschrieben, mit einem Träger und gegebenenfalls mit weiteren Aktiv- oder Hilfsstoffen vermischt wird. Geeignete Trägerstoffe sowie Aktiv- oder Hilfsstoffe sind im nachfolgenden Teil ausführlich beschrieben.

Das Vermischen kann ein Lösen, Emulgieren oder Dispergieren der Verbindung gemäß Formel I in dem Träger zur Folge haben.

Die Zubereitungen können neben den Verbindungen nach Formel I sowie den gegebenenfalls anderen Inhaltsstoffen weitere organische UV-Filter, sogenannte hydrophile oder lipophile Sonnenschutzfilter, die im UVA-Bereich und/oder UVB-Bereich und/oder IR und/oder VIS-Bereich (Absorber) wirksam sind, enthalten. Diese Substanzen können insbesondere unter Zimtsäurederivaten, Salicylsäurederivaten, Campherderivaten, Triazinderivaten, β,β-Diphenylacrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische wie auch anorganische UV Filter sind in den Patentanmeldungen EP-A 0 487 404 sowie WO2009/077356 angegeben. Im Folgenden werden die genannten UV-Filter meist nach der INCI-Nomenklatur benannt.

Insbesondere für eine Kombination geeignet sind:
para-Aminobenzoesäure und deren Derivate: PABA, Ethyl PABA, Ethyl dihydroxypropyl PABA, Ethylhexyl dimethyl PABA, z. B. vertrieben unter dem Namen "Escalol 507" von der Fa. ISP, Glyceryl PABA, PEG-25 PABA, z. B. vertrieben unter dem Namen "Uvinul P25" von der Fa. BASF.

Salicylate: Homosalate vertrieben unter dem Namen "Eusolex HMS" von der Fa. Merck; Ethylhexyl salicylate, z. B. vertrieben unter dem Namen "Neo Heliopan OS" von der Fa. Symrise, Dipropylene glycol salicylate, z. B. vertrieben unter dem Namen "Dipsal" von der Fa. Scher, TEA salicylate, z. B. vertrieben unter dem Namen "Neo Heliopan TS" von der Fa. Symrise.

β,β-Diphenylacrylate Derivate: Octocrylene, z. B. vertrieben unter dem Namen "Eusolex® OCR" von der Firma Merck, "Uvinul N539" von der Fa. BASF, Etocrylene, z. B. vertrieben unter dem Namen "Uvinul N35" von der BASF. Ferner z.B. Methoxycrylen, vertrieben unter dem Namen Solastay S1 von der Firma Hallstar.

Benzophenone Derivate: Benzophenone-1, z. B. vertrieben unter dem Namen "Uvinul 400"; Benzophenone-2, z. B. vertrieben unter dem Namen "Uvinul D50" ; Benzophenone-3 oder Oxybenzone, z. B. vertrieben unter dem Namen "Uvinul M40";Benzophenone-4, z. B. vertrieben unter dem Namen "Uvinul MS40"; Benzophenone-9, z. B. vertrieben unter dem Namen "Uvinul DS-49" von der Fa. BASF, Benzophenone-5, Benzophenone-6, z. B. vertrieben unter dem Namen "Helisorb 11" von der Fa. Norquay, Benzophenone-8, z. B. vertrieben unter dem Namen "Spectra-Sorb UV-24" von der Fa. American Cyanamid, Benzophenone-12 n-hexyl 2-(4-diethylamino-2-hydroxybenzoyl) benzoate oder 2-Hydroxy-4-methoxybenzophenon, vertrieben von der Fa. Merck, Darmstadt unter dem Namen Eusolex® 4360.

Benzylidencampher Derivate: 3-Benzylidenecamphor, z. B. vertrieben unter dem Namen "Mexoryl SD" von der Fa. Chimex, 4-Methylbenzylidenecamphor, z. B. vertrieben unter dem Namen "Eusolex 6300" von der Fa. Merck, Benzylidenecamphorsulfonsäure, z. B. vertrieben unter dem Namen "Mexoryl SL" von der Fa. Chimex, Camphor benzalkonium methosulfate, z. B. vertrieben unter dem Namen "Mexoryl SO" von der Fa. Chimex, Terephthalylidenedicamphorsulfonsäure, z. B. vertrieben unter dem Namen "Mexoryl SX" von der Fa Chimex, Polyacrylamidomethylbenzylidenecamphor vertrieben unter dem Namen "Mexoryl SW" von der Fa. Chimex.

Phenylbenzimidazol Derivate: Phenylbenzimidazolesulfonsäure, z. B. vertrieben unter dem Namen "Eusolex 232" von der Fa. Merck, Dinatrium phenyl dibenzimidazole tetrasulfonat, z. B. vertrieben unter dem Namen "Neo Heliopan AP" von der Fa. Symrise.

Phenylbenzotriazol Derivate: Drometrizole trisiloxane, z. B. vertrieben unter dem Namen "Silatrizole" von der Fa. Rhodia Chimie, Methylenebis(benzo-triazolyl)tetramethylbutylphenol in fester Form, z. B. vertrieben unter dem Namen "MIXXIM BB/100" von der Fa. Fairmount Chemical, oder in mikronisierter Form als wässrige Dispersion, z. B. vertrieben unter dem Namen "Tinosorb M" von der Fa. BASF.

Triazin Derivate: Ethylhexyltriazone, z. B. vertrieben unter dem Namen "Uvinul T150" von der Fa. BASF, Diethylhexylbutamidotriazone, z. B. vertrieben unter dem Namen "Uvasorb HEB" von der Fa. Sigma 3V. Weitere Triazinderivate sind exemplarisch 2,4,6-tris(diisobutyl 4'-aminobenzalmalonate)-s-triazine, oder 2,4,6-Tris-(biphenyl)-1,3,5-triazine, Butyl 4-({4-{[4-(butoxycarbonyl)phenyl]amino}-6-[(3-{1,3,3,3-tetramethyl-1-[(trimethylsilyl)oxy] isiloxanyl} propyl)amino]-1,3,5-triazin-2-yl}amino)benzoate, vertrieben unter dem namen Mexoryl SBS. Struktur von Mexoryl SBS: 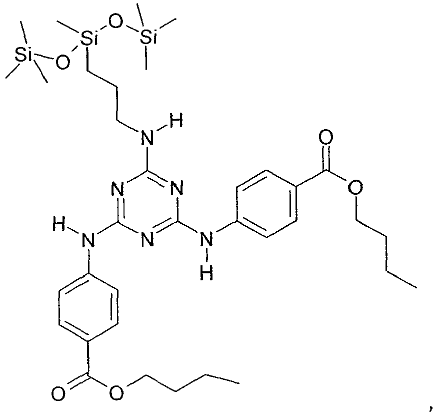 sowie Bis-ethylhexyloxyphenol methoxyphenyl triazine, z.B. vertrieben unter dem Namen Tinosorb S durch die Firma BASF.

Anthranilin Derivate: Menthyl anthranilate, z. B. vertrieben unter dem Namen "Neo Heliopan MA" von der Fa. Symrise.

Imidazol Derivate: Ethylhexyldimethoxybenzylidenedioxoimidazoline propionat.

Benzalmalonat Derivate: Polyorganosiloxane enthaltend funktionelle benzalmalonate Gruppen, wie z.B. Polysilicone-15, z. B. vertrieben unter dem Namen "Parsol SLX" von der Hoffmann LaRoche.

4,4-Diarylbutadien Derivate: 1,1-Dicarboxy(2,2'-dimethylpropyl)-4,4-diphenylbutadiene.

Benzoxazole Derivate: 2,4-bis[5-(1-dimethylpropyl)benzoxazol-2-yl(4-phenyl) imino]-6-(2-ethylhexyl)imino-1,3,5-triazine, z. B. vertrieben unter dem Namen Uvasorb K2A von der Fa. Sigma 3V und Mischungen dieses enthaltend.

### Piperazinderivate wie beispielsweise die Verbindung

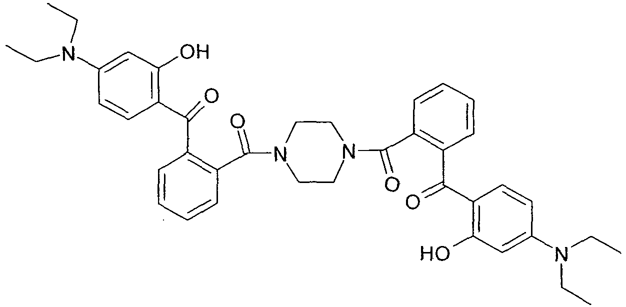

oder die UV-Filter der folgenden Strukturen 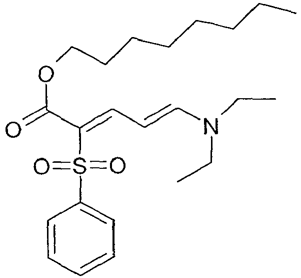 oder

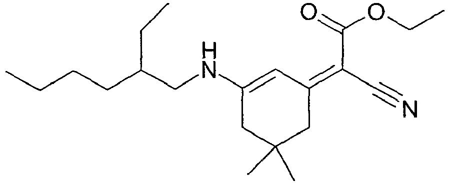

Bevorzugt kann auch mit UV-Filtern auf Basis von Polysiloxancopolymeren mit einer statistischen Verteilung gemäß nachfolgender Formel kombiniert werden, wobei z.B. a = 1,2; b= 58 und c=2,8 sind:

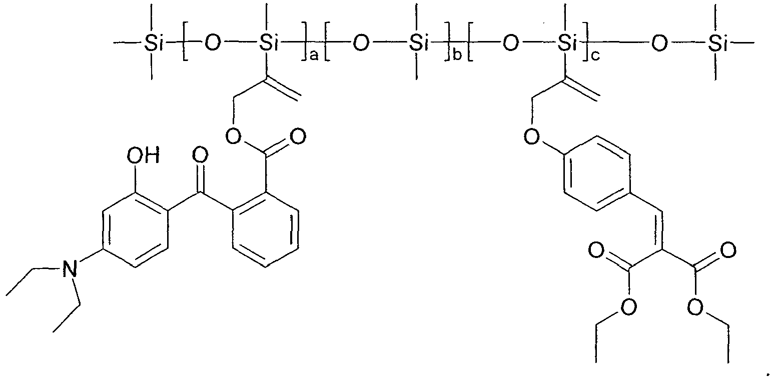

Die in der Liste aufgeführten Verbindungen sind nur als Beispiele aufzufassen. Selbstverständlich können auch andere UV-Filter verwendet werden.

Geeignete organischen UV-schützende Substanzen sind bevorzugt aus der folgenden Liste auszuwählen: Ethylhexyl salicylate,
Phenylbenzimidazolesulfonic acid, Benzophenone-3, Benzophenone-4, Benzophenone-5, n-Hexyl 2-(4-diethylamino-2-hydroxybenzoyl)benzoate, 4-Methylbenzylidenecamphor, Terephthalylidenedicamphorsulfonic acid, Disodium phenyldibenzimidazoletetrasulfonate, Methylenebis(benzotriazolyl)tetramethylbutylphenol, Ethylhexyl Triazone, Diethylhexyl Butamido Triazone, Drometrizole trisiloxane, Polysilicone-15, 1,1-Dicarboxy(2,2'-dimethylpropyl)-4,4-diphenylbutadiene, 2,4-Bis[5-1 (dimethylpropyl)benzoxazol-2-yl(4-phenyl) imino]-6-(2-ethylhexyl)imino-1,3,5-triazine und Mischungen davon.

Diese organischen UV-Filter werden in der Regel in einer Menge von 0,01 Gewichtsprozent bis 20 Gewichtsprozent, vorzugsweise 1 Gew.-% - 10 Gew.-%, in Formulierungen eingearbeitet.

Die Kombination mit organischen UV-Filtern, wie zuvor beschrieben, oder anorganischen UV-Filtern, wie nachfolgend beschrieben, ist insbesondere vorteilhaft, wenn die Verbindungen der Formel I, wie zuvor beschrieben, zum Schutz von Haut und Haar vor Photoalterung durch Licht eingesetzt werden.

Die Zubereitungen können neben den Verbindungen der Formel I sowie den gegebenenfalls anderen organischen UV-Filtern, wie zuvor beschrieben, weitere anorganische UV-Filter, sogenannte partikuläre UV-Filter enthalten.

Diese Kombinationen mit partikulären UV-Filtern sind sowohl als Pulver als auch als Dispersion oder Paste der folgenden Typen möglich.

Hierbei sind sowohl solche aus der Gruppe der Titandioxide wie z.B. gecoatetes Titandioxid (z.B. Eusolex^{®} T-2000, Eusolex^{®}T-AQUA, Eusolex^{®}T-AVO, Eusolex^{®}T-OLEO), Zinkoxide (z.B. Sachtotec^{®}), Eisenoxide oder auch Ceroxide und/oder Zirkonoxide bevorzugt.

Ferner sind auch Kombinationen mit pigmentärem Titandixoxid oder Zinkoxid möglich, wobei die Partikelgröße dieser Pigmente größer oder gleich 200 nm sind, beispielsweise Hombitan® FG oder Hombitan® FF-Pharma.

Weiter kann es bevorzugt sein, wenn die Zubereitungen anorganische UV-Filter enthalten, die mit üblichen Methoden, wie beispielsweise in Cosmetics & Toiletries, February 1990, Vol. 105, pp. 53-64 beschrieben, nachbehandelt wurden. Hierbei können eine oder mehrere der folgenden Nachbehandlungskomponenten gewählt sein: Amino Säuren, Bienenwachs, Fettsäuren, Fettsäurealkohole, anionische Tenside, Lecithin, Phospholipide, Natrium-, Kalium-, Zink-, Eisen- oder Aluminiumsalze von Fettsäuren, Polyethylene, Silikone, Proteine (besonders Collagen oder Elastin), Alkanolamine, Siliciumdioxid, Aluminiumoxid, weitere Metalloxide, Phosphate, wie Natriumhexametaphosphat oder Glycerin.

Bevorzugt einzusetzende partikuläre UV-Filter sind dabei:
- unbehandelte Titandioxide wie z.B. die Produkte Microtitanium Dioxide MT 500 B der Fa. Tayca; Titandioxd P25 der Fa. Degussa,
- Nachbehandelte mikronisierte Titandioxide mit Aluminiumoxid und Siliciumdioxid Nachbahandlung wie z.B. das Produkt "Microtitanium Dioxide MT 100 SA der Tayca; oder das Produkt "Tioveil Fin" der Fa. Uniqema,
- Nachbehandelte mikronisierte Titandioxide mit Aluminiumoxid und/oder Aluminiumstearate/laurate Nachbehandlung wie z.B. Microtitanium Dioxide MT 100 T der Fa. Tayca, Eusolex T-2000 der Firma Merck,
- Nachbehandelte mikronisierte Titandioxide mit Eisenoxid und/oder Eisenstearate Nachbehandlung wie z.B. das Produkt "Microtitanium Dioxide MT100 F" der Fa. Tayca,
- Nachbehandelte mikronisierte Titandioxide mit Siliciumdioxide, Aluminiumoxid und Silicon Nachbehandlung wie z.B. das Produkt "Microtitanium Dioxide MT 100 SAS",der Fa. Tayca,
- Nachbehandelte mikronisierte Titandioxide mit Natrumhexametaphosphate, wie z.B. das Produkt "Microtitanium Dioxide MT 150 W" der Fa. Tayca.

Die zur Kombination einzusetzenden behandelten mikronisierten Titandioxide können auch nachbehandelt sein mit:
- Octyltrimethoxysilane; wie z.B. das Produkt Tego Sun T 805 der Fa. Degussa,
- Siliciumdioxid; wie z.B. das Produkt Parsol T-X der Fa. DSM,
- Aluminiumoxid und Stearinsäure; wie z.B. das Produkt UV-Titan M160 der Fa. Sachtleben,
- Aluminium und Glycerin; wie z.B. das Produkt UV-Titan der Fa. Sachtleben,
- Aluminium und Silikonölen, wie z.B. das Produkt UV-Titan M262 der Fa. Sachtleben,
- Natriumhexamethaphosphat und Polyvinylpyrrolidon,
- Polydimethylsiloxane, wie z.B. das Produkt 70250 Cardre UF TiO2SI3" der Fa. Cardre,
- Polydimethylhydrogensiloxane, wie z.B. das Produkt Microtitanium Dioxide USP Grade Hydrophobic" der Fa. Color Techniques.

Ferner kann auch die Kombination mit folgenden Produkten vorteilhaft sein:
- Unbehandelte Zinkoxide wie z. B. das Produkt Z-Cote der Fa. BASF (Sunsmart), Nanox der Fa. Elementis
- Nachbehandelte Zinkoxide wie z.B die folgenden Produkte:
   ∘ "Zinc Oxide CS-5" der Fa. Toshibi (ZnO nachbehandelt mit polymethylhydrogenosiloxane)
   ∘ Nanogard Zinc Oxide FN der Fa. Nanophase Technofogies
   ∘ "SPD-Z1" der Fa Shin-Etsu (ZnO nachbehandelt mit einem Silikongepfropften Acrylpolymer, dispergiert in Cyclodimethylsiloxane
   ∘ "Escalol Z100" der Fa ISP (Aluminiumoxid nachbehandeltes ZnO dispergiert in einer ethylhexyl methoxycinnamate/PVPhexadecene/methicone copolymer Mischung)
   o "Fuji ZNO-SMS-10" der Fa. Fuji Pigment (ZnO nachbehandelt mit Siliciumdioxid und Polymethylsilesquioxan);
   o Unbehandeltes Ceroxide Mikropigment z.B. mit der Bezeichnung "Colloidal Cerium Oxide" der Fa Rhone Poulenc
   ∘ Unbehandelte und/oder nachbehandelte Eisenoxide mit der Bezeichnung Nanogar der Fa. Arnaud.

Beispielhaft können auch Mischungen verschiedener Metalloxide , wie z.B. Titandioxid und Ceroxid mit und ohne Nachbenhandlung eingesetzt werden, wie z.B. das Produkt Sunveil A der Fa. Ikeda. Außerdem können auch Mischungen von Aluminiumoxid, Siliciumdioxid und Silikonnachbehandelten Titandioxid. Zinkoxid-Mischungen wie z.B. das Produkt UV-Titan M261 der Fa. Sachtleben in Kombination mit dem erfindungsgemäßen UV-Schutzmittel verwendet werden.

Diese anorganischen UV-Filter werden in der Regel in einer Menge von 0,1 Gewichtsprozent bis 25 Gewichtsprozent, vorzugsweise 2 Gew.-% - 10 Gew.-%, in die Zubereitungen eingearbeitet.

Durch Kombination von einer oder mehrerer der genannten Verbindungen mit UV-Filterwirkung kann die Schutzwirkung gegen schädliche Einwirkungen der UV-Strahlung optimiert werden.

Alle genannten UV-Filter können auch in verkapselter Form eingesetzt werden. Insbesondere ist es von Vorteil organische UV-Filter in verkapselter Form einzusetzen. Daher kann es bevorzugt sein, wenn ein oder mehrere der oben genannten UV-Filter in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Die schützende Wirkung gegen oxidativen Stress bzw. gegen die Einwirkung von Radikalen kann jedoch weiter verbessert werden, wenn die erfindungsgemäßen Mittel oder Zubereitungen ein oder mehrere weitere Antioxidantien enthalten, wobei es dem Fachmann keinerlei Schwierigkeiten bereitet geeignet schnell oder zeitverzögert wirkende Antioxidantien auszuwählen.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathionin-sulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid).
Ein weiteres Antioxidanz bzw. auch ein Chelatbildner ist Pentasodium ethylenediamine tetramethylene phosphonate.

Geeignete Antioxidantien sind auch Verbindungen der allgemeinen Formeln A oder B 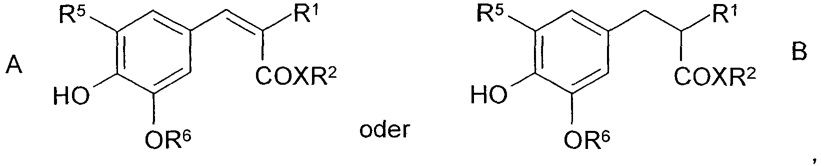 worin
R¹ aus der Gruppe -C(O)CH₃, -CO₂R³, -C(O)NH₂ und -C(O)N(R⁴)₂ ausgewählt werden kann,
X O oder NH,
R² lineares oder verzweigtes Alkyl mit 1 bis 30 C-Atomen,
R³ lineares oder verzweigtes Alkyl mit 1 bis 20 C-Atomen,
R⁴ jeweils ungabhängig voneinander H oder lineares oder verzweigtes Alkyl mit 1 bis 8 C-Atomen,
R⁵ lineares oder verzweigtes Alkyl mit 1 bis 8 C-Atomen oder lineares oder verzweigtes Alkoxy mit 1 bis 8 C-Atomen und
R⁶ lineares oder verzweigtes Alkyl mit 1 bis 8 C-Atomen bedeutet, vorzugsweise Derivate der 2-(4-Hydroxy-3,5-dimethoxybenzyliden)-malonsäure und/oder 2-(4-Hydroxy-3,5-dimethoxybenzyl)-malonsäure, besonders bevorzugt 2-(4-Hydroxy-3,5-dimethoxybenzyliden)-malonsäure-bis-(2-ethylhexyl)ester (z.B. Oxynex^{®} ST Liquid) und/oder 2-(4-Hydroxy-3,5-dimethoxybenzy)-malonsäure-bis-(2-ethylhexyl)ester (z.B. RonaCare^{®} AP).

Mischungen von Antioxidantien sind ebenfalls zur Verwendung in den erfindungsgemäßen Mitteln oder Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex^{®} AP), natürliche Tocopherole, L-(+)-Ascorbylpalmitat, L-(+)-Ascorbinsäure und Zitronensäure (z.B. Oxynex^{®} K LIQUID), Tocopherolextrakte aus natürlichen Quellen, L-(+)-Ascorbylpalmitat, L-(+)-Ascorbinsäure und Zitronensäure (z.B. Oxynex^{®} L LIQUID), DL-α-Tocopherol, L-(+)-Ascorbylpalmitat, Zitronensäure und Lecithin (z.B. Oxynex^{®} LM) oder Butylhydroxytoluol (BHT), L-(+)-Ascorbylpalmitat und Zitronensäure (z.B. Oxynex^{®} 2004). Derartige Antioxidantien werden mit Verbindungen der Formel I in solchen Zusammensetzungen überlicherweise in Verhältnissen im Bereich von 1000:1 bis 1:1000, bevorzugt in Mengen von 100:1 bis 1:100 eingesetzt.

In den erfindungsgemäßen Mitteln oder Zubereitungen können als weitere Inhaltsstoffe Vitamine enthalten sein. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂) in den erfindungsgemäßen kosmetischen Zubereitungen enthalten, insbesondere bevorzugt Retinol, Nicotinsäureamid, Vitamin-A-Palmitat, Vitamin C und dessen Derivaten, DL-α-Tocopherol, Tocopherol-E-Acetat, Nicotinsäure, Pantothensäure und Biotin, ganz besonders bevorzugt Retinol oder Nicotinsäureamid. Vitamine werden dabei mit Verbindungen der Formel I überlicherweise in Verhältnissen im Bereich von 1000:1 bis 1:1000, bevorzugt in Mengen von 100:1 bis 1:100 eingesetzt.

Geeignete anti-aging Wirkstoffe, insbesondere für hautpflegende Zubereitungen, sind vorzugsweise sogenannte kompatible Solute. Vorzugsweise werden als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N.-Acetylornithin, Trimethylamine-N-oxid Di-myo-inositol-phosphat (DIP), cyclisches 2,3-diphosphoglycerat (cDPG), 1,1- Diglycerin-Phosphat (DGP), ß-Mannosylglycerat (Firoin), ß- Mannosylglyceramid (Firoin-A) oder/und Dimannosyl-di-inositolphosphat (DMIP) oder ein optisches Isomer, Derivat, z.B. eine Säure, ein Salz oder Ester dieser Verbindungen oder Kombinationen davon eingesetzt.

Besonders bevorzugte Anti-aging-Wirkstoffe sind Pyrimidincarbonsäuren, Aryloxime, Bioflavonoide, bioflavonoidhaltige Extrakte, Chromone oder Retinoide.

Bekannte Bioflavonoide sind beispielsweise Troxerutin, Tilirosid, α-Glucosylrutin, Rutin oder Isoquercetin, wobei die genannte Auswahl nicht beschränkend wirken soll.

Bioflavonoidhaltige Extrakte sind beispielsweise Gingko Biloba oder Emblica.

Bekannte Anti-aging-Stoffe sind auch Chromone, wie beispielsweise in EP 1508327 beschrieben oder Retinoide, beispielsweise Retinol (Vitamin A), Retinsäure, Retinaldehyd oder auch synthetisch modifizierte Verbindungen von Vitamin A.

Die beschriebenen Chromone und Retinoide sind gleichzeitig auch wirksame Anti-Cellulite-Wirkstoffe. Ein ebenfalls bekannter Anti-Cellulite-Wirkstoff ist Koffein.

Geeignete anti-aging Wirkstoffe sind beispielsweise die von der Firma Merck vertriebenen Produkte 5,7-Dihydroxy-2-methyl-chromon, vermarktet unter dem Handelsnamen RonaCare®Luremine oder die Produkte Ronacare®Isoquercetin, Ronacare®Tilirosid oder Ronacare®Cyclopeptide-5.

Ferner können die erfindungsgemäßen Zubereitungen mindestens einen Selbstbräuner als weiteren Inhaltsstoff enthalten.
Als vorteilhafte Selbstbräuner können unter anderem eingesetzt werden: 1,3-Dihydroxyaceton, Glycerolaldehyd, Hydroxymethylglyoxal, γ-Dialdehyd, Erythrulose, 6-Aldo-D-Fructose, Ninhydrin, 5-Hydroxy-1,4-naphtochinon (Juglon) oder 2-Hydroxy-1,4-naphtochinon (Lawson). Ganz besonders bevorzugt ist das 1,3-Dihydroxyaceton, Erythrulose oder deren Kombination.

Zubereitungen mit Selbstbräunereigenschaften, insbesondere solche, die Dihydroxyaceton enthalten, neigen bei der Anwendung auf der menschlichen Haut zu Fehlgerüchen, die vermutlich durch Abbauprodukte des Dihydroxyacetons selbst oder durch Produkte von Nebenreaktionen verursacht werden und die von den Anwendern teilweise als unangenehm empfunden werden. Es hat sich gezeigt, dass diese Fehlgerüche bei Verwendung von Formaldehydfängern und/oder Flavonoiden vermieden werden. Daher kann die erfindungsgemäße Zubereitung enthaltend mindestens einen Selbstbräuner vorzugsweise auch Formaldehydfänger sowie gegebenenfalls Flavonoide zur Verbesserung des Geruches enthalten.

Die Zubereitungen können auch ein oder mehrere weitere hautaufhellende Wirkstoffe oder synonym Antipigmentierungswirkstoffe enthalten. Hautaufhellende Wirkstoffe können prinzipiell alle dem Fachmann bekannten Wirkstoffe sein. Beispiele von Verbindungen mit hautaufhellender Aktivität sind Hydrochinon, Kojisäure, Arbutin, Aloesin, Niacinamide, Azelainsäure, Elaginsäure, Tranexamsäure, Kalium 4-Methoxysalicylat, Maulbeerbaumextrakt, Magnesium-ascorbyl-phosphat, Süßholzwurzelextrakt, Emblica, Ascorbinsäure oder Rucinol sowie Substanzen wie unter WO2007121845 beschrieben Durch solche Zubereitungen kann beispielsweise der Hautkontrast zwischen Hell- und Dunkelpartien reduziert werden. Die Haut erscheint somit homogener gefärbt. Das Erscheinungsbild ist jünger als das stark kontrastierter Haut.

Geeignet sind Zubereitungen für eine äußerliche Anwendung, beispielsweise als Creme oder Milch (O/W, W/O, O/W/O, W/O/W), als Lotion oder Emulsion, in Form ölig-alkoholischer, ölig-wässriger oder wässrigalkoholischer Gele bzw. Lösungen auf die Haut aufgesprüht werden kann. Sie können als feste Stifte vorliegen oder als Aerosol konfektioniert sein. Für eine innerliche Anwendung sind Darreichungsformeln wie Kapseln, Dragees, Pulver, Tabletten-Lösungen oder Lösungen geeignet.

Vorzuziehende Hilfsstoffe stammen aus der Gruppe der Konservierungsstoffe, Stabilisatoren, Lösungsvermittler, Färbemittel, Geruchsverbesserer. Als Konservierungsstoffe werden bevorzugt zugelassene Konservierungsstoffe verwendet, die in der Kosmetikverordnung, Anlage 6 als Positivliste aufgeführt sind oder auch antimikrobiellen Pigmente, wie beispielsweise in WO 2004/0092283 oder WO 2004/091567 beschrieben.

Geeignete Konservierungsstoffe sind daher auch Alkylester der p-Hydroxybenzoesäure, Hydantoinderivate, Propionat-Salze oder eine Vielzahl von Ammoniumverbindungen.

Ganz besonders bevorzugte Konservierungsstoffe sind Methylparaben, Propylparaben, Imidazolidinyl-Harnstoff, Natrium-dehydroxyacetat oder Benzylalkohol. Konservierungsmittel werden in Mengen zwischen 0.5 bis 2 Gew% eingesetzt.

Emollients oder Weichmacher werden oft in kosmetische Zubereitungen eingearbeitet. Sie werden bevorzugt in 0.5 bis 50 Gew%, bevorzugt zwischen 5 und 30 Gew% bezogen auf die Gesamtzusammensetzung eingesetzt. Generell können Weichmacher in Klassen eingeordnet werden, wie beispielsweise die Kategorie der Ester, Fettsäuren oder Fettalkohole, Polyole, Kohlenwasserstoffe und Öle enthaltend mindestens eine Amidstruktur-Einheit.

Repräsentative Öle enthaltend mindestens eine Amidstruktur-Einheit zusammen mit ihrer Synthese sind insbesondere in EP 1044676 und EP 0928608 beschrieben. Eine besonders bevorzugt angegebene Verbindung ist Isopropyl-N-lauroylsarcosinat, welches unter der Produktbezeichnung Eldew SL-205 von Ajinomoto kommerziell erhältlich ist.

Beispielhafte Kohlenwasserstoffe als Weichmacher sind Verbindungen, die generell 12 bis 30 C-Atome haben. Spezielle Beispiele sind Arylalkylbenzoate, Alkylbenzoate, Mineralöle, Vaseline, Squalene oder Isoparaffine.

Weitere Emollients oder Hydrophobiermittel sind bevorzugt C₁₂ bis C₁₅ Alkylbenzoate, Dioctyladipat, Octylstearat, Octyldodecanol, Hexyllaurat, Octyldodecyl-neopentanoat, Cyclomethicone, Dicapryl-ether, Dimethicone, Phenyl-trimethicone, Isopropyl-myristat, Capriylic/Capric-glyceride, Propylenglycol-dicaprylat/dicaprat oder Decyl-oleat.

Eine weitere Kategorie funktioneller Inhaltsstoffe von kosmetischen Zubereitungen im Sinne der Erfindung sind Verdickungsmittel. Verdickungsmittel werden in der Regel in Mengen zwischen 0.1 bis 20 Gew.-%, vorzugsweise zwischen 0.5 bis 10 Gew.-% bezogen auf die Gesamtmenge eingesetzt. Beispielhaft für diese Verbindungen sind vernetzte Polyacrylat-Materialien, kommeziell erhältlich under der Marke Carbopol von B. F. Goodrich Company. Verwendet werden können auch Verdickungsmittel, wie Xanthan-Gum, Carrageenan-Gum, Gelatin-Gum, Karayagummi, Pectin-Gum oder Johannisbrotkernmehl.

Unter gewissen Umständen ist es möglich, dass eine Verbindung sowohl ein Verdickungsmittel als auch ein Weichmacher sein kann. Beispiele hierfür sind Silicon-Gums (kinematische Viskosität > 10 Centistokes), Ester wie beispielsweise Glycerolstearat oder Cellulosederivate, beispielsweise Hydroxypropylcellulose.

Salben, Pasten, Cremes und Gele können die üblichen Trägerstoffe enthalten, die für die topische Verabreichung geeignet sind, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen leichtflüchtigen, verflüssigten Treibmittel, z.B. Chlorfluorkohlenwasserstoffe, Propan/Butan oder Dimethylether, enthalten. Auch Druckluft ist vorteilhaft zu verwenden.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Ein bevorzugter Lösungsvermittler generell ist 2-Isopropyl-5-methyl-cyclohexancarbonyl-D-Alaninmethylester.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle, beispielsweise Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Puder-, Emulsions- und Wachs-Make up sowie Sonnenschutz-, Prä-Sun- und After-Sun-Präparate.

Zu den bevorzugten Zubereitungsformen gehören insbesondere auch Emulsionen.

Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Die Zubereitungen können in bevorzugter Weise Hilfsstoffe enthalten, wie beispielsweise kosmetische Öle (z.B. Caprylic/Capric Triglycerides, C12-15 Alkyl Benzoate, Isopropylmyristat, Arylalkyl Benzoate wie z.B. Phenethylbenzoat (X-Tend 226), Isopropyl N-dodecanoyl-N-methylglycinate (Eldew SL 205), Phytosteryl otyldodecyl lauroyl glutamate (Eldew PS 203) oder Ölkomponenten der Marke Cosmacol wie Dimyristyl Tartrate, Tri C14-C15 Alkyl Citrate, C12-C13 Alkyl Lactate, Tridecyl Salicylate, C12-C13 Alkyl Octanoate, C12-C13 Alkyl Malate, C12-C13 Alkyl Citrate, C12-C13 Alkyl Tartrate), oder polarprotische Hilfsstoffe (z.B. Propylenglycol, Glycerin, Isopropanol, Ethanol) oder sog. Lösungsvermittler (z.B. Butylphthalimide, Isopropylphthalimide, Dimethylisosorbide).

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Die wässrige Phase der einzusetzenden Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylen-glykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Emulsionen sind vorteilhaft und enthalten z. B. die genannten Fette, Öle, Wachse und anderen Fettkörper, sowie Wasser und einen Emulgator, wie er üblicherweise für einen solchen Typ der Formulierung verwendet wird.

In einer bevorzugten Ausführungsform enthalten die einzusetzenden Zubereitungen hydrophile Tenside. Die hydrophilen Tenside werden bevorzugt gewählt aus der Gruppe der Alkylglucoside, der Acyllactylate, der Betaine sowie der Cocoamphoacetate.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren^{®} 1200 (Henkel KGaA), Oramix^{®} NS 10 (Seppic).

Die kosmetischen und dermatologischen Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-A-43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-Emulgatoren in den bevorzugten O/W-Emulsionen zu verwenden.

Vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen.

Es ist ferner von Vorteil, die Fettsäureethoxylate ausfolgender Gruppe zu wählen:
Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,

### Polyethylenglycol(20)oleat.

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)-glyceryllaurat, Polyethylenglycol(22)glyceryllaurat, Polyethylenglycol-(23)glyceryllaurat, Polyethylenglycol(6)glycerylcaprat/cprinat, Polyethylen-glycol(20)glyceryloleat, Polyethylenglycol(20)glycerylisostearat, Polyethylenglycol(18)glyceryloleat(cocoat) zu wählen.

Es ist ebenfalls günstig, die Sorbitanester aus der Gruppe Polyethylen-glycol(20)sorbitanmonolaurat, Polyethylenglycol(20)sorbitanmonostearat, Polyethylenglycol(20)sorbitanmonoisostearat, Polyethylenglycol(20)sorbi-tanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:
Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkohole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat oder PEG-30-dipolyhydroxystearat.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform ist eine Emulsion, welche als Schutzcreme oder -milch vorliegt und beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfasst. Die öligalkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane.

Ein weiterer Gegenstand der Erfindung ist auch die Verwendung der Struktureinheit der Formel I-1 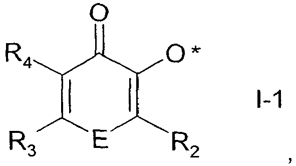 worin R₂, R₃, R₄ und E eine bei den Verbindungen der Formel I angegebene Bedeutung oder eine bevorzugt angegebene Bedeutung haben, als Linker zur Funktionalisierung von proteinhaltigen Matrices, wobei das Symbol* die Anknüpfungsstelle in Form einer kovalenten Einfachbindung zu einem Molekül bedeutet, welches an die Matrix adsorbiert oder kovalent gebunden werden soll.

Wesentlich für die Adhäsion und/oder Bindung der Verbindungen der Formel I, wie zuvor beschrieben, ist die Struktureinheit der Formel I-1. Die Natur der kovalenten Einfachbindung, mit der das zu adsorbierende oder zu bindende Molekül an den Linker gebunden ist, ist unabhängig für die Adhäsion und/oder Bindung an die proteinhaltige Matrix. Durch diesen Linker der Formel I-1 erhalten derartige Moleküle, beispielsweise Wirkstoffe, eine oberflächenadhäsive filmbildende Eigenschaft und können so auf geeignete Oberflächen sehr gleichmäßig aufziehen. So können beispielsweise Wirkstoffe vielfältiger Struktur an die proteinhaltige Matrix, insbesondere an die Haut, Haar und/oder Nägel, gebunden werden. Dies führt beispielsweise zu einer Immobilisierung der gewünschten Wirkstoffe, beispielsweise von UV-Absorbern, UV-Filtern, Farbstoffen wie z.B. Fluoreszenzfarbstoffen zur Gewebemarkierung, oder ermöglicht eine kontrollierte Wirkstoffabgabe, beispielsweise für pharmakologische, antimikrobielle, fungizide, herbizide, insektizide oder kosmetische Wirkstoffe oder für Röntgenkontrastmittel.

Durch diesen Linker der Formel I-1 erhalten derartige Moleküle, beispielsweise die Klasse der organischen UV-Absorber oder UV-Filter, ebenfalls eine amphiphile Eigenschaft und besitzen dadurch eine Oberflächenaktivität, die sie zwar nicht als ein Tensid kennzeichnet, die aber die Wirkstoffe zu einem Eigenschaftsprofil verhilft, welches von dem Eigenschaftsprofil klassischer, insbesondere öllöslicher, organischer UV-Absorber oder UV-Filter abweicht.

Auch ohne weitere Ausführungen wird davon ausgegangen, dass ein Fachmann die obige Beschreibung in weitestem Umfang nutzen kann. Die bevorzugten Ausführungsformen und Beispiele sind deswegen lediglich als beschreibende, keinesfalls als in irgendeiner Weise limitierende Offenbarung aufzufassen. Die vollständige Offenbarung aller vor- und nachstehend aufgeführten Anmeldungen und Veröffentlichungen sind durch Bezugnahme in diese Anmeldung eingeführt. Die Gewichtsprozentverhältnisse der einzelnen Inhaltsstoffe in den Zubereitungen der Beispiele gehören ausdrücklich zur Offenbarung der Beschreibung und können daher als Merkmale herangezogen werden.

Weitere wichtige Merkmale und Vorteile der Erfindung ergeben sich aus den Unteransprüchen und aus den Beispielen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Bevorzugte Ausführungsformen der Erfindung sind in den Beispielen dargestellt und werden in der nachfolgenden Beschreibung näher erläutert, ohne den Umfang der vorliegenden Erfindung zu beschränken.

### Beispiele:

### Beispiel 1:

### Synthese von 2-Methyl-4-oxo-4H-pyran-3-yl-2-(4-dibutytamino-2-hydroxy-benzoyl)-benzoesäureester

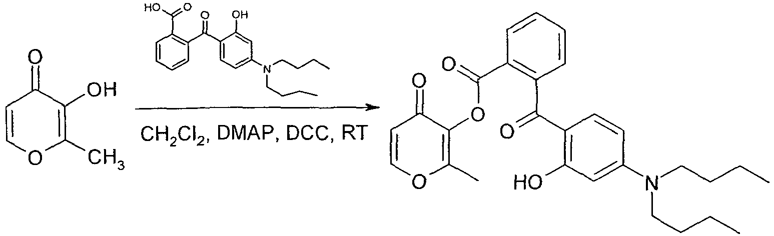

15,0g 2-(4-Dibutylamino-2-hydroxy-benzoyl)-benzoesäure, 6,1 g 3-Hydroxy-2-methyl-4-pyranon und 0,35 g 2-(Dimethylamino)-pyridin werden unter Argon in 120 mL Dichlormethan vorgelegt. Zu dieser Lösung tropft man eine Lösung von 10,0 g N,N'-Dicyclohexylcarbodiimid (DCC) in 30 mL Dichlormethan und rührt 2 Tage bei Raumtemperatur. Zur Eliminierung überschüssigen DCCs gibt man nun 4,4 g Oxalsäuredihydrat und lässt für 2,5 h bei Raumtemperatur nachrühren. Die Reaktionslösung wird für 30 min im Eiswasserbad gekühlt und anschließend abfiltriert. Die Mutterlauge wird nun mit 100 mL Wasser versetzt und extrahiert. Die organische Phase wird anschließend nochmals mit Wasser, dann mit gesättigter NaCl-Lösung nachgewaschen, mit Na₂SO₄ getrocknet, filtriert und einrotiert.
Man erhält nach Wiederaufnahme in Essigsäureethylester und Aufreinigung der Lösung an Kieselgel 7,9 g 2-Methyl-4-oxo-4H-pyran-3-yl-2-(4-dibutylamino-2-hydroxy-benzoyl)-benzoesäureester.
¹³C-NMR (300 MHz, D₆-DMSO): ):δ [ppm] = 13,7; 14,3; 19,5; 28,8; 49,9; 96,5; 104,4; 108,9; 115,8; 126,6; 128,2; 129,8; 130,6; 133,3; 134,2; 137,7; 140,5; 154,2; 156,0; 159,2; 162,4; 164,7; 170,7; 186,5.

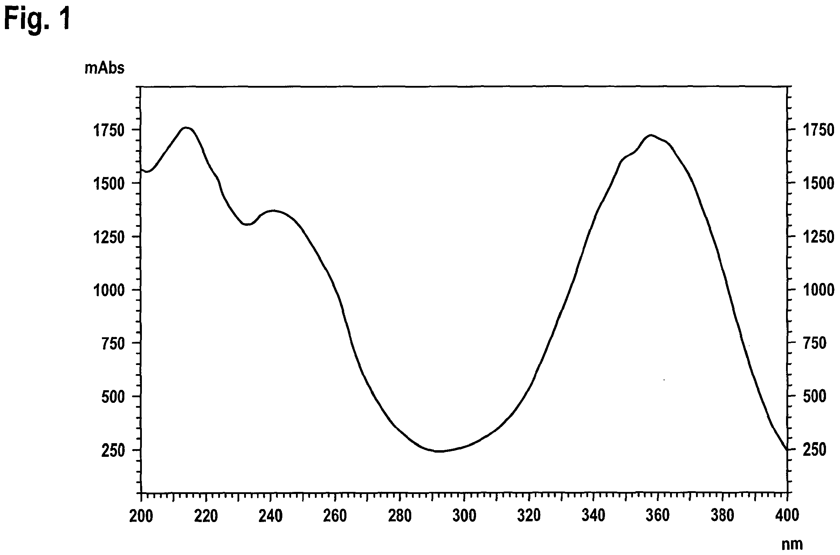

Figur 1 zeigt das UV-Spektrum (200 bis 400nm) der Verbindung 2-Methyl-4-oxo-4H-pyran-3-yl-2-(4-dibutylamino-2-hydroxy-benzoyl)-benzoesäureester.

### Beispiel 2:

### Synthese von 2-Methyl-4-oxo-4H-pyran-3-yl-2-(E)-3-(4-methoxy-phenyl)acrylsäureester

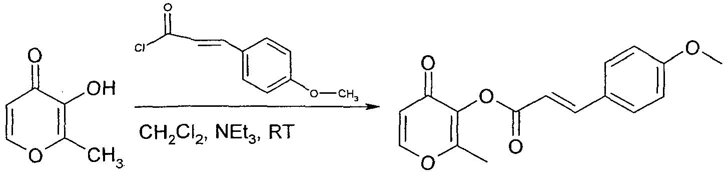

2,4g 3-Hydroxy-2-methyl-4-pyranon werden in 50 mL Dichlormethan bei Raumtemperatur unter Argon gelöst. Anschließend werden 4,5 g trans-4-Methoxyzimtsäurechlorid eingetragen und 3,17 mL Triethylamin langsam zugetropft. Man lässt für 4 h bei Raumtemperatur rühren. Zum Abfiltrieren wird die Reaktionslösung auf 0°C gekühlt. Die Mutterlauge extrahiert man bei Raumtemperatur mit 25 mL 1N HCl. Die organische Phase wird anschließend nochmals mit Wasser nachgewaschen, mit Na₂SO₄ getrocknet, filtriert und das Lösungsmittel wird abdestilliert. Zur weiteren Aufreiningung suspendiert man in 15 mL i-Propanol, erwärmt kurz und lagert bei 8°C über Nacht im Kühlschrank. Nach Absaugen, Nachwaschen und Trocknen erhält man 4,8 g 2-Methyl-4-oxo-4H-pyran-3-yl-2-(E)-3-(4-methoxy-phenyl)acrylsäureester.
1 H-NMR (300 MHz, D₆-DMSO):δ [ppm] = 2,29 (s, 3H); 3,85 (s, 3H); 6,46 (d, 1H); 6,71 (d, 1 H); 7,03 (d, 2H); 7,76 (d, 2H); 7,86 (d, 1H); 8,12 (d, 1 H) .
¹³C-NMR (300 MHz, D₆-DMSO): ):δ [ppm] = 14,5; 55,4; 113,2; 114,5; 114,5; 115,9; 126,3; 130,6; 131,0; 137,8; 147,0; 155,9; 159,2; 161,6; 163,3; 171,2.

### Beispiel 3:

### Synthese von 2-Methyl-4-oxo-4H-pyran-3-yl-3-(benzotriazol-2-yl-4-hydroxy)-benzoesäureester

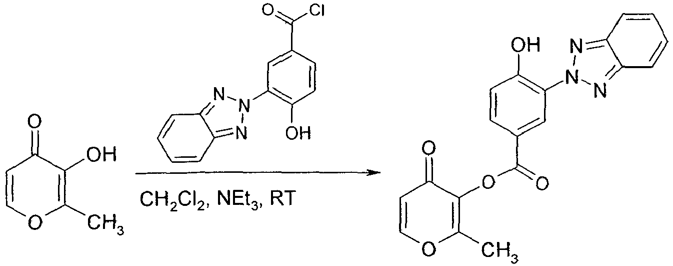

Analog zu Beispiel 2 werden 2,5 g 3-Hydroxy-2-methyl-4-pyranon mit 7,5 g Benzotriazol-2-yl-4-hydroxy-benzoesäurechlorid umgesetzt. Man erhält 2-Methyl-4-oxo-4H-pyran-3-yl-3-(benzotriazol-2-yl-4-hydroxy)-benzoesäureester.

### Beispiel 4:

### Synthese von (E)-3-[4-(2-Methyl-4-oxo-4H-pyran-3-yloxy)-phenyl]-acrysäure 2-ethyl-hexylester

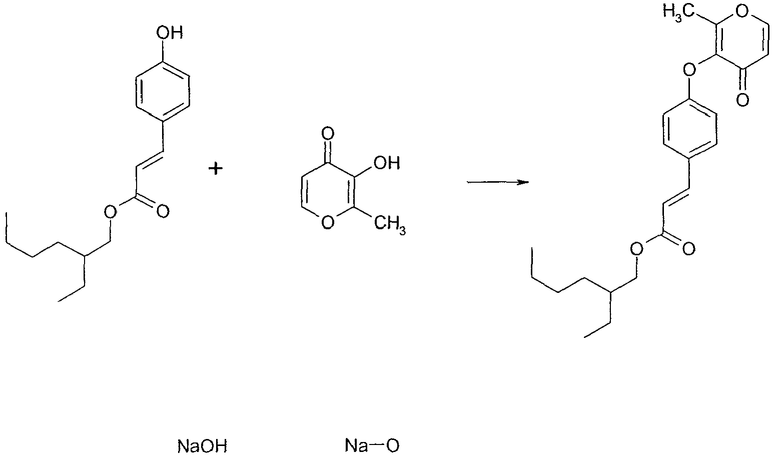

In einem mit Argon gespülten 3-Hals-Kolben werden 10 g (79 mmol) 3-Hydroxy-2-methyl-4-pyranonin 100 ml Dimethylformamid gelöst, 22,8 gTriphenylphosphin zugegeben und bei 0°C 17,6 gDiisopropylazodicarboxylat (87 mmol) langsam zugetropft. Nach 30 min wird der in 45 ml Dimethylformamid gelöste (E)-3-(4-Hydroxy-phenyl)-acrylsäure 2-ethyl-hexylester (23g, 83mmol)zugetropft. Nach 30 min bei 0°C wird noch 6 Stunden bei Raumtemperatur gerührt. Die Reaktionslösung wird zum Rückstand eingedampft, in 100 ml Ethylacetat/Hexan 1:2 suspendiert, filtriert und durch Zugabe von 100 ml Hexan das Produkt als weißer Feststoff ausgefällt.

### Beispiel 5:

### Synthese von (R)-5-[(R)-2-(3-Benzotriazol-2-yl-4-hydroxy-benzyloxy)-1-hydroxy-ethyl]-3,4-dihydroxy-5H-furan-2-on

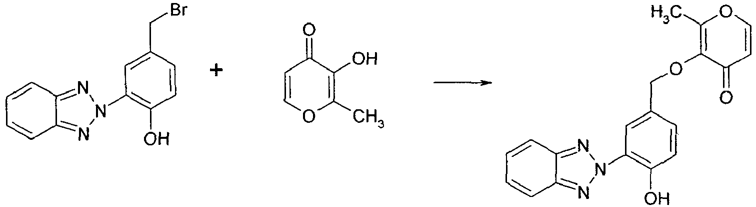

Es werden 10 g 3-Hydroxy-2-methyl-4-pyranon (79 mmol) in 250 ml THF (Tetrahydrofuran) gelöst und bei 0°C 3,2 gNatriumhydroxid (79 mmol) zugegeben. Nach 30 min wird das in 100 ml THF gelöste 2-Benzotriazol-2-yl-4-bromomethyl-phenol (28,8 g) zugetropft, für 60 min bei 0°C und 7 Stunden bei 70°C gerührt. Die Reaktionslösung wird nach Abkühlen auf Raumtemperatur einrotiert. Nach Wiederaufnahme im Lösungsmittelund Aufreinigung der Lösung an Kieselgel erhält man (R)-5-[(R)-2-(3-Benzotriazol-2-yl-4-hydroxy-benzyloxy)-1-hydroxy-ethyl]-3,4-dihydroxy-5H-furan-2-on.

### Beispiel 6:

### Synthese von 1,2-Dimethyl-4-oxo-1,4-dihydro-pyridin-3-yl-2-(E)-3-(4-methoxy-phenyl)acrylsäureester

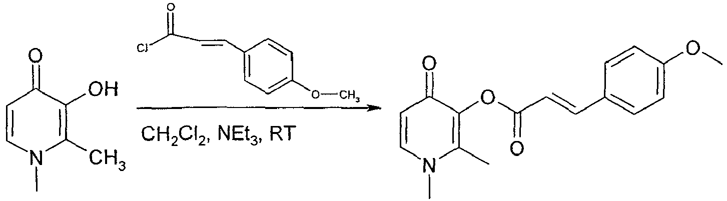

Zu einer Suspension von 5.00g (35.9 mmol) 3-Hydroxy-1,2-dimethyl-1H-pyridin-4-on in 100mL Dichlormethan und 8.50g (43.2 mmol) E-4-Methoxyzimtsäurechlorid werden nach 5 min 6.00mL Triethylamin zugetropft. Die Reaktionsmischung klart auf und die Innentemperatur steigt um 5°C an. Das orangerote Reaktionsgemisch wird weiter 3h bei RT gerührt. Danach werden 100mL Wasser zugegeben und es wird vorsichtig mit 25%iger Salzsäure angesäuert. Dabei fällt ein gelber flockiger Niederschlag aus. Dieser wird abgetrennt und i. Vak getrocknet. Aus der Mutterlauge wird nach Zugabe von weiteren 100mL Wasser nochmals gelber Feststoff ausgefällt und abgetrennt. Die vereinigten Feststoffe werden mit 25mL Dichlormethan 30 min gerührt, abgesaugt, mit wenig kaltem Dichlormethan nachge-waschen und anschließend i.Vak. bei 40°C getrocknet. Es werden 4.4g (41%) Produkt als hellbeiges Pulver erhalten.

### ¹H-NMR(400MHz, DMSO-d₆): δ= 2.49 (s, 3H), 3.78 (s, 3H), 4.07 (s, 3H), 6.69 (d, 1H, ³J = 16.3 Hz), 6.98 (d, 2H, ³J = 9.0 Hz), 7.35 (d, 1H, ³J = 7.3 Hz), 7.71 (d, 2H, ³J = 9.0 Hz), 7.89 (d, 1H, ³J = 16.3 Hz), 8.56 (d, 1H, ³J = 7.3 Hz).

### Beispiel 7: Bindungsnachweis

### Nachweis der Proteinaffinität im Lysinmodell:

Die Substanzen nach Beispiel 1 und Beispiel 2 werden jeweils zu 10mg in einen 10 mL Messkolben eingewogen und in 5 mL Tetrahydrofuran (THF) gelöst. Der Messkolben wird nun mit einer Lysinlösung in destilliertem Wasser bis zur Marke aufgefüllt. Die Konzentration der Lysinlösung beträgt 200mg/L Wasser. Man rührt die Lösung im Messkolben für 24 h bei 35°C. Nach 1 h und nach 24 h werden Proben gezogen. Davon pipettiert man je 5 µL mit einer Mikropipette auf die Startzone einer HPTLC Silica gel 60 F245 Platte (Merck Art. 1.05628). Zur Entwicklung der HPTLC Platte verwendet man das Laufmittel bestehend aus THF/Heptan im Verhältnis 75/25 enthaltend 0,1 vol% Ameisensäure. Nach Entwicklung detektiert man in einem CAMAG ATS4 bei 254nm und 366nm und ermittelt die Rf-Werte.

Ergebnis: Die Substanz des Beispiels 1 besitzt einen Rf-Wert von 0,55. Bereits nach 1 h in Kombination mit Lysin ist die Substanz nach Beispiel 1 komplett in einer Maillardreaktion mit Lysin umgesetzt. Die Ausgangssubstanz ist nicht mehr detektierbar. Demgegenüber sind Maillardprodukte entstanden, die in der Startzone der HPTLC-Platte detektiert werden. Analoge Ergebnisse erzielt man für die Substanz des Beispiels 2 (Rf 0,47), wobei nach 1h noch Ausgangssubstanz 2 detektierbar ist, nach 24h aber die Reaktion vervollständigt ist. Dann ist die Substanz des Beispiels 2 nicht mehr nachweisbar, während in der Startzone der HPTLC-Platte Maillardprodukte detektiert werden.

### Beispiel 8: Untersuchung im Liquidskin model

1mmol der Substanz gemäß Beispiel 1 (2-Methyl-4-oxo-4H-pyran-3-yl-2-(4-dibutylamino-2-hydroxy-benzoyl)-benzoesäureester) wird mit 1mmol DL-Lysin in 94 ml Ethylenglycol und 6 ml Wasser (Kaliumhydrogenphthalatgepuffert, pH = 4) gelöst und für 3 Std. bei RT gerührt (= Liquid*skin* model). Anschließend werden die L*-a*-b*-Werte dieser Lösung per UV-VIS-Spektrometer (Varian Cary-100) gemessen und mit den Ausgangswerten verglichen.

| | delta L* | delta a* | delta b* |
|---|---|---|---|
| Substanz gemäß Beispiel 1 | -4,07 | -2,75 | +7,83 |

Die Substanz nach Beispiel 1 zeigt eine Farbveränderung von Lysin.

**Beispiel A: Wasserfestes Sonnenschutzspray**

| A | | | |
|---|---|---|---|
| **Substanz nach Beispiel 2** | 1,00 | 1,00 | 2,00 |
| Diethylhexyl Syringylidenemalonate, Caprylic/Capric Triglyceride (Oxynex^{®} ST Liquid) | | 0,50 | |
| RonaCare^{®} AP | | 2,00 | |
| Ascorbyl Palmitate | | 1,00 | 1,00 |
| Cyprylic/capric Triglyceride (Miglyol 812 N) | 7,00 | 7,00 | 7,00 |
| Butylphthalimide isopropylphthalimide (Pelemol^{®} BIP) | 10,00 | 10,00 | 10,00 |
| C12-15 alkyl benzoate (Tegosoft^{®} TN) | 10,00 | 10,00 | 10,00 |
| Phenethyl benzoate (X-Tend 226) | 5,00 | 5,00 | 5,00 |
| RonaCare^{®} Tocopherolacetat | 1,00 | 1,00 | 1,00 |

| B | | | |
|---|---|---|---|
| Cyclopentasiloxane (Dow Corning 245) | 43,80 | 41,30 | 41,80 |
| Phenyltrimethicone (Dow Corning 556) | 2,00 | 2,00 | 2,00 |
| Cyclopentasiloxane, dimethiconol Dow Corning 1501 Fluid | 20,00 | 20,00 | 20,00 |
| Parfümöl (q.s.) | 0,20 | 0,20 | 0,20 |

**Beispiel B: Pump Haarspray**

| A | | | |
|---|---|---|---|
| **Substanz nach Beispiel 1** | 1,00 | 2,00 | 4,00 |
| Ethanol 96% reinst | Ad 100 | Ad 100 | Ad 100 |
| PVP/VA copolymer PVP/VA W 735 | 6,00 | 6,00 | 600 |

| B | | | |
|---|---|---|---|
| Diethylhexyl Syringylidenemalonate, Caprylic/Capric Triglyceride (Oxynex^{®} ST Liquid) | 0,06 | 0,25 | 0,50 |
| PEG-75 Lanolin BHT (Solan E - Low Dioxane) | 0,20 | 0,20 | 0,20 |
| Parfum (Fraq 280853 Green Activating) | 0,10 | 0,10 | 0,10 |

| C | | | |
|---|---|---|---|
| Wasser, demineralisiert | 13,00 | 13,00 | 13,00 |
| Titriplex III | 0,10 | 0,10 | 0,10 |
| PEG-12 dimethicone Dow Corninq 193 Fluid | 0,50 | 0,50 | 0,50 |
| 0,1% D&C Red No 33 (Cl 17200) in Wasser | 0,20 | 0,20 | 0,20 |
| PEG-40 Hydrogenated Castor Oil (Cremophor RH 410) | 1,00 | 1,00 | 1,00 |

**Beispiel C: W/O-Emulsionen**

| **Emulsion** | **A** | **B** | **C** | **D** | **E** | **F** |
|---|---|---|---|---|---|---|
| Polyglyceryl-2-Dipolyhydroxystearat | 3 | 5 | 3 | | | |
| PEG-30 Dipolyhydroxystearat | | | 2 | 3 | 4 | 5 |
| Natrium-Stärke Octenylsuccinat | 0,5 | 0,4 | | 0,3 | | 1 |
| Glycin | 0,3 | 0,3 | 0,5 | 0,4 | | |
| Alkohol | | 5 | 2 | 5 | 4 | |
| Magnesiumsulfat | 0,2 | 0,3 | 0,3 | 0,4 | 0,5 | 0,2 |
| C12-15 Alkyl Benzoat | 5 | 3 | | | 5 | |
| C₁₂₋₁₃ Alkyl Tartrat | | 2 | | | | |
| Butylenglycol Dicaprylat/Dicaprat | 5 | | | | 3 | 3 |
| Dicaprylyl Ether | | | | | 2 | |
| Mineralöl | | 4 | | 6 | | 8 |
| Octyldodecanol | 2 | | | | | |
| Dicaprylcaprat | | 2 | | | 2 | 2 |
| Cyclomethicon | 5 | | 5 | 10 | | |
| Dimethicon | | | | 5 | | |
| Isohexadecan | | 1 | | | | |
| Butylenglycol | 5 | 8 | | | | 3 |
| Propylenglykol | | | 1 | | 5 | 3 |
| Glycerin | 3 | 5 | 7 | 10 | 3 | 3 |
| C18-38 Säuretriglyceride | 0,5 | | 1 | | 1 | |
| Titandioxid | 5 | 6 | 4 | | | 4 |
| Zinkoxid | 5 | | | | | |
| Bis-Ethylhexyloxyphenol Methoxyphenyltriazin | | 3 | 3 | 2 | | |
| Ethylhexyltriazon | | 4,5 | 3 | | 3 | |
| **Substanz nach Beispiel 1** | 0,5 | | 1,0 | 1,0 | 3,0 | 1,5 |
| **Substanz nach Beispiel 2** | 0,5 | 1,0 | | | | 1,5 |
| Diethylhexylbutamidotriazon | | | 1,5 | 4 | | |
| Butyl Methoxydibenzoylmethan | 2 | 3 | 4 | | 1 | 3 |
| Uvinul^{®} A Plus | | | | 4 | 2 | |
| Ethylhexylmethoxycinnamat | | | | | 7 | 5 |
| Taurin | 0,1 | | | 0,5 | 0,2 | |
| Vitamin E Acetat | 0,2 | 02 | | 0,3 | 0,1 | 0,5 |
| Na₂H₂EDTA | 0,1 | 0,1 | 0,2 | 0,2 | 0,2 | 0,5 |
| C8-C16 Alkylpolyglycosid | 1 | | | | | |
| Phenoxyethanol | 1,0 | 1,0 | 1,0 | 1,0 | 1,0 | 1,0 |
| Ethylhexylglycerin | 0,25 | 0,25 | 0,25 | 0,25 | 0,25 | 0,25 |
| Natriumhydroxid | q.s. | q.s. | q.s. | q.s | q.s. | q.s. |
| Wasser | ad 100,0 | ad 100,0 | ad 100,0 | ad 100,0 | ad 100,0 | ad 100,0 |

**Beispiel D: Haarpflegeformulierung**

| **Gehalt in g Komponente per 100 g Formulierung** | | | | | | |
|---|---|---|---|---|---|---|
| **Komponente** | **A** | **B** | **C** | **D** | **E** | **F** |
| Disodium EDTA | 0.100 | 0.100 | 0.100 | 0.100 | 0.10 0 | 0.100 |
| Oxynex^{®}ST | 2.000 | 2.000 | 2.000 | 2.000 | 2.00 0 | 2.000 |
| **Substanz** nach **Beispiel 2** | | 0,25 | 0,50 | 1,50 | 2,00 | 2,00 |
| **Substanz nach Beispiel 1** | 0,25 | | | 0,50 | | 2,00 |
| Hexamidine diisethionate | 0.100 | 0 | 0 | 0 | 0 | 0 |
| Tetrahydrocurcumin | 0 | 0.500 | 0 | 0 | 0 | 0 |
| Glycyrrhetinic acid | 0 | 0 | 0.300 | 0 | 0 | 0 |
| Thiotaine®¹ | 0 | 0 | 0 | 5.000 | 0 | 0 |
| N-undecylenoyl-L-phenyl-alanine | 0 | 0 | 0 | 0 | 1.00 0 | 0 |
| N-acetyl glucosamine | 0 | 0 | 0 | 0 | 0 | 2.000 |
| Niacinamide | 5.000 | 5.000 | 5.000 | 5.000 | 5.00 0 | 5.000 |
| Citric acid | 0.015 | 0 | 0 | 0 | 0 | 0 |
| Isohexadecane | 3.000 | 3.000 | 3.000 | 3.000 | 3.00 0 | 3.000 |
| Isopropyl isostearate | 1.330 | 1.330 | 1.330 | 1.330 | 1.33 0 | 1.330 |
| Isopropyl N-laurosylsarcosinate | 0 | 0 | 5.000 | 0 | 0 | 0 |
| Sucrose polycottonseedate | 0.670 | 0.670 | 0.670 | 0.670 | 0.67 0 | 0.670 |
| Polymethylsilsesquioxane | 0.250 | 0.250 | 0.250 | 0.250 | 0.25 0 | 0.250 |
| Cetearyl glucoside + cetearyl alcohol | 0.200 | 0.200 | 0.200 | 0.200 | 0.20 0 | 0.200 |
| Behenyl alcohol | 0.400 | 0.400 | 0.400 | 0.400 | 0.40 0 | 0.400 |
| Ethylparaben | 0.200 | 0.200 | 0.200 | 0.200 | 0.20 0 | 0.200 |
| Propylparaben | 0.100 | 0.100 | 0.100 | 0.100 | 0.10 0 | 0.100 |
| Cetyl alcohol | 0.320 | 0.320 | 0.320 | 0.320 | 0.32 0 | 0.320 |
| Stearyl alcohol | 0.480 | 0.480 | 0.480 | 0.480 | 0.48 0 | 0.480 |
| Tocopheryl acetate | 0.500 | 0.500 | 0.500 | 0.500 | 0.50 0 | 0.500 |
| PEG-100 stearate | 0.100 | 0.100 | 0.100 | 0.100 | 0.10 0 | 0.100 |
| Glycerin | 7.000 | 7.000 | 7.000 | 7.000 | 7.00 0 | 7.000 |
| Titanium dioxide | 0.604 | 0.604 | 0.604 | 0.604 | 0.60 4 | 0.604 |
| Polyacrylamide + C13-14 isoparaffin + laureth-7 | 3.000 | 2.000 | 2.000 | 2.000 | 2.00 0 | 2.000 |
| Panthenol | 1.000 | 1.000 | 1.000 | 1.000 | 1.00 0 | 1.000 |
| Benzyl alcohol | 0.400 | 0.400 | 0.400 | 0.400 | 0.40 0 | 0.400 |
| Ascorbinsäure | 0,200 | 0,200 | 0,200 | 0,200 | 0,20 0 | 0,200 |
| Dimethicone + dimethiconol | 2.000 | 2.000 | 2.000 | 2.000 | 2.00 0 | 2.000 |
| Water (to 100 g) | to 100 | to 100 | to 100 | to 100 | to 100 | to 100 |
| TOTAL | 100 | 100 | 100 | 100 | 100 | 100 |

**Beispiel E: Haarpflegeformulierung**

| **Gehalt in g Komponente per 100 g Formulierung** | | | |
|---|---|---|---|
| **Komponente** | **G** | **H** | **I** |
| Disodium EDTA | 0.100 | 0.100 | 0.100 |
| Oxynex^{®} ST | 2.000 | 2.000 | 2.000 |
| **Substanz nach Beispiel 2** | 0,50 | 3,50 | 1,50 |
| Cetyl pyridinium chloride | 0.200 | 0 | 0 |
| Pitera^{®} | 0 | 10 | 0 |
| Ascorbyl glycoside | 0 | 0 | 2.000 |
| Niacinamide | 5.000 | 5.000 | 5.000 |
| Polyquaternium 37 | 0 | 0 | 0 |
| Isohexadecane | 3.000 | 3.000 | 3.000 |
| Isopropyl isostearate | 1.330 | 1.330 | 1.330 |
| Sucrose polycottonseedate | 0.670 | 0.670 | 0.670 |
| Polymethylsilsesquioxane | 0.250 | 0.250 | 0.250 |
| Cetearyl glucoside + cetearyl alcohol | 0.200 | 0.200 | 0.200 |
| Behenyl alcohol | 0.400 | 0.400 | 0.400 |
| Ethylparaben | 0.200 | 0.200 | 0.200 |
| Propylparaben | 0.100 | 0.100 | 0.100 |
| Cetyl alcohol | 0.320 | 0.320 | 0.320 |
| Stearyl alcohol | 0.480 | 0.480 | 0.480 |
| Tocopheryl acetate | 0.500 | 0.500 | 0.500 |
| PEG-100 stearate | 0.100 | 0.100 | 0.100 |
| Glycerin | 7.000 | 7.000 | 7.000 |
| Titanium dioxide | 0.604 | 0.604 | 0.604 |
| Polyacrylamide + C13-14 isoparaffin + laureth-7 | 2.000 | 2.000 | 2.000 |
| Panthenol | 1.000 | 1.000 | 1.000 |
| Benzyl alcohol | 0.400 | 0.400 | 0.400 |
| Dimethicone + dimethiconol | 2.000 | 2.000 | 2.000 |
| Water (to 100 g) | to 100 | to 100 | to 100 |
| TOTAL | 100 | 100 | 100 |

**Beispiel F: O/W-Emulsionen**

| **Emulsion** | **A** | **B** | **C** | **D** | **E** | **F** |
|---|---|---|---|---|---|---|
| Glyceryl Stearat Citrat | 2,5 | 2 | 3 | | | |
| Sorbitanstearat | 0,5 | | | 2 | 1,5 | 2 |
| Polyglyceryl-3 Methylglycose Distearat | | | | 2,5 | 3 | 3 |
| Polyglyceryl-2 Dipolyhydroxystearat | | 0,8 | | | | 0,5 |
| Cetearylalkohol | | | | 1 | | |
| Stearylalkohol | 2 | | | | | 2 |
| Cetylalkohol | | 1 | | | 3 | |
| Acrylates/C₁₀₋₃₀ Alkyl Acrylat Crosspolymer | | 0,2 | | | 0,1 | |
| Carbomer | | 0,2 | 0,3 | 0,2 | | |
| Xanthan Gum | 0,4 | | 0,2 | 0,2 | 0,3 | 0,4 |
| C₁₂₋₁₅ Alkyl Benzoat | 5 | 3 | | | 5 | |
| C₁₂₋₁₃ Alkyl Tartrat | | 2 | | | | |
| Butylenglycol Dicaprylat/Dicaprat | 5 | | | | 3 | 3 |
| Dicaprylyl Ether | | | | | 2 | |
| Octyldodecanol | 2 | | | | | |
| Dicaprylcaprat | | 2 | | | 2 | 2 |
| Cyclomethicon | 5 | | 5 | 10 | | |
| Dimethicon | | | | 5 | | |
| Isohexadecan | | 1 | | | | |
| Butylenglycol | 5 | 8 | | | | 3 |
| Propylengykol | | | 1 | | 5 | 3 |
| Glycerin | 3 | 5 | 7 | 10 | 3 | 3 |
| C18-C38 Säuretriglyceride | 0,5 | | 1 | | 1 | |
| Titandioxid | 5 | | | 2 | | |
| 2,2'-Methylen-bis-(6-(2H-benzotriazol-2-yl)-(1,1,3,3-tetramethylbutyl)phenol) | 2,5 | | | | | |
| 2,4,6-Tris-(biphenyl)-1,3,5-triazin | | 2 | | | | |
| C8-C16 Alkylpolyglycosid | 1 | 0,6 | | | | |
| UVASorb^{®} K2A | | | 2 | | | |
| Uvinul^{®} A Plus | 2 | | | | | 1 |
| Homosalat | | 5 | | 1 | | |
| Phenylbenzimidazol Sulfonsäure | | | 2 | | | 1 |
| Benzophenon-3 | 2 | | | | 2 | |
| Octylsalicylat | 5 | 5 | | 2 | | |
| Octocrylen | 2 | | | | 3 | 1 |
| **Substanz nach Beispiel 1** | 1,0 | | | | | 0,5 |
| **Substanz nach Beispiel 2** | | 1,0 | | | | 0,5 |
| **Substanz nach Beispiel 3** | | | 1,0 | | | 0,5 |
| **Substanz nach Beispiel 4** | | | | 1,0 | | 0,5 |
| **Substanz nach Beispiel 5 oder 6** | | | | | 1,0 | 0,5 |
| Bis-Ethylhexyloxyphenol Methoxyphenyltriazin | | 3 | 2 | 1 | | |
| Parsol^{®} SLX | | | 3 | | | |
| Dihydroxyacetat | | | | | 4 | |
| Taurin | 0,1 | | | 0,5 | 0,2 | |
| 8-Hexadecen-1,16-dicarbonsäure | | 0,2 | | | | |
| Vitamin E Acetat | 0,2 | 0,2 | | 0,3 | 0,1 | 0,5 |
| Na₂H₂EDTA | 0,1 | 0,1 | 0,2 | 0,2 | 0,2 | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100.0 | ad 100,0 | ad 100,0 | ad 100,0 | ad 100, 0 |

**Beispiel G: O/W-Emulsionen**

| **Emulsion** | **G** | **H** | **I** | **K** | **L** | **M** |
|---|---|---|---|---|---|---|
| Ceteareth-20 | 1 | 1,5 | 1 | | | |
| Sorbitanstearat | | | 0,5 | | 0,5 | |
| Glyceryl Stearat SE | | | | 1 | 1 | 1,5 |
| Emulgade F^{®} | | | | 2,5 | 2,5 | 3 |
| Cetearylalkohol | | | | 1 | | |
| Stearylalkohol | | | | | 1,5 | |
| Cetylalkohol | | | 0,5 | | | 2 |
| Acrylates/C₁₀₋₃₀ Alkyl Acrylat Crosspolymer | 0,2 | 0,4 | 0,3 | 0,1 | | |
| Carbomer | | | | | 0,3 | |
| Xanthan Gum | | | | 0,4 | | 0,4 |
| C12-15 Alkyl Benzoat | 5 | 3 | | | 5 | |
| 2-Phenylbenzoat | | 2 | | | | |
| Butylenglycol Dicaprylat/Dicaprat | 5 | | | | 3 | 2 |
| Dicaprylyl Ether | | | | | 2 | |
| Diethylhexylnaphthalat | 2 | | | | | |
| Dicaprylcaprat | | 2 | | 2 | 2 | 2 |
| Cyclomethicon | 5 | | 5 | 10 | | |
| Isohexadecan | | | | 5 | | |
| Mineralöl | | 1 | | | | |
| Propylenglykol | | | 4 | | | |
| Glycerin | 5 | 7 | 3 | 5 | 6 | 8 |
| C18-38 Säuretriglyceride | 0,5 | | 1 | | 1 | |
| Titandioxid | 5 | | 3 | 2 | | |
| NeoHeliopan^{®} AP | | 2 | | | 1 | 1 |
| Phenylbenzimidazol Sulfonsäure | 1 | | | 1 | 2 | 1 |
| Ethylhexylmethoxycinnam at | 5 | | 4 | 4 | | |
| Ethylhexyltriazon | | 2 | | 1 | | |
| Diethylhexylbutamidotriaz an | 1 | | | | | |
| Butyl Methoxydibenzoylmethan | 2,5 | | 2 | 2 | | 1 |
| Bis-Ethylhexyloxyphenol Methoxyphenyltriazin | 2 | | | | | |
| 4-Methylbenzyliden Camphor | 3 | | | | | |
| Parsol® SLX | | | | | 2 | |
| **Substanz nach Beispiel 2** | 1,0 | | 0,5 | 1,0 | 2,5 | |
| **Substanz nach Beispiel 1** | | 1,0 | 0,5 | 1,0 | | 2,5 |
| Kreatinin | 0,1 | 0,01 | 0,05 | | | |
| Kreatin | 0,5 | 0,2 | 0,1 | | | |
| Licorice Extrakt/Licochalkon | | | | 0,5 | | |
| Vitamin E Acetat | 0,2 | | | 0,5 | 0,5 | 0,5 |
| Tapioka Stärke | | 3 | | | 2 | |
| Na₂H₂EDTA | 0,1 | | 0,2 | | | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |

**Beispiel H: O/W-Emulsionen**

| **Emulsion** | **N** | **O** | **P** | **Q** | **R** | **S** |
|---|---|---|---|---|---|---|
| Glycerylstearat SE | | 2 | | 2 | | |
| Glycerylstearat | 2 | | 2 | | | |
| PEG-40 Stearat | | | 2 | | 1 | |
| PEG-10 Stearat | | | | 2,5 | 1 | |
| Ceteareth-20 | | | | | | 2,6 |
| Natrium Cetyl Phosphate | | | | | 2 | |
| Glyceryl Stearat, Ceteareth-12, Ceteareth-20, Cetearyl Alcohol, Cetyl Palmitat | | | | | | 5,4 |
| Stearinsäure | 3 | 2 | | | 2 | |
| Stearylalkohol | | 2 | 2 | | | |
| Stearylalkohol | 0,5 | | 2 | | | |
| Cetylalkohol | 3 | | | 2 | | |
| Acrylates/C₁₀₋₃₀ Alkyl Acrylat Crosspolymer | | | 0,2 | | 0,4 | |
| Carbomer | | 0,3 | | 0,3 | 0,3 | |
| Xanthan Gum | | 0,3 | 0,4 | | | |
| C12-15 Alkyl Benzoat | 5 | | | | 5 | 3 |
| 2-Phenylbenzoat | 5 | | | | | |
| Butylenglycol | | 5 | | 4 | | 3 |
| Dicaprylat/Dicaprat | | | | | | |
| Dicaprylyl Ether | | 2 | | | 3 | |
| Diethylhexylnaphthalat | 3 | | | | | |
| Cyclomethicon | 2 | | 10 | 2 | | |
| Isohexadecan | | | | 2 | 3 | |
| Mineralöl | | | | | 3 | |
| Propandiol | | 3 | | 5 | | |
| Glycerin | 3 | 5 | 10 | 7 | 4 | 5 |
| Titandioxid | 2 | 4 | | | | |
| Zinkoxid | | | | | 2 | |
| Drometrizole Trisiloxane | | | | | 3 | |
| Ethylhexylmethoxycinnam at | | 6 | 5 | | | |
| Phenylbenzimidazol Sulfonsäure | | 0,5 | 2 | | 1 | |
| Homosalat | 5 | | | 7 | | |
| Butyl Methoxydibenzoylmethan | | 3 | | | | |
| Bis-Ethylhexyloxyphenol Methoxyphenyltriazin | | 2 | 3 | | | |
| Octylsalicylat | | | | 5 | | |
| Octocrylen | | | | | 3 | |
| **Substanz nach Beispiel 2** | 0,25 | 1,5 | 0,5 | 2,5 | 1,0 | 5,0 |
| Parsol^{®} SLX | 4 | | | | | 5 |
| PVP Hexadecen Copolymer | 0,5 | | 1 | | 0,8 | |
| Coenzym Q 10 | 0,2 | 0,02 | | 0,3 | | |
| Vitamin E Acetat | 0,2 | | 0,3 | | 0,8 | 0,5 |
| Na₂H₂EDTA | 0,1 | | | | | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |

**Beispiel I: Hydrodisperionen (Lotionen und Sprays)**

| | **A** | **B** | **C** | **D** | **E** | **F** |
|---|---|---|---|---|---|---|
| Glyceryl Stearat Citrat | | 0,40 | | | | |
| Cetyl Alkohol | | | | | 2,00 | |
| Natrium Carbomer | | | | | 0,30 | |
| Acrylates/C₁₀₋₃₀ Alkyl Acrylate Crosspolymer | 0,30 | | 0,30 | 0,40 | 0.10 | 0,10 |
| Cetsareth-20 | | | 1,00 | | | |
| Xanthan Gummi | | | | 0,15 | | 0,50 |
| Dimethicon / Vinyl Dimethicon Crosspolymer | | | | 5,00 | | 3,00 |
| UVASorb^{®} K2A | | | | | 3,50 | |
| Uvinul^{®}A Plus | 0,25 | | | 0,50 | 2,00 | 1,50 |
| Butyl Methoxydibenzoylmethan | 1,20 | | 3,50 | | | |
| Bis-Ethylhexyloxyphenol | 2,00 | 2,00 | | 0,25 | | |
| Methoxyphenyl Triazin | | | | | | |
| Terephthaliden Dicampher Sulfonsäure | | | | | | 0,50 |
| Dinatrium Phenyl Dibenzimidazol Tetrasulfonat | | | | | | 1,00 |
| Phenylbenzimidazol Sulfonsäure | | | 2,00 | | | |
| Ethylhexyl Methoxycinnamat | 5,00 | | 7,00 | | 5,00 | 8,00 |
| Diethylhexyl Butamido Triazon | | | 2,00 | 2,00 | | |
| Ethylhexyl Triazon | 4,00 | 3,00 | | | 4,00 | |
| Octocrylen | | | | 10,00 | | 2,50 |
| **Substanz nach Beispiel 2, 3, 4, 5 oder 6** | 0,25 | | 0,5 | | 1,0 | 2,0 |
| **Substanz nach Beispiel 1** | | 0,5 | | 1,0 | | 1,0 |
| C12-15 Alkyl Benzoat | 2,00 | | 2,50 | | | |
| Phenethyl Benzoat | 4,00 | | | 7,50 | | 5,00 |
| C₁₈₋₃₆ Triglycerid Fettsäure | | | 1,00 | | | |
| Butylenglycol Dicaprylat/Dicaprat | | | | | 6,00 | |
| Dicaprylyl Carbonat | | 3,00 | | | | |
| Dicaprylylether | | 2,00 | | | | |
| Cyclomethicon | | | | 1,50 | | |
| Lanolin | | | | | 0,35 | |
| PVP Hexadecen Copolymer | 0,50 | | 0,50 | | 0,50 | 1,00 |
| Ethylhexyloxyglycerin | | 0,75 | | 1,00 | | 0,50 |
| Glycerin | 10,00 | 5,00 | 5,00 | | 5,00 | 15,00 |
| Butylenglycol | | 7,00 | | | | |
| Glycin Soja | | | | 1,00 | | |
| Vitamin E Acetat | 0,50 | 0,25 | 050 | 0,25 | 0,75 | 1,00 |
| α-Glycosylrutin | | | | | 0,25 | |
| Trinatrium EDTA | | 1,00 | 1.00 | 0,10 | 0,20 | |
| Idopropinylbutylcarbamat | 0,20 | 0,10 | | | | 0,15 |
| Methylparaben | 0,50 | | 0,20 | | 0,15 | |
| Phenoxyethanol | 0,50 | 0,40 | 0,40 | | 1,00 | 0,60 |
| Ethanol | 3,00 | 10,00 | 4,00 | 3,50 | | 1,00 |
| Parfüm, Farbstoffe | q.s. | q.s. | q.s. | qs. | q.s. | q.s. |
| Wasser | ad 100 | ad 100 | ad 100 | ad 100 | ad 100 | ad 100 |
| Neutralisationsmittel (Natriumhydroxid, Kaliumhydroxid) | qs | qs | qs | qs | qs | qs |

**Beispiel J: wässrige und wässrig/alkoholische Formulierungen**

| | **A** | **E** | **C** | **D** | **E** | **F** |
|---|---|---|---|---|---|---|
| Ethanol | 50 | 5 | 2 | 40 | 15 | |
| Hydroxyethylcellulose | 0.5 | | | | | |
| Acrylates/C10-30 Alkyl Acrylate Crosspolymer | | | | 0,3 | 0,6 | |
| Cocamidopropylbetain | | | 0,3 | | | |
| UVASorb® K2A | | | | | 2 | |
| Uvinul® APlus | 5 | | | | | |
| Butyl Methoxydibenzoylmethan | 0,5 | | | 3 | | |
| Dinatrium Phenyl Dibenzimidazol Tetrasulfonat | | 2 | 1 | | | |
| Phenylbenzimidazol Sulfonsäure | | 5 | 3 | | 2 | 4 |
| Ethylhexyl Methoxycinnamat | 10 | | | | 3 | |
| Diethylhexyl Butamido Triazon | | | | 3 | | |
| Ethylhexyl Triazon | | | | | 2 | |
| Octocrylen | | | | 5 | | |
| **Substanz nach Beispiel 2** | 2,5 | 0,75 | 1,5 | 3,0 | 3,5 | 4,0 |
| C₁₂₋₁₅ Alkyl Benzoat | | | | 3 | | |
| C18-36 Triglycerid Fettsäure | | | | 1 | | |
| Butylenglycol Dicaprylat/Dicaprat | 2 | | | | | |
| C12-13 Alkyl Tartrat | | | | | 5 | |
| Cyclomethicon | 4 | | | 2 | | |
| Insekt Repellent^{®} 3535 | | | | 5 | | |
| Dimethicon | | | | | 3 | |
| PVP Hexadecen Copolymer | | 0,5 | | 1 | | 0.5 |
| Ethylhexylglycerin | | 0,5 | | | | |
| Glycerin | 5 | 7 | 3 | 8 | | S |
| Butylenglycol | | | 5 | | 5 | |
| Metylpropandiol | | | | 4 | | |
| Vitamin E Acetat | | 0,3 | 0,2 | 0,5 | | |
| Panthenol | 0.5 | | 0,2 | | | 0,3 |
| Kreatinin | | | 0,01 | | 0,02 | |
| Creatin | | | 0,1 | | 0,2 | |
| PEG-40 Hydriertes Ricinusöl | | 0,5 | 0,3 | | | 0.5 |
| Trinatrium EDTA | 0,3 | 0,2 | 0,2 | 0,2 | 0,2 | 0,5 |
| Methylparaben | q.s. | q.s. | q.s. | q.s. | q.s. | q.s. |
| Natriumhydroxid | q.s. | q.s. | q.s. | q.s. | q.s. | q.s. |
| Parfüm, Farbstoffe | q.s. | q.s. | q.s. | q.s. | q.s. | q.s. |
| Wasser | ad 100 | ad 100 | ad 100 | ad 100 | ad 100 | ad 100 |

**Beispiel K: kosmetische Schäume**

| **Emulsion** | **A** | **B** | **C** |
|---|---|---|---|
| Stearinsäure | 2 | 2 | |
| Palmitinsäure | | | 1,5 |
| Cetylalkohol | 2,5 | 2 | |
| Stearylalkohol | | | 3 |
| PEG-100 Stearat | | | 3,5 |
| PEG-40 Stearat | | 2 | |
| PEG-20 Stearat | 3 | | |
| Sorbitanstearat | | 0,8 | |
| C₁₂₋₁₅ Alkyl Benzoat | 5 | | |
| C₁₂₋₁₃ Alkyl Tartrat | | | 7 |
| Butylenglycol Dicaprylat/Dicaprat | | 6 | |
| Dicaprylyl Ether | | | 2 |
| Cyclomethicon | | 2 | 3 |
| Butylenglycol | 1 | | |
| Isohexadecan | 2 | | |
| Methylpropandiol | | | |
| Propylenglykol | | | 5 |
| Glycerin | 5 | 7 | |
| UVASorb® K2A | | | 2 |
| Uvinul® A Plus | 2 | 3 | |
| **Substanz nach Beispiel 2** | 0,5 | 1,0 | 1,5 |
| Parsol SLX^{®} | | 3 | |
| Homosalat | | 5 | |
| Phenylbenzimidazol Sulfonsäure | | 2 | 2 |
| Benzophenon-3 | 2 | | |
| Octylsalicylat | | 5 | |
| Octocrylen | 2 | | |
| Bis-Ethylhexyloxyphenol Methoxyphenyltriazin | | 3 | |
| 2,2'-Methylen-bis-(6-(2H-benzotriazol-2-yl)-4-(1,1,3,3-tetramethylbutyl)-phenol) | | | 8 |
| 2,4,6-Tris-(biphenyl)-1,3 5-triazin | 5 | | 4 |
| C8-C16 Alkylpolyglycoside | 1 | | |
| Vitamin E Acetat | 0,6 | 0,5 | 0,2 |
| Kreatin/Kreatinin | | | 0,5 |
| Na₂H₂EDTA | 0,50 | | |
| Phenoxyethanol | 1,0 | 1,0 | 1,0 |
| Ethylhexylglycerin | 0,5 | 0,5 | 0,5 |
| Natriumhydroxid | q.s. | q.s. | q.s. |
| Wasser | ad 100,0 | ad 100,0 | ad 100,0 |

**Beispiel K Fortsetzung: kosmetische Schäume**

| **Emulsion** | **D** | **E** | **F** | **G** |
|---|---|---|---|---|
| Stearinsäure | 2 | | | |
| Palmitinsäure | | | 3 | 3 |
| Cetylalkohol | 2 | 2 | | |
| Cetylstearylalkohol | | | 2 | 2 |
| Stearylalkohol | | | | |
| PEG-100 Stearat | | 4 | | |
| PEG-40 Stearat | 2 | | | |
| PEG-20 Stearat | | | 3 | 3 |
| Sorbitanstearat | 0,8 | | | |
| Tridecyl Trimellitate | | 5 | | |
| C₁₂₋₁₅ Alkyl Benzoat | | | 3 | 3 |
| Butylenglycol Dicaprylat/Dicaprat | 8 | | | |
| Octyldodecanol | | 2 | | |
| Cocoglyceride | | | | 2 |
| Dicaprylyl Ether | | | 2 | 2 |
| Cyclomethicon | | | | |
| Dimethicon | 1 | | 2 | 2 |
| Isohexadecan | | 3 | | |
| Methylpropandiol | | 4 | | |
| Propylenglykol, | | | | |
| Glycerin | 5 | | 6 | 6 |
| NeoHeliopan^{®} AP | | 2 | | |
| Phenylbenzimidazol Sulfonsäure | 1 | | | 1 |
| **Substanz nach Beispiel 2** | 0,75 | | 1,5 | |
| **Substanz nach Beispiel 1** | | 0,75 | 1,5 | 3,0 |
| Ethylhexylmethoxycinna mat | 5 | | 4 | 4 |
| Ethylhexyltriazon | | 2 | | 1 |
| Eusolex T-AVO^{®} | 2 | | | |
| Diethylhexylbutamidotria zon | 1 | | | |
| Butyl Methoxydibenzoylmetha n | 2,5 | | 2 | 2 |
| Bis-Ethylhexyloxyphenol Methoxy-phenyltriazin | 2 | | | |
| Vitamin E Acetat | 0,2 | | 0,3 | 0,3 |
| Na₂H₂EDTA | | | | |
| Parfum, Konservierungsmitte | | | | |
| Farbstoffe, usw. | | | | |
| Natriumhydroxid | | q-s. | q.s. | |
| Triethanolamin | q.s. | | | q.s. |
| Wasser | ad 100,0 | ad 100,0 | ad 100,0 | ad 100,0 |

**Beispiel L:. Haarspülung**

| | Gewichtsprozent [%] |
|---|---|
| Cetearyl Alcohol | 10 |
| Sunflowerseedamidopropyl Ethyldimonium Ethosulfate | 0,5 |
| Ceteareth-20 | 3,0 |
| Panthenol | 0,4 |
| Phenyl Trimethicone | 0,3 |
| Hydroxypropyl Guar Hydroxypropyltrimonium Chloride | 0,8 |
| **Substanz nach Beispiel 2** | **1,0** |
| Passiflora Incarnata Seed Oil | 0,2 |
| Basic red 51 | 0,1 |
| Basic red 76 | 0,2 |
| Parfüm | 1,0 |
| Konservierungsmittel | q.s. |
| Citric Acid / Sodium Hydroxide | q.s. to pH 5.5 |
| Aqua | ad 100 |

**Beispiel M:. Haarspülung**

| | Gewichtsprozent [%] |
|---|---|
| Cetearyl Alcohol | 5,0 |
| Cetrimonium Chloride | 1,0 |
| Polysilicone-15 | 0,5 |
| Panthenol | 0,4 |
| Dimethicone | 0,8 |
| Hydroxypropyl Guar Hydroxypropyltrimonium | 1,0 |
| Chloride | |
| **Substanz nach Beispiel 1** | **0,5** |
| **Substanz nach Beispiel 2** | **0,5** |
| Hydrogenated Grapeseed Oil | 1,5 |
| Tiliroside | 0,05 |
| Avocado Extrakt | 0,5 |
| Parfüm | 1,0 |
| Konservierungsmittel | q.s. |
| Citric Acid / Sodium Hydroxide | q.s. to pH 5.5 |
| Aqua | ad 100 |

**Beispiel N:. Haarschaum**

| | Gewichtsprozent [%] |
|---|---|
| Quaternium-80 | 0,2 |
| Polyquaternium-11 | 0,7 |
| PEG-60 Hydrogenated Castor Oil | 0,5 |
| Phenoxyethanol | 1,0 |
| RonaCare® AP | 0,25. |
| **Substanz nach Beispiel 1, 2, 3, 4, 5 oder 6** | **0,3** |
| Passiflora Incarnata Seed Oil | 0,2 |
| Citric Acid / Sodium Hydroxide | q.s. to pH 4.5 |
| Aqua | ad 100 |

Die Formulierung kann als leave-on oder rinse-off Formulierung eingesetzt werden und ist als Aerosol in Verhältnis Formulierung/Treibgas = 90/10 in einem Druckbehältnis verpackt. Als Treibgase können z.B. Propan oder Butan oder deren Gemische eingesetzt werden.

**Beispiel O: Shampoo**

| | Gewichtsprozent [%] |
|---|---|
| Sodium Laureth Sulfate | 5,0 |
| Cocamidopropy Betaine | 5,0 |
| Lauroyl Glutamic Acid | 3,0 |
| Decyl Glucoside | 5,0 |
| Polyquaternium-10 | 0,5 |
| PEG-3 Distearate | 0,8 |
| **Substanz nach Beispiel 2** | **0,5** |
| Nachtkerzenöl | 0,3 |
| Basic Red 51 | 0,1 |
| Ubiquinone | 0,1 |
| Decan-1,2-diol | 0,5 |
| Parfüm | 1,0 |
| Konservierungsmittel | q.s. |
| Sodium Chloride | 0,8 |
| Citric Acid / Sodium Hydroxide | q.s. to pH 5.5 |
| Aqua | ad 100 |

**Beispiel P: Shampoo**

| | Gewichtsprozent [%] |
|---|---|
| Palm Kernel / Coco Glucoside | 5,0 |
| Cocamidopropyl Betaine | 6,0 |
| Sodium Laureth Sulfate | 4,0 |
| Guar Hydroxypropyl Trimonium Chloride | 0,5 |
| Benzyl Alcohol | 0,5 |
| Hanfsamenöl | 0,5 |
| **Substanz nach Beispiel 1, 2, 3, 4, 5 oder 6** | **0,5** |
| Parfüm | 1,0 |
| Konservierungsmittel | q.s. |
| Sodium Chloride | 0,8 |
| Lactid Acid / Sodium Hydroxide | q.s. to pH 5,0 |
| Aqua | ad 100 |

**Beispiel Q: Shampoo**

| | Gewichtsprozent [%] |
|---|---|
| Sodium Lauryl Glucose Carboxylate | 5,0 |
| Palm Kernel / Coco Glucoside | 5,0 |
| Cocamidopropyl Betaine | 5,0 |
| Polyquaternium-7 | 0,2 |
| **Substanz nach Beispiel 1, 2, 3, 4, 5 oder 6** | **0,5** |
| Benzyl Alcohol | 0,5 |
| Passiflora Incarnata Seed Oil | 0,5 |
| PEG-60 Hydrogenated Castor Oil | 0,5 |
| PEG-18 Glyceryl Cocoate/Oleate | 1,0 |
| Parfüm | 1,0 |
| Konservierungsmittel | q.s. |
| Sodium Chloride | 0,8 |
| Lactid Acid / Sodium Hydroxide | q.s. to pH 6,0 |
| Aqua | ad 100 |

**Beispiel R: Haarfärberezepturen**

| | 1 | 2 | 3 | 4 | 5 | 6 | 7 |
|---|---|---|---|---|---|---|---|
| Benzyl Alcohol | 2,5 | | | | | | |
| Propylene Carbonate | 10 | | | | | | |
| Ethanol | 5,0 | | | | | | |
| Hydroxyethylcellulose | 2,0 | | | | | | |
| Pirenoxin NatriumCAS 51410-30-1 | 2,0 | | | | | | 2,0 |
| Tramsanguin CAS 34083-17-5 | | 1,0 | | | | 1,0 | |
| Cinnabarin CAS 606-59-7 | | | 1,0 | | | | |
| Cinnabarinsäure CAS 146-90-7 | | | | 1,0 | | | |
| Resorcinblau CAS 71939-12-3 | | | | | 1,0 | | |
| **Substanz nach Beispiel 1, 2, 3, 4, 5 oder 6** | 1,0 | 1,0 | 2,0 | 1,0 | 1,0 | 0,5 | |
| Parfüm | 1,0 | 1,0 | 1,0 | 1,0 | 1,0 | 1,0 | 1,0 |
| Konservierungsmittel | q.s. | q.s. | q.s. | q.s. | q.s. | q.s. | q.s. |
| Citric Acid | q.s. ad pH 5.5 | q.s. ad pH 5.5 | q.s. ad pH 5.5 | q.s. ad pH 5.5 | q.s. ad pH 5.5 | q.s. ad pH 5.5 | q.s. ad pH 5.5 |
| Aqua | ad 100 | ad 100 | ad 100 | ad 100 | ad 100 | ad 100 | ad 100 |

## Patentansprüche

1. Nicht-therapeutische Verwendung von Verbindungen der Formel I, 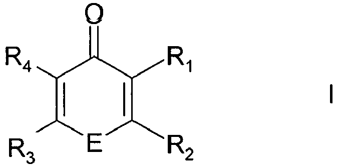 wobei
E NR₅ oder O bedeutet,
R₂, R₃ oder R₄ jeweils unabhängig voneinander -H, -A, -OA,-OH₂)ₚ-OH, -C(O)OA, COOH oder COOX bedeuten,
p eine ganze Zahl von 1 bis 4 bedeutet,
X das Gegenion zu der [COO-]-Gruppe ist,
R₅ A bedeutet,
A Alkyl mit 1 bis 20 C-Atomen bedeutet und
R₁ ein Substituent der Formel II, III, IV, V, VI, VII, VIII, IX, X, XI, XII, XIII, XIV oder XV ist, 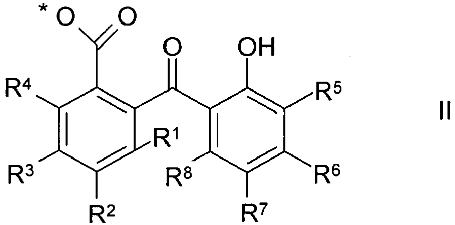 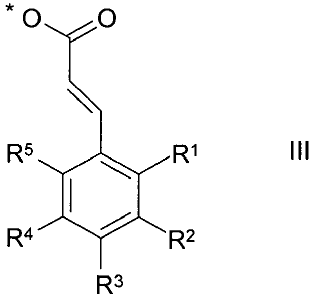 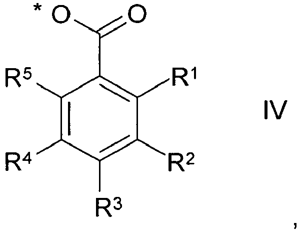 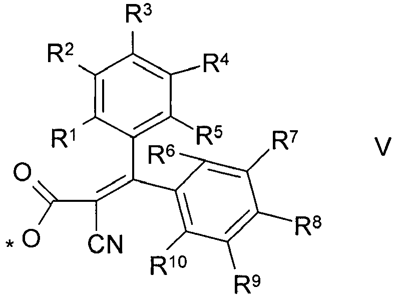 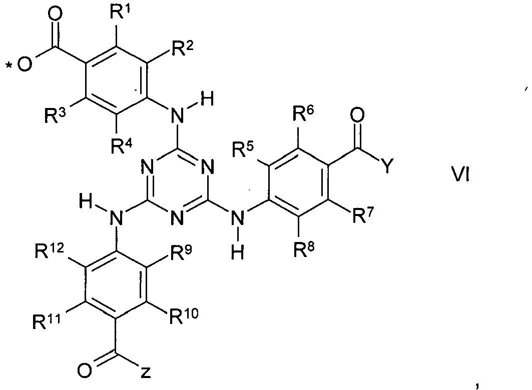 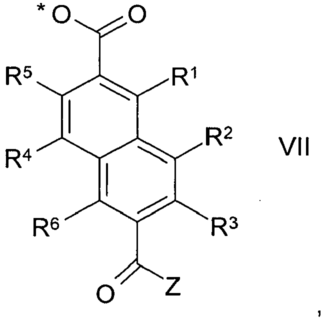 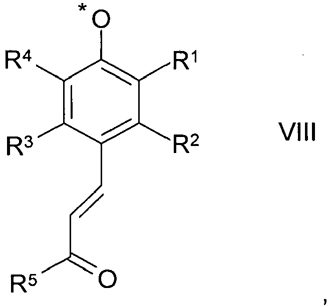 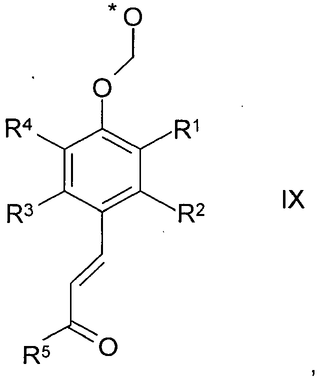 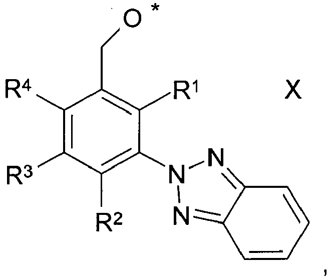 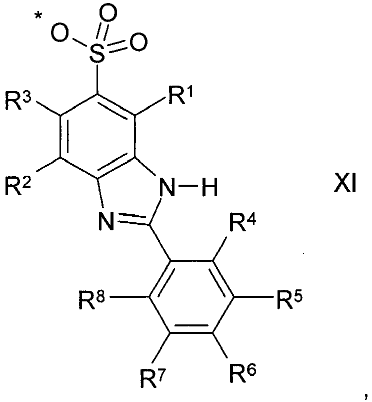 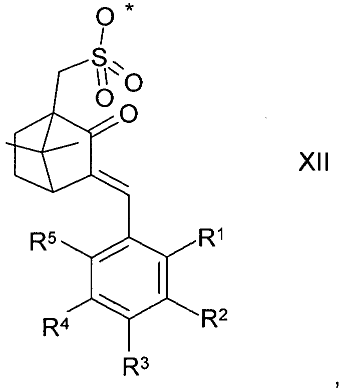 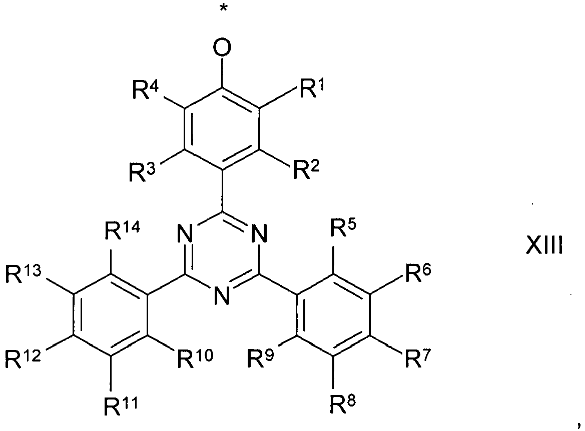 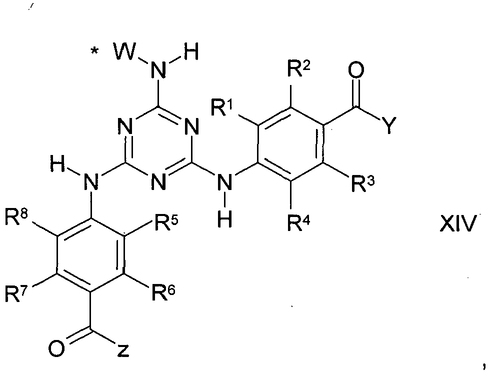 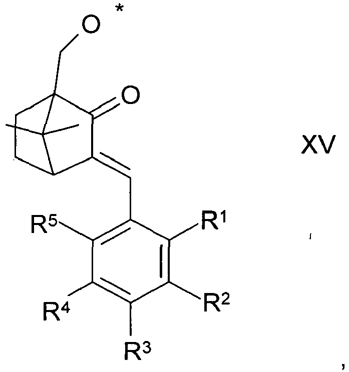 wobei
R¹ bis R¹⁴ jeweils unabhängig voneinander -H, -OH, -OA, -A, -NH₂,-NHA, -NA₂, -NH-(CH₂-CH₂-O)ₙ-H, -N[(CH₂-CH₂-O)ₙ-H]₂, -[NHA₂]X,-[NA₃]X, -SO₃H, -[SO₃]X, 2H-Benzotriazol-2-yl- oder 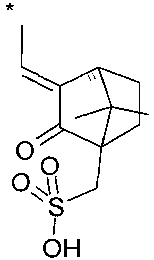 bedeuten,
n eine ganze Zahl von 1 bis 25 ist,
X das Gegenion zu den Kationen [NHA₂]⁺ und [NA₃]⁺ oder dem Anion [SO₃]⁻ ist und
Y und Z jeweils unabhängig voneinander die Struktureinheit I-1 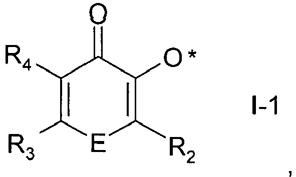 A, Hydroxy, -OA oder -NH-C(CH₃)₃ bedeuten, wobei A,
R₂, R₃, R₄ und E eine zuvor genannte Bedeutung haben,
W -(CH₂)ₘ-O oder -(CH₂)ₒ-C(O)-O bedeutet und
m und o jeweils unabhängig voneinander eine ganze Zahl von 1 bis 4 bedeuten,
mit der Bedingung, dass mindestens ein Susbstituent der Substituenten R¹ bis R¹⁴ in den Formeln II, III, IV, VII, X, XII und XV OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X, 2H-Benzotriazol-2-yl- oder 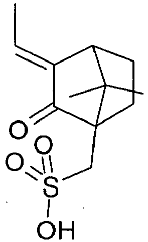 bedeutet, und/oder deren Salze, Tautomere, Stereoisomere und/oder Solvate, einschließlich deren Mischungen in allen Verhältnissen, als proteinadhäsive Wirkstoffe.

2. Nicht-therapeutische Verwendung von Verbindungen der Formel I, 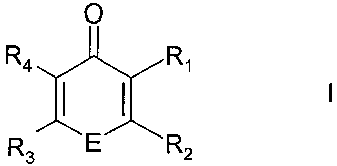 wobei
E NR₅ oder O bedeutet,
R₂, R₃ oder R₄ jeweils unabhängig voneinander -H, -A, -OA, -(CH₂)ₚ-OH,
-C(O)OA, COOH oder COOX bedeuten,
p eine ganze Zahl von 1 bis 4 bedeutet,
X das Gegenion zu der [COO-]-Gruppe ist,
R₅ A bedeutet,
A Alkyl mit 1 bis 20 C-Atomen bedeutet,
R₁ ein Substituent der Formel II, III, IV, V, VI, VII, VIII, IX, X, XI, XII, XIII, XIV oder XV ist, 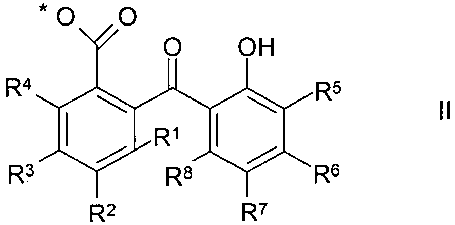 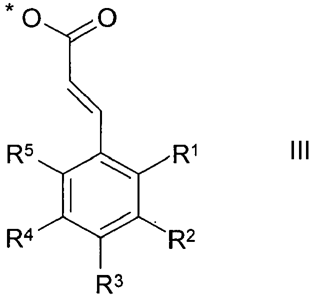 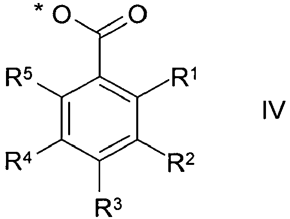 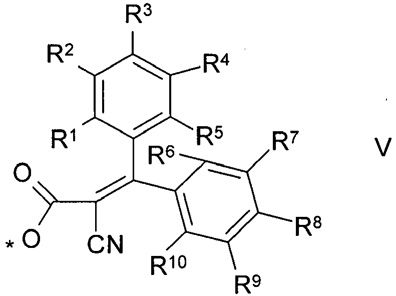 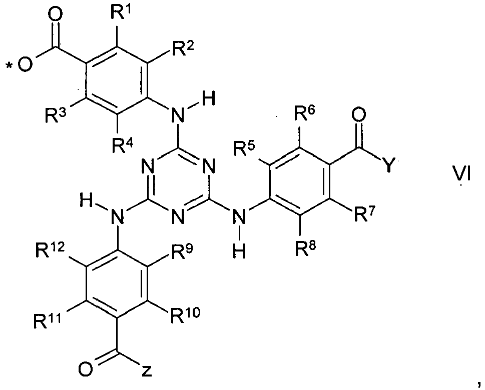 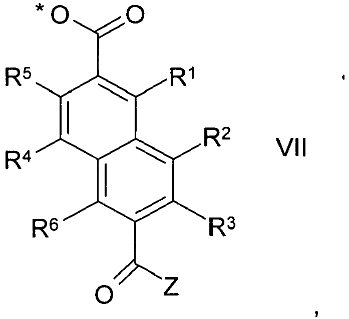 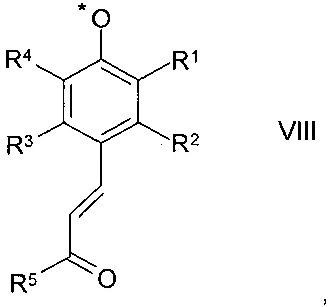 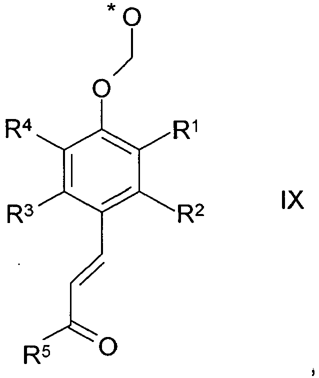 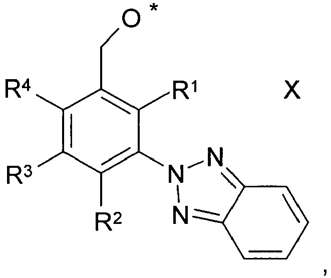 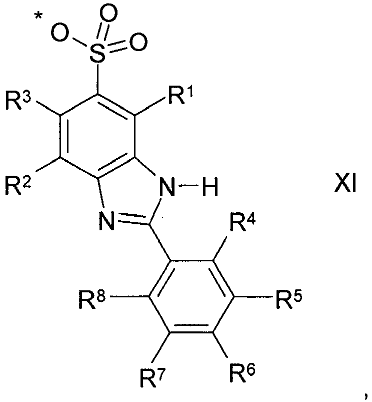 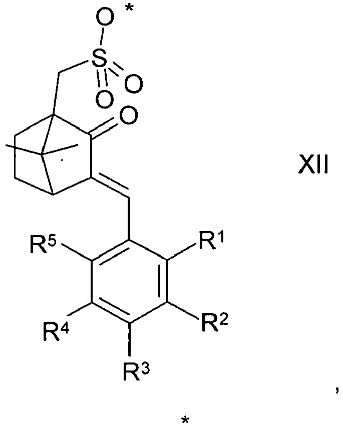 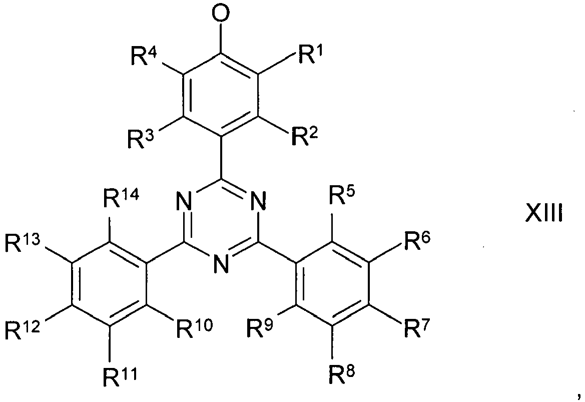 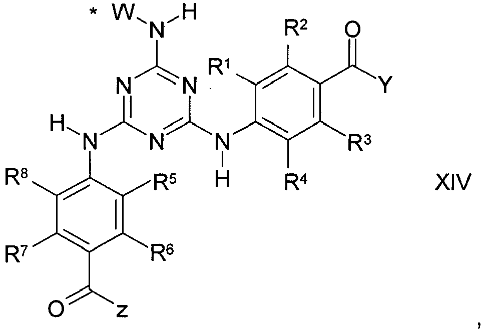 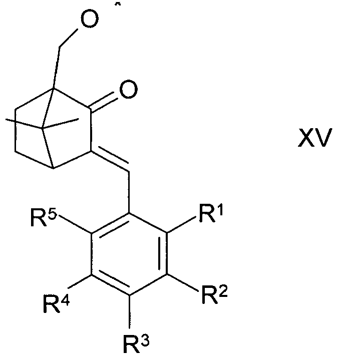 wobei
R¹ bis R¹⁴ jeweils unabhängig voneinander -H, -OH, -OA, -A, -NH₂,-NHA, -NA₂, -NH-(CH₂-CH₂-O)ₙ-H, -N[(CH₂-CH₂-O)ₙ-H]₂, -[NHA₂]X,-[NA₃]X, -SO₃H, -[SO₃]X, 2H-Benzotriazol-2-yl- oder 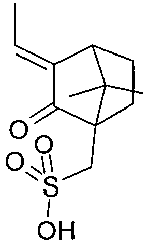 bedeuten,
n eine ganze Zahl von 1 bis 25 ist,
X das Gegenion zu den Kationen [NHA₂]⁺ und [NA₃]⁺ oder dem Anion [SO₃]⁻ is und
Y und Z jeweils unabhängig voneinander die Struktureinheit I-1 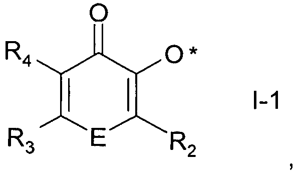 A, Hydroxy, -OA oder -NH-C(CH₃)₃ bedeuten, wobei A,
R₂, R₃, R₄ und E eine zuvor genannte Bedeutung haben,
W -(CH₂)ₘ-O oder -(CH₂)ₒ-C(O)-O bedeutet und
m und o jeweils unabhängig voneinander eine ganze Zahl von 1 bis 4 bedeuten,
mit der Bedingung, dass mindestens ein Susbstituent der Substituenten R¹ bis R¹⁴ in den Formeln II, III, IV, VII, X, XII und XV OH, -OA, -A, -NH₂, -NHA, -NA₂, -NH-(CH₂-CH₂-O)ₙ- -N[(CH₂-CH₂-O)ₙ-H]₂, -[NHA₂]X, -[NA₃]X, -SO₃H, -[SO₃]X, 2H-Benzotriazol-2-yl- oder 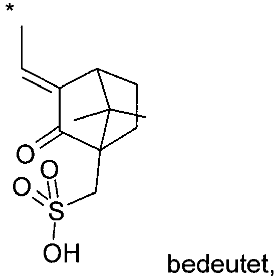 und/oder deren Salze, Tautomere, Stereoisomere und/oder Solvate, einschließlich deren Mischungen in allen Verhältnissen, zum Schutz der Haut, des Haares und/oder der Nägel vor UV-induzierten Schäden.

3. Verbindungen der Formel I 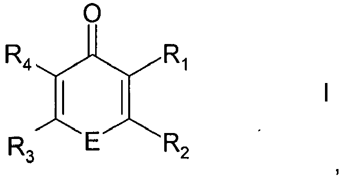 wobei
E NR₅ oder O bedeutet,
R₁ ein Substituent der Formel II, III, V, VI, VII, VIII, IX, X, XI, XII, XIII, XIV oder XV ist, 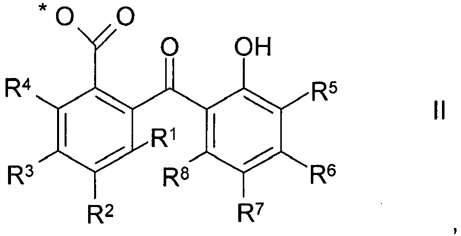 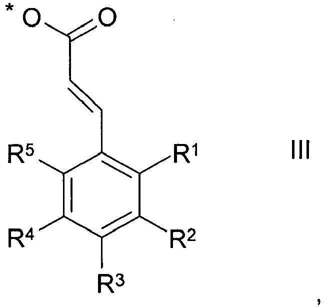 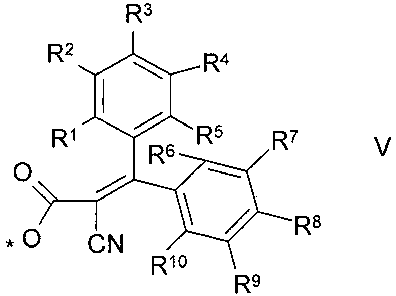 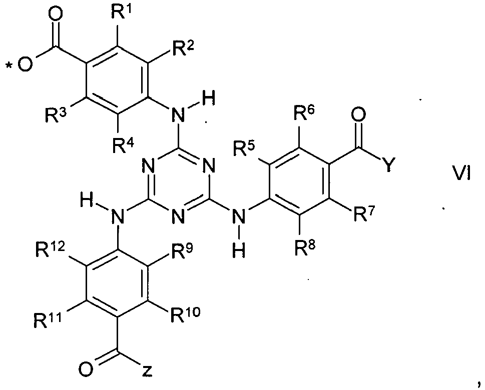 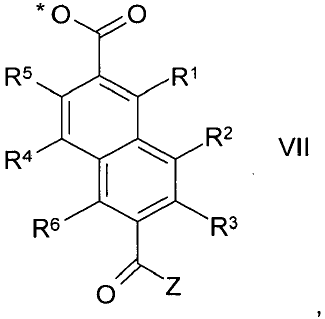 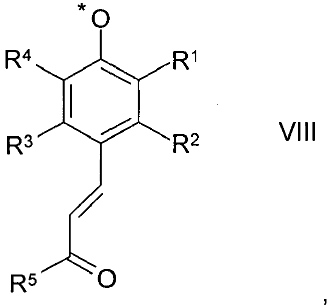 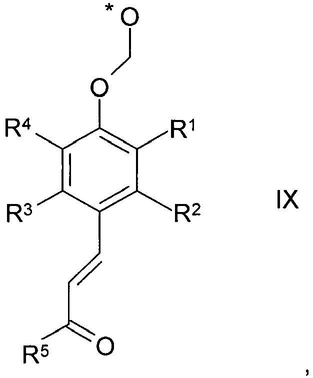 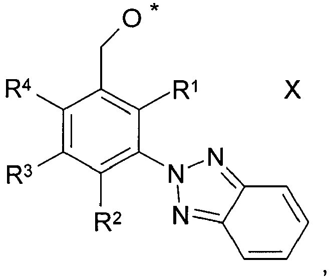 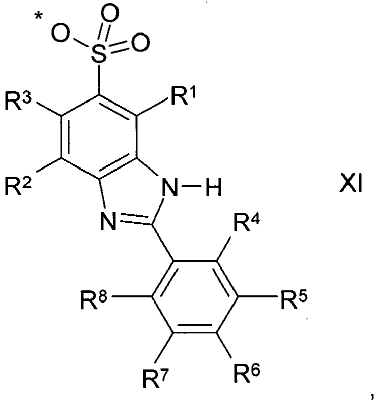 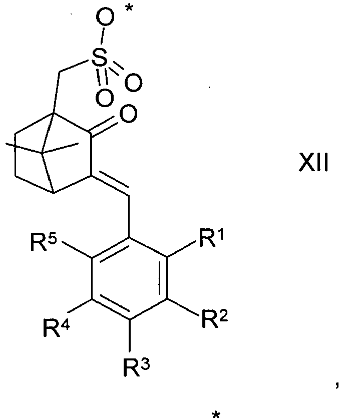 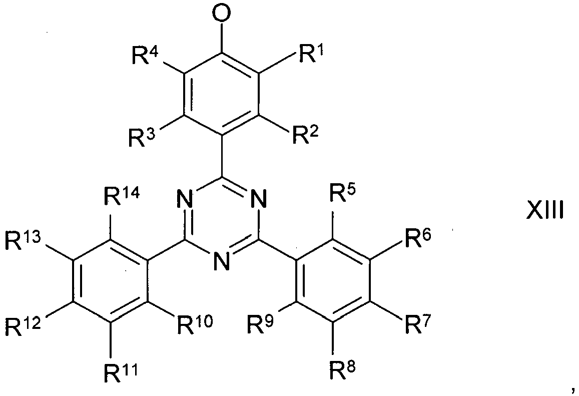 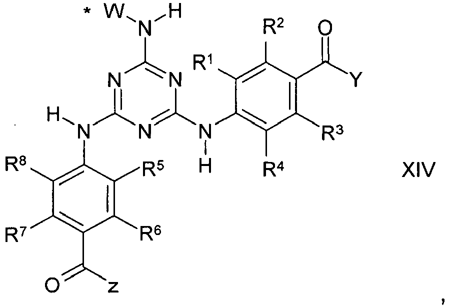 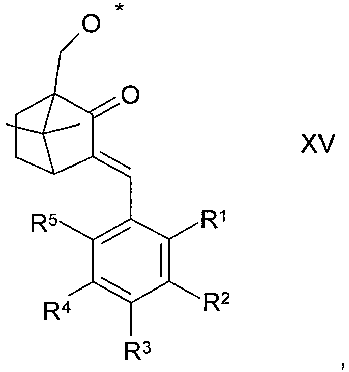 wobei
R¹ bis R¹⁴ jeweils unabhängig voneinander -H, -OH, -OA, -A, -NH₂,-NHA, -NA₂, -NH-(CH₂-CH₂-O)ₙ-H, -N[(CH₂-CH₂-O)ₙ-H]₂, -[NHA₂]X,-[NA₃]X, -SO₃H, -[SO₃]X, 2H-Benzotriazol-2-yl- oder 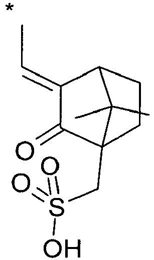 bedeuten,
n eine ganze Zahl von 1 bis 25 ist,
X das Gegenion zu den Kationen [NHA₂]⁺ und [NA₃]⁺ oder dem Anion [SO₃]⁻ ist und
Y und Z jeweils unabhängig voneinander die Struktureinheit I-1 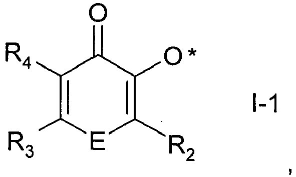 A, Hydroxy, -OA oder -NH-C(CH₃)₃ bedeuten, W -(CH₂)ₘ-O oder -(CH₂)ₒ-C(O)-O bedeutet und
m und o jeweils unabhängig voneinander eine ganze Zahl von 1 bis 4 bedeuten,
mit der Bedingung, dass mindestens ein Susbstituent der Substituenten R¹ bis R¹⁴ in den Formeln II, III, VII, X, XII und XV
OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X, 2H-Benzotriazol-2-yl- oder 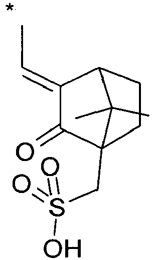 bedeutet, oder
R₁ ein Substituent der Formel IV bedeutet, 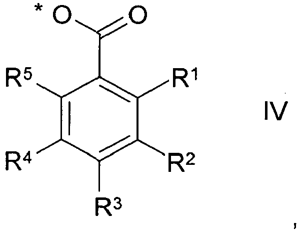 wobei
R² 2H-Benzotriazol-2-yl bedeutet, R⁴ und R⁵ H bedeuten, ein Substituent von R¹ oder R³ H bedeutet und der andere Substituent -OH, -OA, -A,-NH₂, -NHA, -NA₂, -NH-(CH₂-CH₂-O)ₙ-H oder -N[(CH₂-CH₂-O)ₙ-H]₂ bedeutet,
R₂ oder R₄ jeweils unabhängig voneinander -H, -A, -OA, -(CH₂)ₚ-OH,-C(O)OA, COOH oder COOX bedeuten,
R₃ -H, Hydroxymethyl, -C(O)OEthyl oder -C(O)OMethyl bedeutet,
p eine ganze Zahl von 1 bis 4 bedeutet,
X das Gegenion zu der [COO-]-Gruppe ist,
R₅ A bedeutet und
A Alkyl mit 1 bis 20 C-Atomen bedeutet,
wobei die Verbindung (E)-3-(4-Hydroxy-phenyl)-acrylsäure-2-methyl-4-oxo-4H-pyran-3-yl-ester ausgenommen ist.

4. Verbindungen nach Anspruch 3, **dadurch gekennzeichnet, dass** in Formel II R⁶ vorzugsweise -OH, -OA, -A, -NH₂, -NHA, -NA₂, -NH-(CH₂-CH₂-O)ₙ-H, -N[(CH₂-CH₂-O)ₙ-H] SO₃H, SO₃X oder 2H-Benzotriazol-2-yl und/oder
in Formel III R³ -OH, -OA, -A, -NH₂, -NHA, -NA₂, -NH-(CH₂-CH₂-O)ₙ-H,-N[(CH₂-CH₂-O)ₙ-H]₂, SO₃H, SO₃X oder 2H-Benzotriazol-2-yl bedeutet und/oder
die Substituenten R², R⁴, R⁷ und R⁹ in Formel V H und die Substituenten R¹, R³, R⁵, R⁶, R⁸ und R¹⁰ jeweils unabhängig voneinander H, -OH, -OA, -A, -NH₂, -NHA, -NA₂, -NH-(CH₂-CH₂-O)ₙ-H, -N[(CH₂-CH₂-O)ₙ-H]₂,-[NHA₂]X, -[NA₃]X, -SO₃H, -[SO₃]X oder 2H-Benzotriazol-2-yl sind und/oder
die Substituenten R², R⁴, R⁵, R⁸, R⁹ und R¹² der Formel VI jeweils unabhängig voneinander H oder OH und die Substituenten R¹, R³, R⁶, R⁷, R¹⁰ und R¹¹ jeweils unabhängig voneinander H, -OH, -OA, -A, -NH₂,-NHA, -NA₂, -NH-(CH₂-CH₂-O)ₙ-H, -N[(CH₂-CH₂-O)ₙ-H]₂, -[NHA₂]X,-[NA₃]X, -SO₃H, -[SO₃]X oder 2H-Benzotriazol-2-yl und Y und Z Hydroxy, -OA oder -NH-C(CH₃)₃ sind und/oder
die Substituenten R² und R⁴ der Formel VII H sind und mindestens einer der Substituenten R¹, R³, R⁵ und R⁶ -OH, -OA, -A, -NH₂, -NHA, -NA₂,-NH-(CH₂-CH₂-O)n-H, -N[(CH₂-CH₂-O)ₙ-H]₂, SO₃H, SO₃X oder 2H-Benzotriazol-2-yl bedeutet und/oder
in Formel VIII R⁵ -OH, -OA, -A, -NH₂, -NHA, -NA₂, -NH-(CH₂-CH₂-O)ₙ-H, -N[(CH₂-CH₂-O)ₙ-H]₂, SO₃H, SO₃X oder 2H-Benzotriazol-2-yl bedeutet und/oder
in Formel IX R⁵ -OH, -OA, -A, -NH₂, -NHA, -NA₂, -NH-(CH₂-CH₂-O)ₙ-H,-N[(CH₂-CH₂-O)ₙ-H]₂, SO₃H, SO₃X oder 2H-Benzotriazol-2-yl bedeutet und/oder
in Formel X R¹ und/oder R₂ -OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X oder 2H-Benzotriazol-2-yl bedeuten und/oder
in Formel XI R⁶ H, -OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X oder 2H-Benzotriazol-2-yl bedeutet und/oder
in Formel XII R³ -OH, -OA, -A, -NH₂, -NHA, -NA₂, -NH-(CH₂-CH₂-O)ₙ-H,-N[(CH₂-CH₂-O)ₙ-H]₂, -[NHA₂]X, -[NA₃]X, -SO₃H, -[SO₃]X, 2H-Benzotriazol-2-yl oder 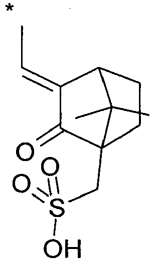 bedeutet und/oder in Formel XIII R⁵, R⁷, R¹² und R¹⁴ jeweils unabhängig voneinander vorzugsweise -OH, -OA, -A, -NH₂, -NHA, -NA₂, -NH-(CH₂-CH₂-O)ₙ-H,-N[(CH₂-CH₂-O)ₙ-H]₂ oder 2H-Benzotriazol-2-y bedeuten und/oder
in Formel XIV W CH₂-CH₂-O oder CH₂-CH₂-CH₂-C(O)O bedeutet und/oder
in Formel XV R³ -OH, -OA, -A, -NH₂, -NHA, -NA₂, -NH-(CH₂-CH₂-O)ₙ-H,-N[(CH₂-CH₂-O)ₙ-H]₂, -[NHA₂]X, -[NA₃]X, -SO₃H, -[SO₃]X, 2H-Benzotriazol-2-yl oder 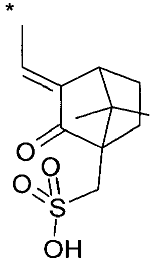 bedeutet.

5. Verfahren zur Herstellung von Verbindungen nach Anspruch 3 oder 4, **dadurch gekennzeichnet, dass** eine Verbindung der Formel XVI 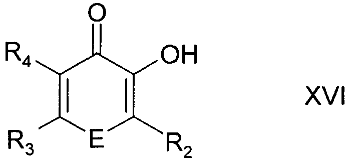 worin R₂, R₃, R₄ und E eine in Anspruch 3 angegebene Bedeutung haben,
mit einer Verbindung der Formel XVII
R₁-M XVII
umgesetzt wird,
worin R₁ eine in Anspruch 3 oder 4 beschriebene Bedeutung hat, wobei die Teilformeln XI und XII ausgeschlossen sind, und
M Alkalimetall- oder Erdalkalimetallkation oder H bedeutet oder
eine Verbindung der Formel XVI, worin R₂, R₃, R₄ und E eine in Anspruch 3 angegebene Bedeutung haben,
mit einem Säurehalogenid oder einem Aktivester der freien Säuren, abgeleitet von den Formeln II, III, IV, V, VI, VII, XI, XII und XIV des Anspruchs 3 oder 4 umgesetzt wird.

6. Zubereitung enthaltend mindestens eine Verbindung nach einem oder mehreren der Ansprüche 3 oder 4.

7. Zubereitung nach Anspruch 6, **dadurch gekennzeichnet, dass** es einen kosmetischen, dermatologischen oder pharmakologisch verträglichen Träger enthält.

8. Zubereitung nach Anspruch 6 oder 7, **dadurch gekennzeichnet, dass** mindestens ein weiterer Wirkstoff enthalten ist, ausgewählt aus der Gruppe UV-Filter, Antioxidantien, Vitamine, Anti-aging-Wirkstoffe, Anti-Cellulite-Wirkstoffe, Selbstbräunungssubstanzen, Antipigmentierstoffe oder hautaufhellende Stoffe.

9. Zubereitung enthaltend (E)-3-(4-hydroxy-phenyl)-acrylsäure-2-methyl-4-oxo-4H-pyran-3-yl-ester oder 2-Hydroxy-benzoesäure 2-methyl-4-oxo-4H-pyran-3-yl-ester, einen kosmetischen, dermatologischen oder pharmakologisch verträglichen Träger und mindestens einen weiteren Wirkstoff, ausgewählt aus der Gruppe UV-Filter, Antioxidantien, Vitamine, Anti-aging-Wirkstoffe, Anti-Cellulite-Wirkstoffe, Selbstbräunungssubstanzen, Antipigmentierstoffe oder hautaufhellender Stoffe.

10. Verfahren zur Herstellung einer Zubereitung nach einem oder mehreren der Ansprüche 6 bis 9, **dadurch gekennzeichnet, dass** mindestens eine Verbindung nach einem oder mehreren der Ansprüche 3 oder 4 oder (E)-3-(4-Hydroxy-phenyl)-acrylsäure-2-methyl-4-oxo-4H-pyran-3-yl-ester oder 2-Hydroxy-benzoesäure 2-methyl-4-oxo-4H-pyran-3-yl-ester mit einem Träger und gegebenenfalls mit weiteren Aktiv- oder Hilfsstoffen vermischt wird.

11. Verwendung der Struktureinheit der Formel I-1 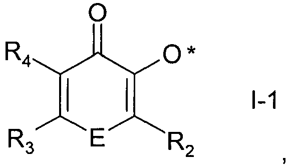 worin R₂, R₃, R₄ und E eine in Anspruch 1 angegebene Bedeutung haben, als Linker zur Funktionalisierung von proteinhaltigen Matrices, wobei das Symbol* die Anknüpfungsstelle in Form einer kovalenten Einfachbindung zu einem Molekül bedeutet, welches an die Matrix adsorbiert oder kovalent gebunden werden soll.

## Claims

1. Non-therapeutic use of compounds of the formula I, 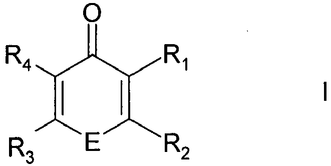 where
E denotes NR₅ or O,
R₂, R₃ or R₄ each, independently of one another, denote -H, -A, -OA, -(CH₂)ₚ-OH, -C(O)OA, COOH or COOX,
p denotes an integer from 1 to 4,
X is the counterion to the [COO⁻] group,
R₅ denotes A,
A denotes alkyl having 1 to 20 C atoms and
R₁ is a substituent of the formula II, III, IV, V, VI, VII, VIII, IX, X, XI, XII, XIII, XIV or XV, 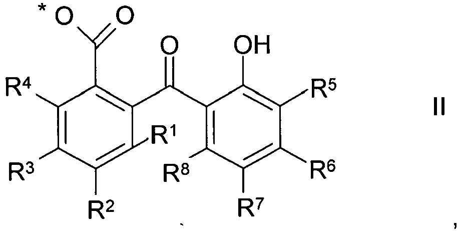 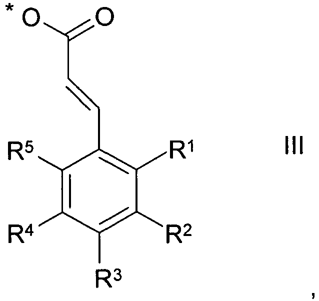 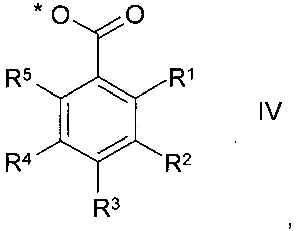 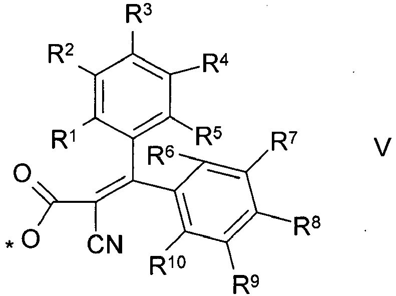 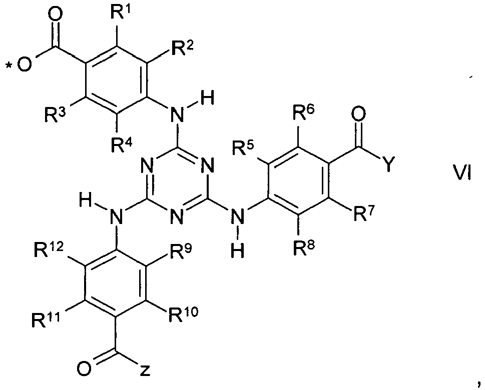 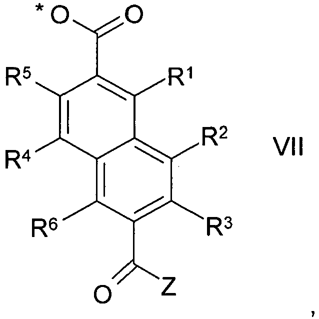 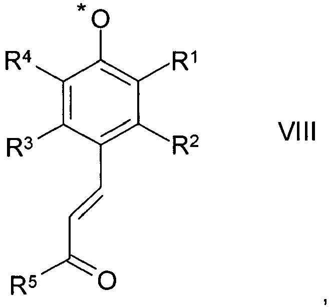 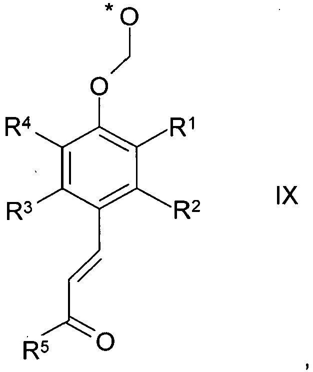 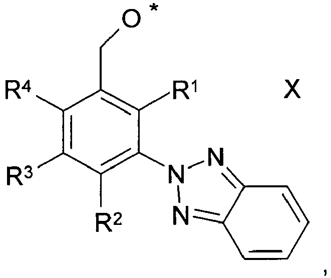 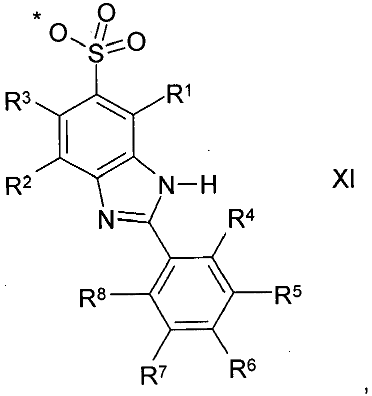 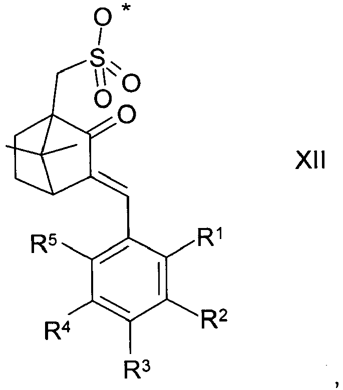 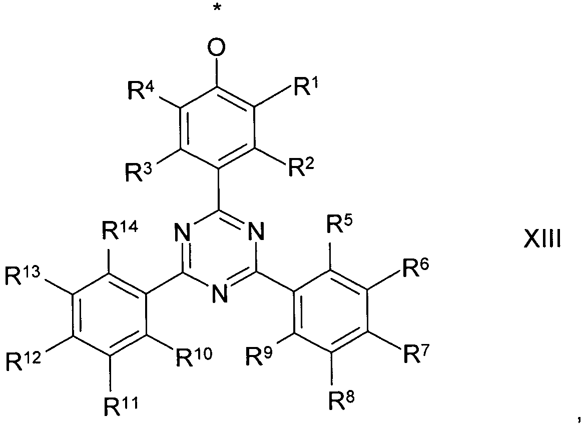 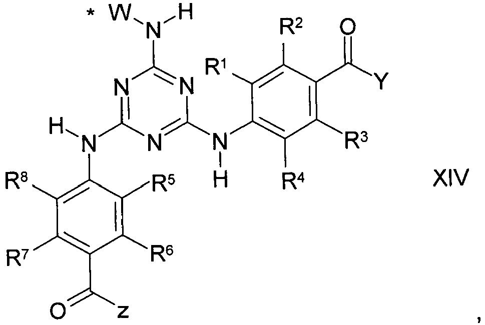 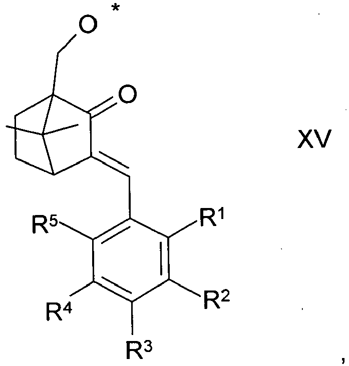 where
R¹ to R¹⁴ each, independently of one another, denote -H, -OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X 2H-benzotriazol-2-yl- or 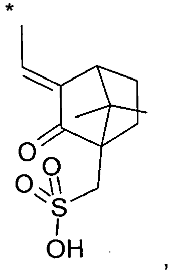
n is an integer from 1 to 25,
X is the counterion to the cations [NHA₂]⁺ and [NA₃]⁺ or the anion [SO₃]⁻ and
Y and Z each, independently of one another, denote the structural unit I-1 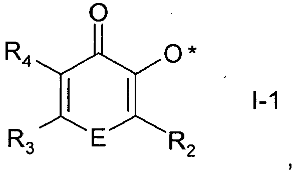 A, hydroxyl, -OA or -NH-C(CH₃)₃, where A, R₂, R₃, R₄ and E have a meaning given above,
W denotes -(CH₂)ₘ-O or -(CH₂)ₒ-C(O)-O and
m and o each, independently of one another, denote an integer from 1 to 4,
with the proviso that at least one substituent of the substituents R¹ to R¹⁴ in the formulae II, III, IV, VII, X, XII and XV denotes
OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X, 2H-benzotriazol-2-yl- or 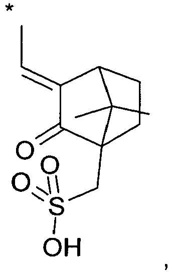 and/or salts, tautomers, stereoisomers and/or solvates thereof, including mixtures thereof in all ratios, as protein-adhesive active compounds.

2. Non-therapeutic use of compounds of the formula I, 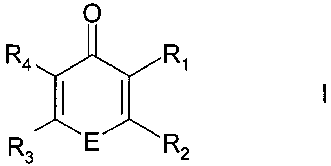 where
E denotes NR₅ or O,
R₂, R₃ or R₄ each, independently of one another, denote -H, -A, -OA, -(CH₂)ₚ-OH, -C(O)OA, COOH or COOX,
p denotes an integer from 1 to 4,
X is the counterion to the [COO⁻] group,
R₅ denotes A,
A denotes alkyl having 1 to 20 C atoms,
R₁ is a substituent of the formula II, III, IV, V, VI, VII, VIII, IX, X, XI, XII, XIII, XIV or XV, 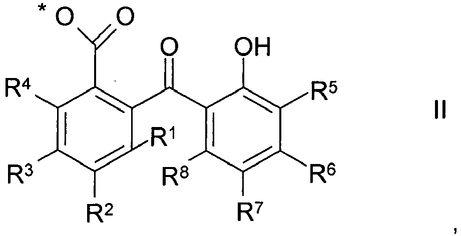 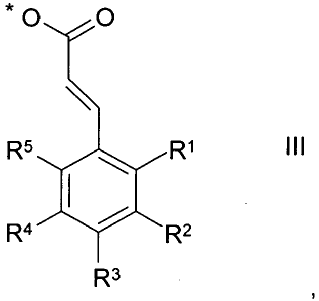 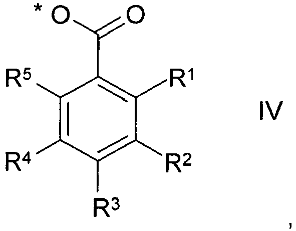 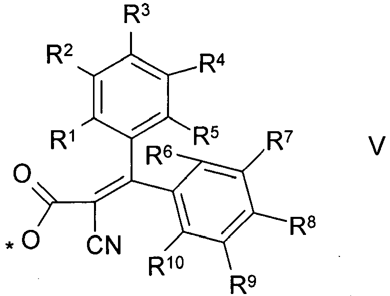 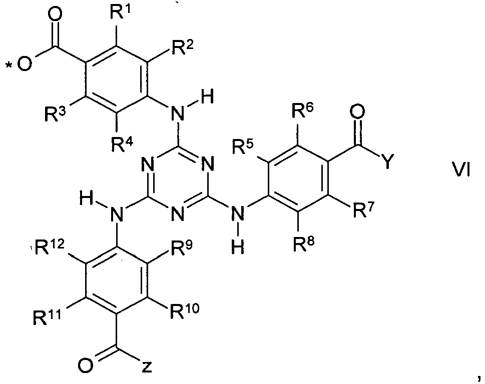 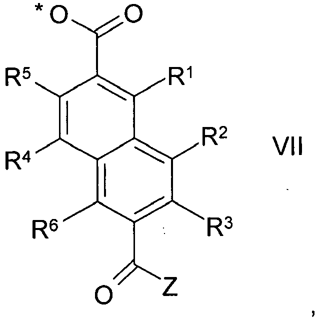 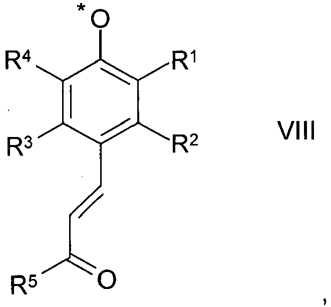 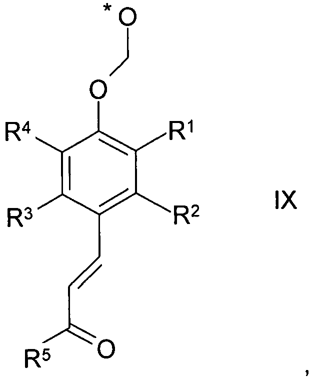 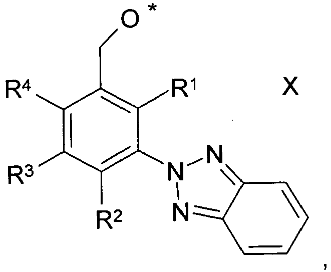 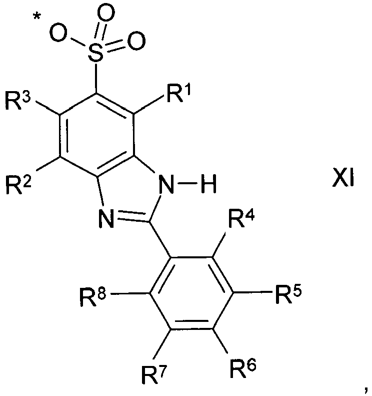 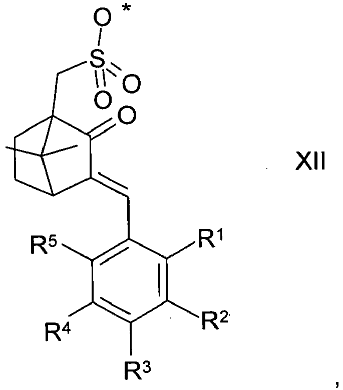 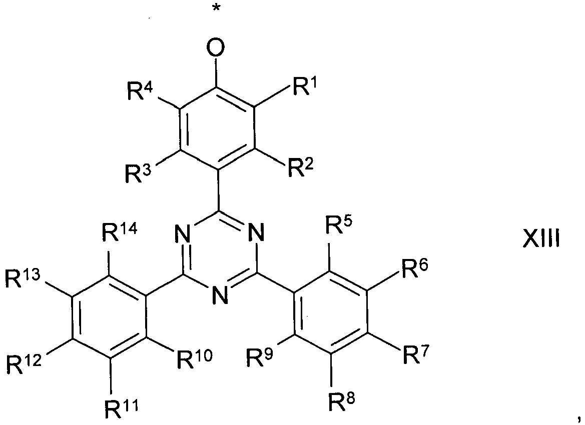 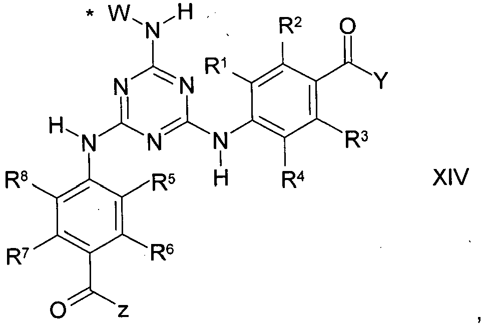 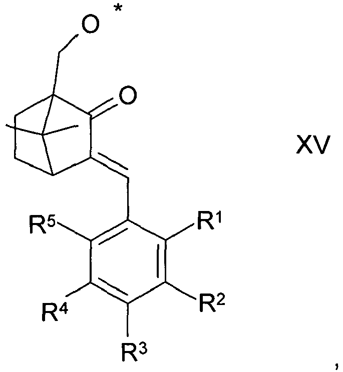 where
R¹ to R¹⁴ each, independently of one another, denote -H, -OH, -OA, -A, -NH₂, -NHA, -NA₂, -NH-(CH₂-CH₂-O)ₙ-H, -N[(CH₂-CH₂-O)ₙ-H] -[NHA₂]X, -[NA₃]X, -SO₃H, -[SO₃]X 2H-benzotriazol-2-yl- or 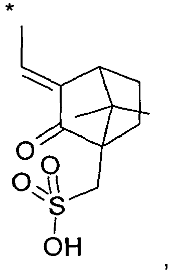
n is an integer from 1 to 25,
X is the counterion to the cations [NHA₂]⁺ and [NA₃]⁺ or the anion [SO₃]⁻ and
Y and Z each, independently of one another, denote the structural unit I-1 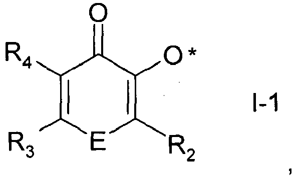 A, hydroxyl, -OA or -NH-C(CH₃)₃, where A, R₂, R₃, R₄ and E have a meaning given above, W denotes -(CH₂)ₘ-O or -(CH₂)ₒ-C(O)-O and
m and o each, independently of one another, denote an integer from 1 to 4,
with the proviso that at least one substituent of the substituents R¹ to R¹⁴ in the formulae II, III, IV, VII, X, XII and XV denotes OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X, 2H-benzotriazol-2-yl- or 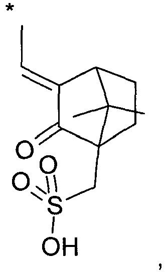
and/or salts, tautomers, stereoisomers and/or solvates thereof, including mixtures thereof in all ratios, for protection of the skin, hair and/or nails against UV-induced damage.

3. Compounds of the formula I 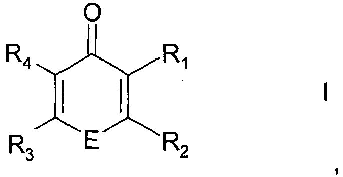 where
E denotes NR₅ or O,
R₁ is a substituent of the formula II, III, V, VI, VII, VIII, IX, X, XI, XII, XIII, XIV or XV, 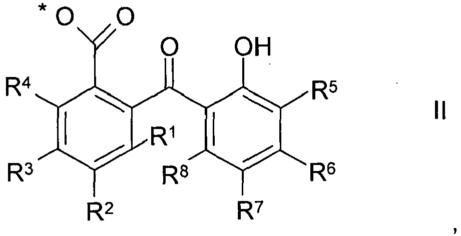 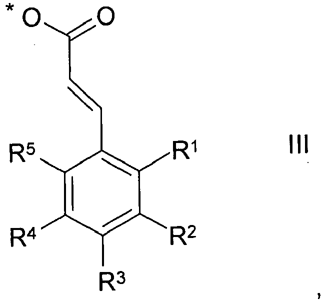 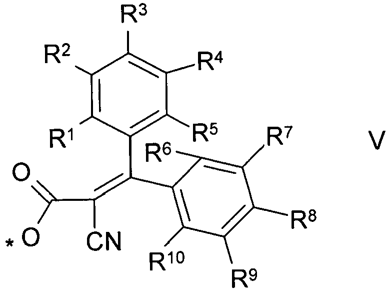 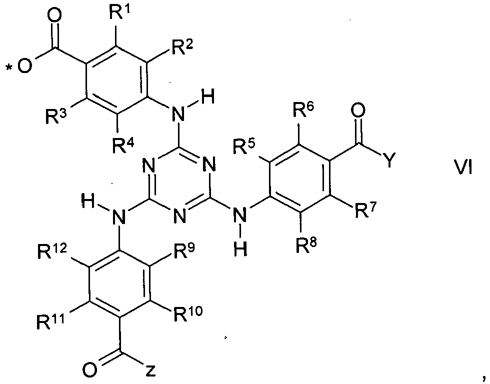 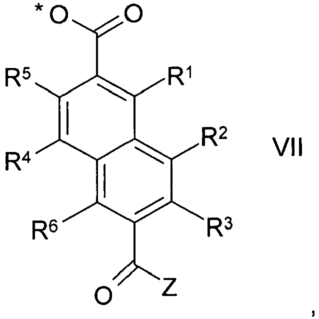 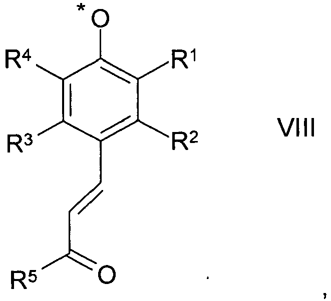 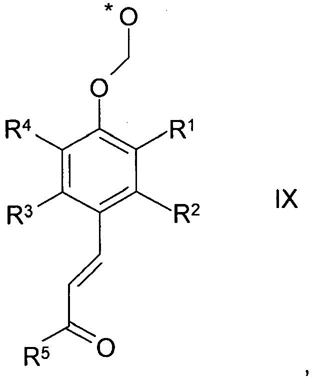 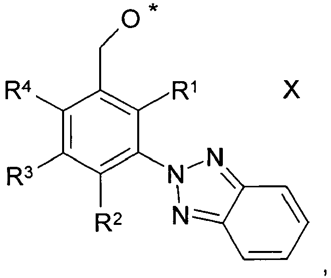 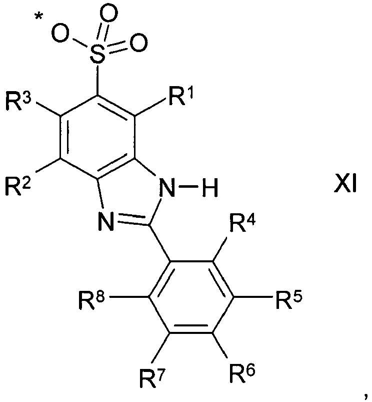 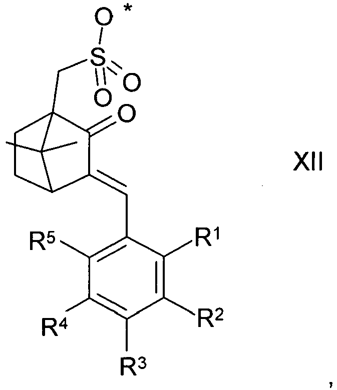 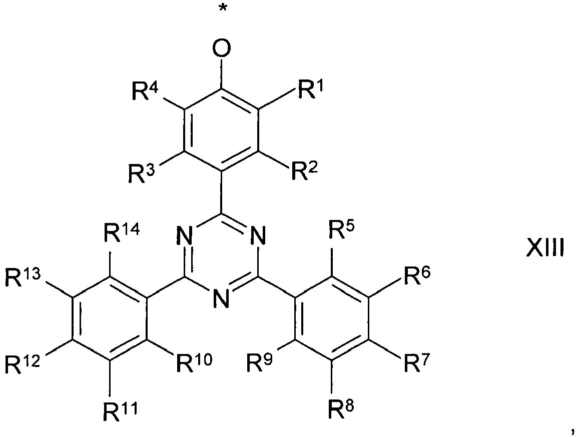 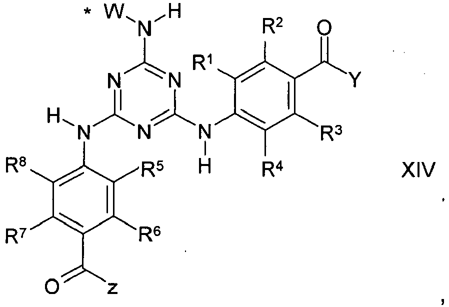 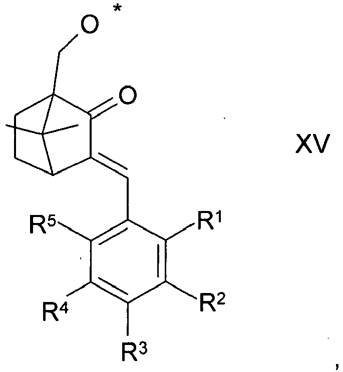 where
R¹ to R¹⁴ each, independently of one another, denote -H, -OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X, 2H-benzotriazol-2-yl- or 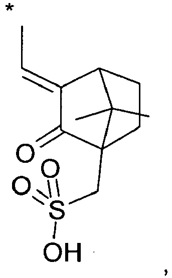
n is an integer from 1 to 25,
X is the counterion to the cations [NHA₂]⁺ and [NA₃]⁺ or the anion [SO₃]⁻ and
Y and Z each, independently of one another, denote the structural unit I-1 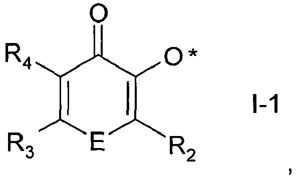 A, hydroxyl, -OA or -NH-C(CH₃)₃, W denotes -(CH₂)ₘ-O or -(CH₂)o-C(O)-O and
m and o each, independently of one another, denote an integer from 1 to 4,
with the proviso that at least one substituent of the substituents R¹ to R¹⁴ in the formulae II, III, VII, X, XII and XV denotes
OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X, 2H-benzotriazol-2-yl- or 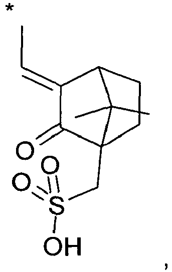 or
R₁ denotes a substituent of the formula IV, 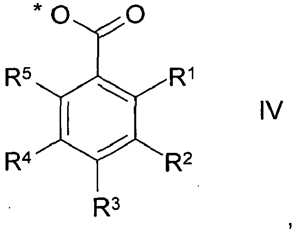 where
R₂ denotes 2H-benzotriazol-2-yl, R⁴ and R⁵ denote H, one substituent from R¹ or R³ denotes H and the other substutuent denotes -OH, -OA, -A, -NH₂, -NHA, -NA₂, -NH-(CH₂-CH₂-O)ₙ-H or -N[(CH₂-CH₂-O)ₙ-H]₂,
R₂ or R₄ each, independently of one another, denote -H, -A, -OA, -(CH₂)ₚ-OH, -C(O)OA, COOH or COOX,
R³ denotes -H, hydroxymethyl, -C(O)Oethyl or -C(O)Omethyl,
p denotes an integer from 1 to 4,
X is the counterion to the [COO-] group,
R₅ denotes A and
A denotes alkyl having 1 to 20 C atoms,
where the compound 2-methyl-4-oxo-4H-pyran-3-yl (E)-3-(4-hydroxy-phenyl)acrylate is excluded.

4. Compounds according to Claim 3, **characterised in that**, in formula II, R⁶ preferably denotes -OH, -OA, -A, -NH₂, -NHA, -NA₂, -NH-(CH₂-CH₂-O)ₙ-H, -N[(CH₂-CH₂-O)ₙ-H]₂, SO₃H, SO₃X or 2H-benzotriazol-2-yl and/or
in formula III, R³ denotes -OH, -OA, -A, -NH₂, -NHA, -NA₂, -NH-(CH₂-CH₂-O)ₙ-H, -N[(CH₂-CH₂-O)ₙ-H]₂, SO₃H, SO₃X or 2H-benzotriazol-2-yl and/or
the substituents R², R⁴, R⁷ and R⁹ in formula V are H and the substituents R¹, R³, R⁵, R⁶, R⁸ and R¹⁰ are each, independently of one another, H, -OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X or 2H-benzotriazol-2-yl and/or
the substituents R², R⁴, R⁵, R⁸, R⁹ and R¹² in formula VI are each, independently of one another, H or OH and the substituents R¹, R³, R⁶, R⁷, R¹⁰ and R¹¹ are each, independently of one another, H, -OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X or 2H-benzotriazol-2-yl and Y and Z are hydroxyl, -OA or -NH-C(CH₃)₃ and/or
the substituents R₂ and R⁴ in formula VII are H and at least one of the substituents R¹, R³, R⁵ and R⁶ denotes -OH, -OA, -A, -NH₂, -NHA, -NA₂, -NH-(CH₂-CH₂-O)ₙ-H, -N[(CH₂-CH₂-O)ₙ-H]₂, SO₃H, SO₃X or 2H-benzotriazol-2-yl and/or
in formula VIII, R⁵ denotes -OH, -OA, -A, -NH₂, -NHA, -NA₂, -NH-(CH₂-CH₂-O)ₙ-H, -N[(CH₂-CH₂-O)ₙ-H]₂, SO₃H SO₃X or 2H-benzotriazol-2-yl and/or
in formula IX, R⁵ denotes -OH, -OA, -A, -NH₂, -NHA, -NA₂, -NH-(CH₂-CH₂-O)ₙ-H, -N[(CH₂-CH₂-O)ₙ-H]₂, SO₃H, SO₃X or 2H-benzotriazol-2-yl and/or
in formula X, R¹ and/or R₂ denote -OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X or 2H-benzotriazol-2-yl and/or
in formula XI, R⁶ denotes H, -OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X or 2H-benzotriazol-2-yl and/or
in formula XII, R³ denotes -OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X, 2H-benzotriazol-2-yl or 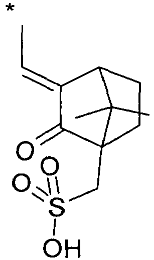 and/or in formula XIII, R⁵, R⁷, R¹² and R¹⁴ each, independently of one another, preferably denote -OH, -OA, -A, -NH₂, -NHA, -NA₂, -NH-(CH₂-CH₂-O)ₙ-H, -N[(CH₂-CH₂-O)ₙ-H]₂ or 2H-benzotriazol-2-y and/or
in formula XIV, W denotes CH₂-CH₂-O or CH₂-CH₂-CH₂-C(O)O and/or
in formula XV, R³ denotes -OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X, 2H-benzotriazol-2-yl or

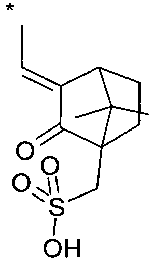

5. Process for the preparation of compounds according to Claim 3 or 4, **characterised in that** a compound of the formula XVI 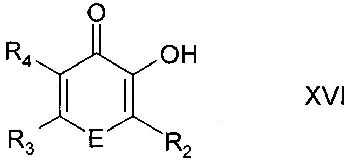
in which R₂, R₃, R₄ and E have a meaning indicated in Claim 3, is reacted with a compound of the formula XVII
R₁-M XVII,
in which R₁ has a meaning described in Claim 3 or 4, where the part-formulae XI and XII are excluded, and
M denotes alkali metal or alkaline-earth metal cation or H or
a compound of the formula XVI, in which R₂, R₃, R₄ and E have a meaning indicated in Claim 3,
is reacted with an acid halide or an active ester of the free acids derived from the formulae II, III, IV, V, VI, VII, XI, XII and XIV of Claim 3 or 4.

6. Preparation comprising at least one compound according to one or more of Claims 3 or 4.

7. Preparation according to Claim 6, **characterised in that** it comprises a cosmetic, dermatological or pharmacologically tolerated vehicle.

8. Preparation according to Claim 6 or 7, **characterised in that** at least one further active compound is present selected from the group UV filters, antioxidants, vitamins, antiageing active compounds, anticellulite active compounds, self-tanning substances, antipigmentation substances or skin-lightening substances.

9. Preparation comprising 2-methyl-4-oxo-4H-pyran-3-yl (E)-3-(4-hydroxy-phenyl)acrylate or 2-methyl-4-oxo-4H-pyran-3-yl 2-hydroxybenzoate, a cosmetic, dermatological or pharmacologically tolerated vehicle and at least one further active compound selected from the group UV filters, antioxidants, vitamins, antiageing active compounds, anticellulite active compounds, self-tanning substances, antipigmentation substances or skin-lightening substances.

10. Process for the preparation of a preparation according to one or more of Claims 6 to 9, **characterised in that** at least one compound according to one or more of Claims 3 or 4 or 2-methyl-4-oxo-4H-pyran-3-yl (E)-3-(4-hydroxyphenyl)acrylate or 2-methyl-4-oxo-4H-pyran-3-yl 2-hydroxybenzoate is mixed with a vehicle and optionally with further active substances or assistants.

11. Use of the structural unit of the formula I-1 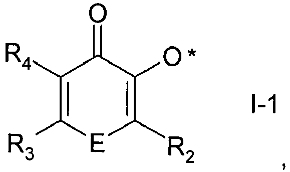 in which R₂, R₃, R₄ and E have a meaning indicated in Claim 1, as linker for the functionalisation of protein-containing matrices, where the symbol * denotes the linking site in the form of a covalent single bond to a molecule which is intended to be adsorbed onto or covalently bonded to the matrix.

## Revendications

1. Utilisation non thérapeutique de composés de formule I, 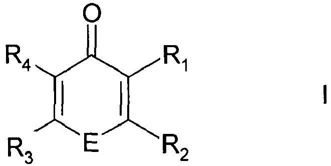 où
E désigne NR₅ ou O,
R₂, R₃ ou R₄ désignent chacun, indépendamment les uns des autres, -H, -A, -OA, -(CH₂)ₚ-OH, -C(O)OA, COOH ou COOX,
p désigne un nombre entier allant de 1 à 4,
X est le contre-ion du groupement [COO⁻],
R₅ désigne A,
A désigne alkyle ayant de 1 à 20 atomes de C et
R₁ est un substituant de formule II, III, IV, V, VI, VII, VIII, IX, X, XI, XII, XIII, XIV ou XV, 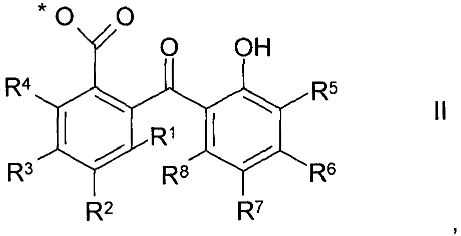 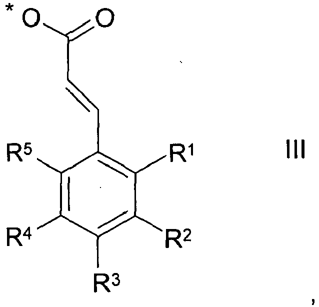 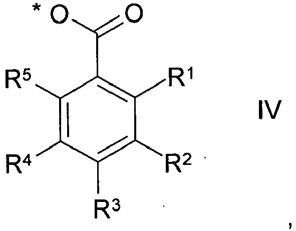 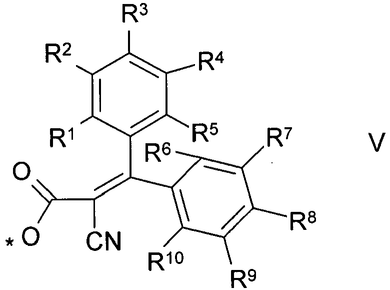 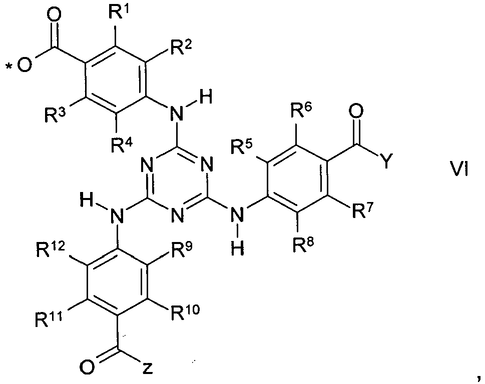 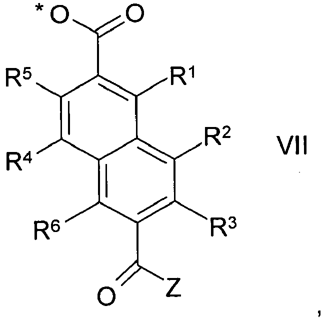 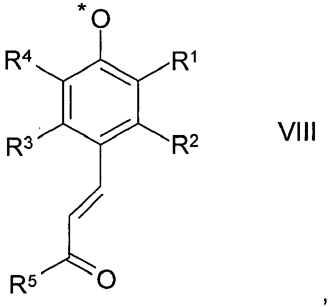 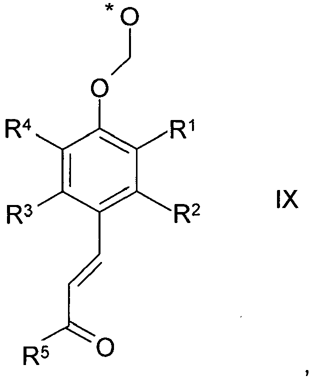 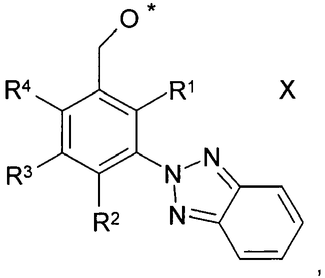 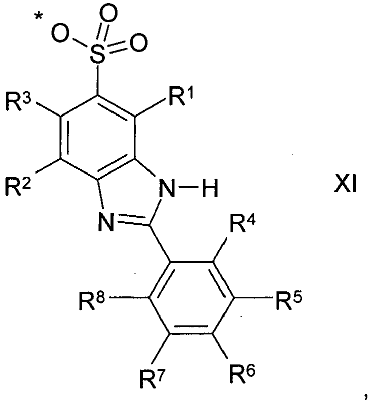 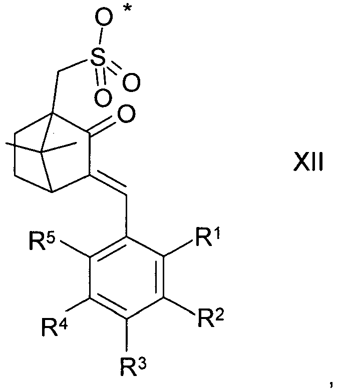 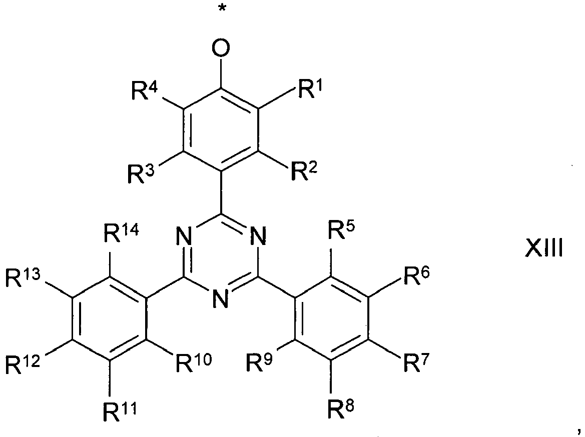 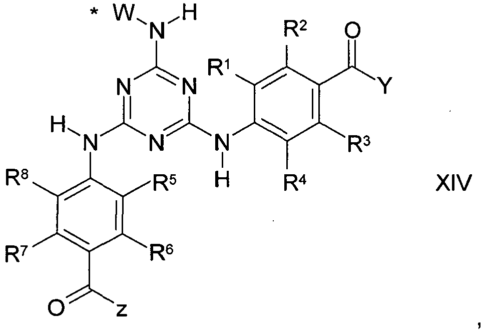 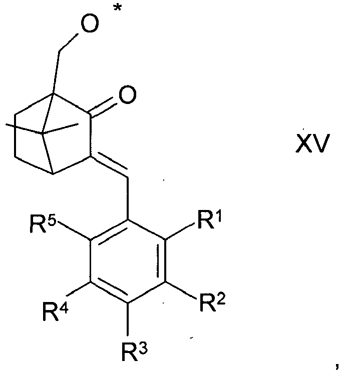 où
R¹ à R¹⁴ désignent chacun, indépendamment les uns des autres, -H, -OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X 2H-benzotriazol-2-yl- ou 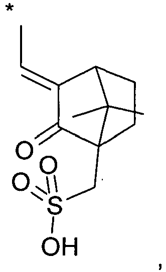
n est un nombre entier allant de 1 à 25,
X est le contre-ion des cations [NHA₂]⁺ et [NA₃]⁺ ou de l'anion [SO₃]⁻ et
Y et Z désignent chacun, indépendamment l'un de l'autre, l'unité structurelle I-1 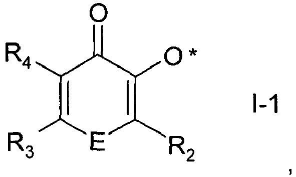 A, hydroxyle, -OA ou -NH-C(CH₃)₃, où A, R₂, R₃, R₄ et E revêtent une signification donnée ci-dessus,
W désigne -(CH₂)ₘ-O ou -(CH₂)ₒ-C(O)-O et
m et o désignent chacun, indépendamment l'un de l'autre, un nombre entier allant de 1 à 4,
à condition qu'au moins un substituant des substituants R¹ à R¹⁴ dans les formules II, III, IV, VII, X, XII et XV désigne
OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X, 2H-benzotriazol-2-yl- ou 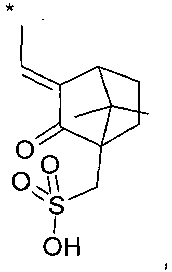 et/ou les sels, tautomères, stéréoisomères et/ou solvates de ceux-ci, y compris des mélanges de ceux-ci en toutes proportions, comme composés actifs d'adhésion de protéines.

2. Utilisation non thérapeutique de composés de formule I, 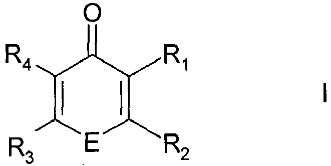 où
E désigne NR₅ ou O,
R₂, R₃ ou R₄ désignent chacun, indépendamment les uns des autres, -H, -A, -OA, -(CH₂)ₚ-OH, -C(O)OA, COOH ou COOX,
p désigne un nombre entier allant de 1 à 4,
X est le contre-ion du groupement [COO⁻],
R₅ désigne A,
A désigne alkyle ayant de 1 à 20 atomes de C,
R₁ est un substituant de formule II, III, IV, V, VI, VII, VIII, IX, X, XI, XII, XIII, XIV ou XV, 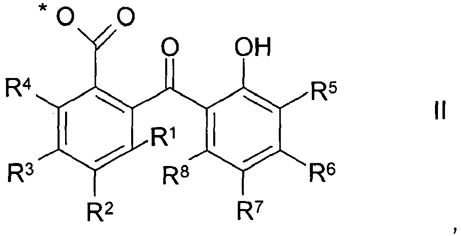 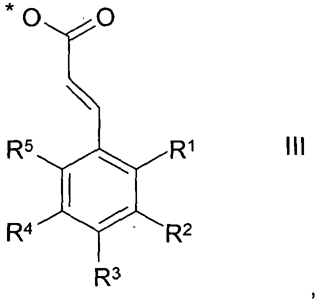 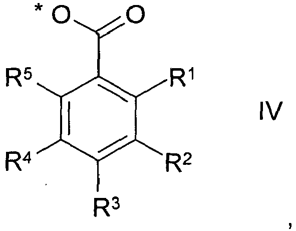 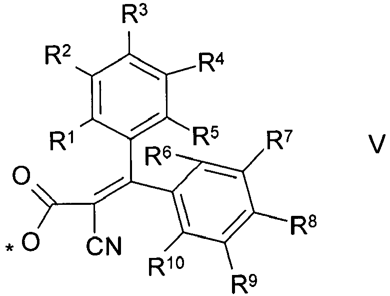 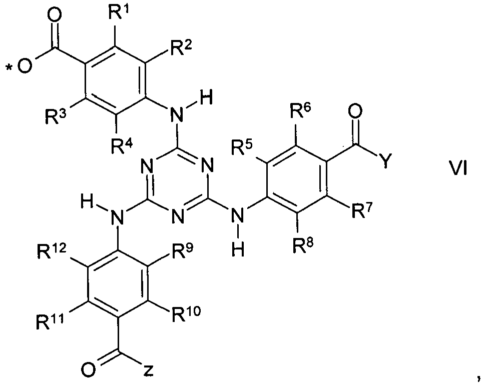 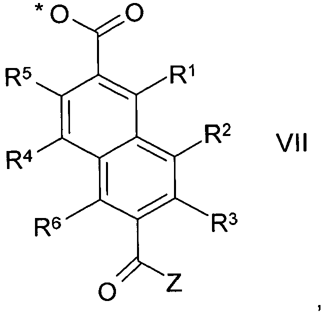 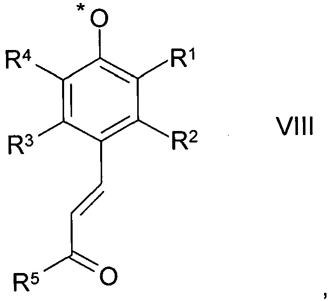 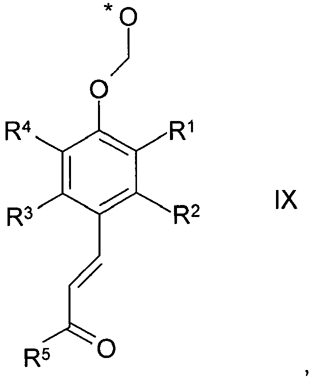 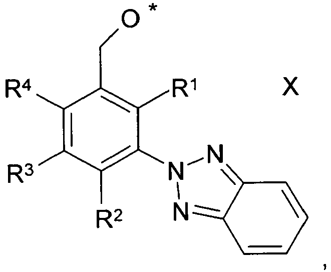 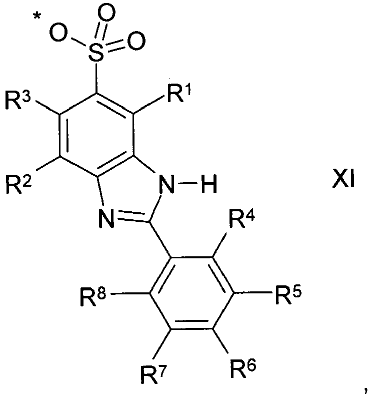 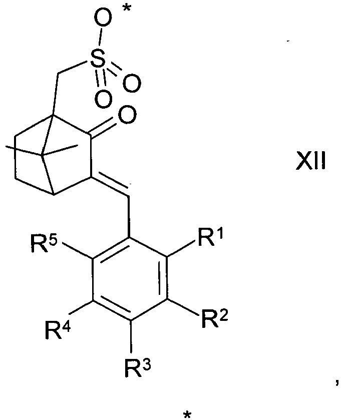 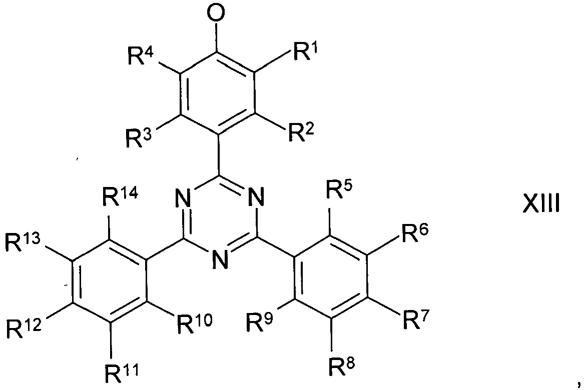 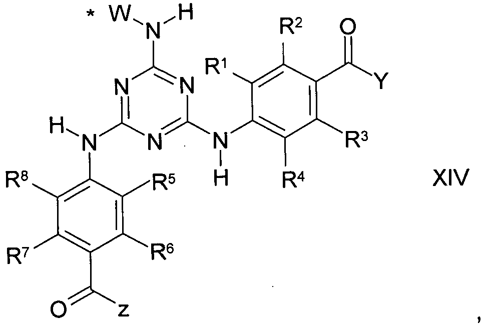 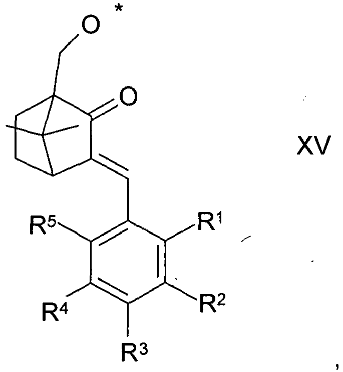 où
R¹ à R¹⁴ désignent chacun, indépendamment les uns des autres, -H, -OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X, 2H-benzotriazol-2-yl- ou 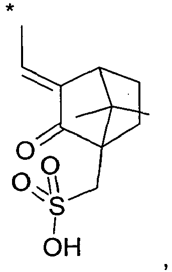
n est un nombre entier allant de 1 à 25,
X est le contre-ion des cations [NHA₂]⁺ et [NA₃]⁺ ou de l'anion [SO₃]⁻ et
Y et Z désignent chacun, indépendamment l'un de l'autre, l'unité structurelle I-1 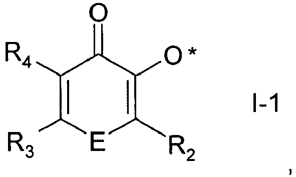 A, hydroxyle, -OA ou -NH-C(CH₃)₃, où A, R₂, R₃, R₄ et E revêtent une signification donnée ci-dessus,
W désigne -(CH₂)ₘ-O ou -(CH₂)ₒ-C(O)-O et
m et o désignent chacun, indépendamment l'un de l'autre, un nombre entier allant de 1 à 4,
à condition qu'au moins un substituant parmi les substituants R¹ à R¹⁴ dans les formules II, III, IV, VII, X, XII et XV désigne OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X, 2H-benzotriazol-2-yl- ou 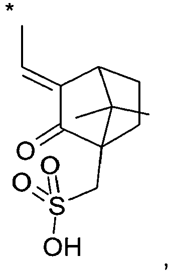 et/ou les sels, tautomères, stéréoisomères et/ou solvates de ceux-ci, y compris des mélanges de ceux-ci en toutes proportions, pour la protection de la peau, des cheveux et/ou des ongles contre un endommagement induit par les UV.

3. Composés de formule I 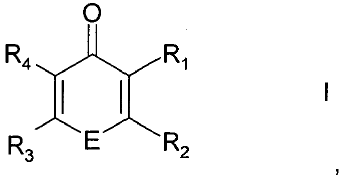 où
E désigne NR₅ ou O,
R₁ est un substituant de formule II, III, V, VI, VII, VIII, IX, X, XI, XII, XIII, XIV ou XV, 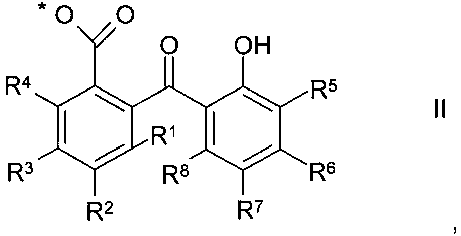 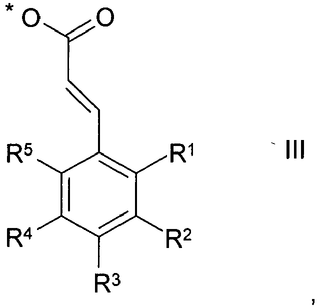 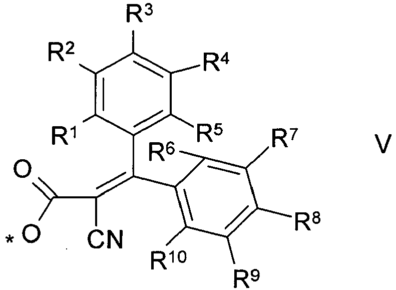 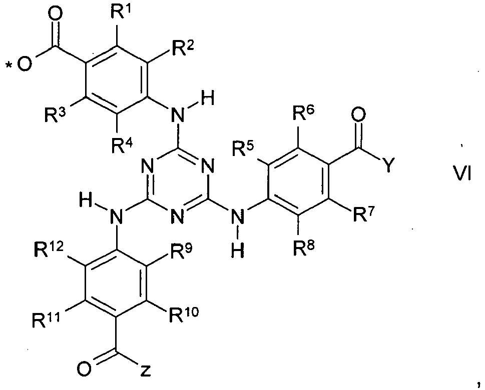 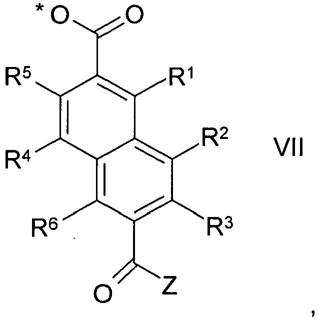 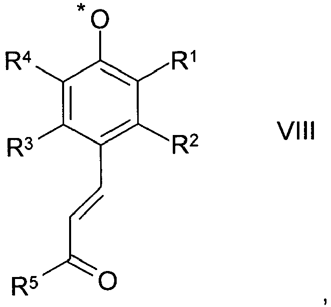 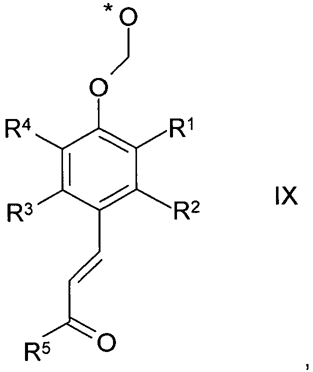 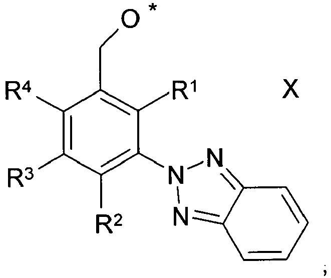 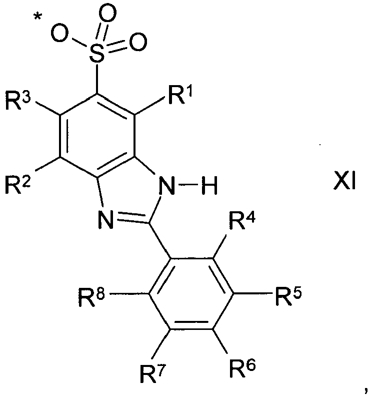 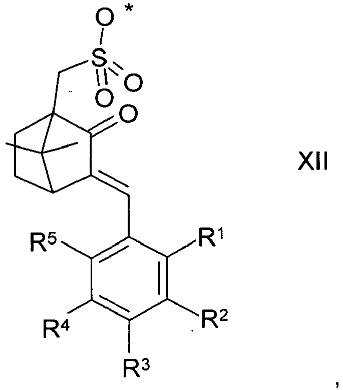 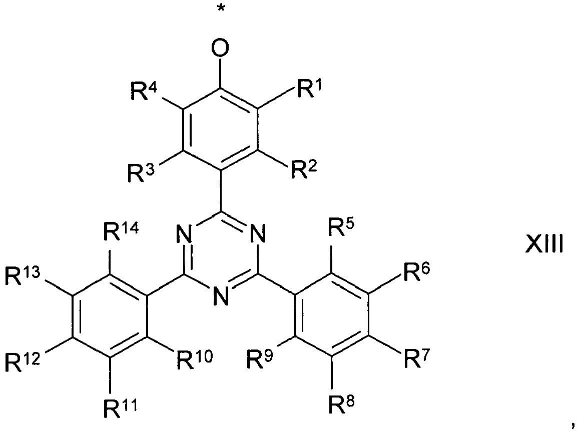 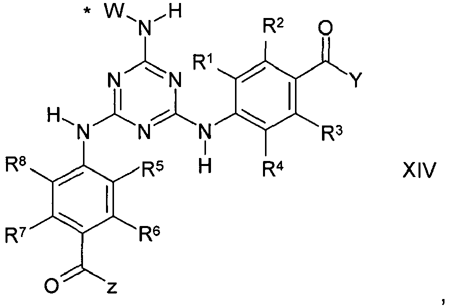 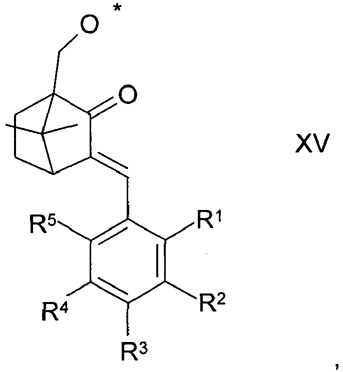 où
R¹ à R¹⁴ désignent chacun, indépendamment les uns des autres, -H, -OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X, 2H-benzotriazol-2-yl- ou 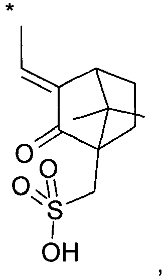
n est un nombre entier allant de 1 à 25,
X est le contre-ion des cations [NHA₂]⁺ et [NA₃]⁺ ou de l'anion [SO₃]⁻ et
Y et Z désignent chacun, indépendamment l'un de l'autre, l'unité structurelle I-1 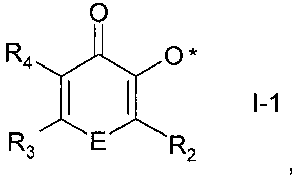 A, hydroxyle, -OA ou -NH-C(CH₃)₃,
W désigne -(CH₂)ₘ-O ou -(CH₂)ₒ-C(O)-O et
m et o désignent chacun, indépendamment l'un de l'autre, un nombre entier allant de 1 à 4,
à condition qu'au moins un substituant parmi les substituants R¹ à R¹⁴ dans les formules II, III, VII, X, XII et XV désigne OH, -OA, -A, -NH₂, -NHA, -NA₂, -NH-(CH₂-CH₂-O)n-H, -N[(CH₂-CH₂-O)ₙ-H]₂, -[NHA₂]X, -[NA₃]X, -SO₃H, -[SO₃]X, 2H-benzotriazol-2-yl- ou 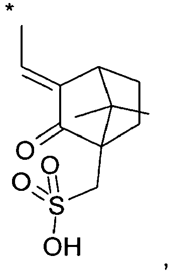 ou
R₁ désigne un substituant de formule IV, 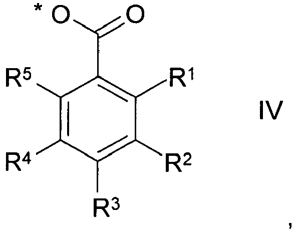 où
R² désigne 2H-benzotriazol-2-yle, R⁴ et R⁵ désignent H, l'un des substituants parmi R¹ ou R³ désigne H et l'autre substituant désigne -OH, -OA, -A, -NH₂, -NHA, -NA₂, -NH-(CH₂-CH₂-O)ₙ-H ou -N[(CH₂-CH₂-O)ₙ-H]₂,
R² ou R₄ désignent chacun, indépendamment l'un de l'autre, -H, -A, -OA, -(CH₂)ₚ-OH, -C(O)OA, COOH ou COOX,
R³ désigne -H, hydroxyméthyle, -C(O)Oéthyle ou -C(O)Ométhyle,
p désigne un nombre entier allant de 1 à 4,
X est le contre-ion du groupement [COO-],
R⁵ désigne A et
A désigne alkyle ayant de 1 à 20 atomes de C,
où le composé de (E)-3-(4-hydroxyphényl)acrylate de 2-méthyl-4-oxo-4H-pyran-3-yle est exclu.

4. Composés selon la revendication 3, **caractérisé en ce que**, dans la formule II, R⁶ désigne de préférence -OH, -OA, -A, -NH₂, -NHA, -NA₂, -NH-(CH₂-CH₂-O)ₙ-H, -N[(CH₂-CH₂-O)ₙ-H]₂, SO₃H, SO₃X ou 2H-benzotriazol-2-yle et/ou
dans la formule III, R³ désigne -OH, -OA, -A, -NH₂, -NHA, -NA₂, -NH-(CH₂-CH₂-O)ₙ-H, -N[(CH₂-CH₂-O)ₙ-H]₂, SO₃H, SO₃X ou 2H-benzotriazol-2-yle et/ou
les substituants R², R⁴, R⁷ et R⁹ dans la formule V sont H et les substituants R¹, R³, R⁵, R⁶, R⁸ et R¹⁰ sont chacun, indépendamment les uns des autres, H, -OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X ou 2H-benzotriazol-2-yle et/ou
les substituants R², R⁴, R⁵, R⁸, R⁹ et R¹² dans la formule VI sont chacun, indépendamment les uns des autres, H ou OH et les substituants R¹, R³, R⁶, R⁷, R¹⁰ et R¹¹ sont chacun, indépendamment les uns des autres, H, -OH, -OA, -A, -NH₂, -NHA, -NA₂, -NH-(CH₂-CH₂-O)n-H, -N[(CH₂-CH₂-O)ₙ-H]₂, -[NHA₂]X, -[NA₃]X, -SO₃H, -[SO₃]X ou 2H-benzotriazol-2-yle et Y et Z sont hydroxyle, -OA ou -NH-C(CH₃)₃ et/ou
les substituants R² et R⁴ dans la formule VII sont H et au moins l'un des substituants R¹, R³, R⁵ et R⁶ désigne -OH, -OA, -A, -NH₂, -NHA, -NA₂, -NH-(CH₂-CH₂-O)n-H, -N[(CH₂-CH₂-O)ₙ-H]₂, SO₃H, SO₃X ou 2H-benzo-triazol-2-yle et/ou
dans la formule VIII, R⁵ désigne -OH, -OA, -A, -NH₂, -NHA, -NA₂, -NH-(CH₂-CH₂-O)ₙ-H, -N[(CH₂-CH₂-O)ₙ-H]₂, SO₃H, SO₃X ou 2H-benzotriazol-2-yle et/ou
dans la formule IX, R⁵ désigne -OH, -OA, -A, -NH₂, -NHA, -NA₂, -NH-(CH₂-CH₂-O)ₙ-H, -N[(CH₂-CH₂-O)ₙ-H]₂, SO₃H, SO₃X ou 2H-benzotriazol-2-yle et/ou
dans la formule X, R¹ et/ou R² désignent -OH, -OA, -A, -NH₂, -NHA, -NA₂, -NH-(CH₂-CH₂-O)n-H, -N[(CH₂-CH₂-O)ₙ-H]₂, -[NHA₂]X, -[NA₃]X, -SO₃H, -[SO₃]X ou 2H-benzotriazol-2-yle et/ou
dans la formule XI, R⁶ désigne H, -OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X ou 2H-benzotriazol-2-yle et/ou
dans la formule XII, R³ désigne -OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X, 2H-benzotriazol-2-yle ou 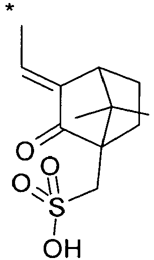 et/ou
dans la formule XIII, R⁵, R⁷, R¹² et R¹⁴ désignent chacun de préférence, indépendamment les uns des autres, -OH, -OA, -A, -NH₂, -NHA, -NA₂, -NH-(CH₂-CH₂-O)n-H, -N[(CH₂-CH₂-O)ₙ-H]₂ ou 2H-benzotriazol-2-yle et/ou
dans la formule XIV, W désigne CH₂-CH₂-O ou CH₂-CH₂-CH₂-C(O)O et/ou
dans la formule XV, R³ désigne -OH, -OA, -A, -NH₂, -NHA, -NA₂, -NH-(CH₂-CH₂-O)ₙ-H, -N[(CH₂-CH₂-O)ₙ-H]₂, -[NHA₂]X, -[NA₃]X, -SO₃H, -[SO₃]X, 2H-benzotriazol-2-yle ou

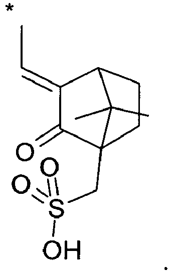

5. Procédé de préparation de composés selon la revendication 3 ou 4,
**caractérisé en ce qu'**un composé de formule XVI 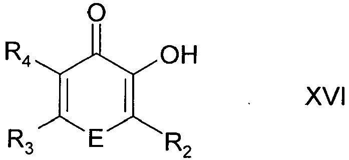
où R₂, R₃, R₄ et E revêtent une signification indiquée selon la revendication 3,
est réagi avec un composé de formule XVII
R₁-M XVII,
où R₁ revêt une signification décrite selon la revendication 3 ou 4, où les formules partielles XI et XII sont exclues, et
M désigne un cation de métal alcalin ou alcalino-terreux ou H ou
un composé de formule XVI, où R₂, R₃, R₄ et E revêtent une signification indiquée selon la revendication 3,
est réagi avec un halogénure d'acide ou un ester actif des acides libres dérivés des formules II, III, IV, V, VI, VII, XI, XII et XIV selon la revendication 3 ou 4.

6. Préparation comprenant au moins un composé selon l'une ou plusieurs parmi les revendications 3 ou 4.

7. Préparation selon la revendication 6, **caractérisé en ce qu'**elle comprend un véhicule cosmétique, dermatologique ou toléré sur le plan pharmacologique.

8. Préparation selon la revendication 6 ou 7, **caractérisé en ce qu'**au moins un autre composé actif est présent, choisi dans le groupe constitué par les filtres anti-UV, les antioxydants, les vitamines, les composés actifs antivieillissement, les composés actifs anticellulite, les substances auto-bronzantes, les substances anti-pigmentation ou les substances d'éclaircissement de la peau.

9. Préparation comprenant du (E)-3-(4-hydroxyphényl)acrylate de 2-méthyl-4-oxo-4H-pyran-3-yle ou du 2-hydroxybenzoate de 2-méthyl-4-oxo-4H-pyran-3-yle, un véhicule cosmétique, dermatologique ou toléré sur le plan pharmacologique et au moins un autre actif composé choisi dans le groupe constitué par les filtres anti-UV, les antioxydants, les vitamines, les composés actifs antivieillissement, les composés actifs anticellulite, les substances auto-bronzantes, les substances anti-pigmentation ou les substances d'éclaircissement de la peau.

10. Procédé de préparation d'une préparation selon l'une ou plusieurs parmi les revendications 6 à 9, **caractérisé en ce qu'**au moins un composé selon l'une ou plusieurs parmi les revendications 3 ou 4 ou du (E)-3-(4-hydroxyphényl)acrylate de 2-méthyl-4-oxo-4H-pyran-3-yle ou du 2-hydroxybenzoate de 2-méthyl-4-oxo-4H-pyran-3-yle est mélangé avec un véhicule et éventuellement avec d'autres substances actives ou auxiliaires.

11. Utilisation de l'unité structurelle de formule I-1 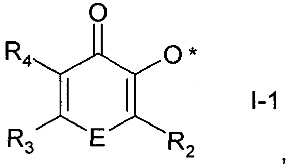 où R₂, R₃, R₄ et E revêtent une signification indiquée selon la revendication 1, comme bras de liaison pour la fonctionnalisation de matrices contenant des protéines, où le symbole * désigne le site de liaison sous la forme d'une liaison simple covalente à une molécule qui est prévue pour être adsorbée ou liée de manière covalente à la matrice.
